(19) Europäisches Patentamt
European Patent Office
Office européen des brevets

(11) Publication number: **0 558 749 A1**

(12) **EUROPEAN PATENT APPLICATION published in accordance with Art. 158(3) EPC**

(21) Application number: **91919641.0**

(22) Date of filing: **18.11.91**

(86) International application number: **PCT/JP91/01572**

(87) International publication number: **WO 92/08729 (29.05.92 92/12)**

(51) Int. Cl.[5]: **C07H 21/04**, A61K 31/70

(30) Priority: **20.11.90 JP 315007/90**

(43) Date of publication of application: **08.09.93 Bulletin 93/36**

(84) Designated Contracting States: **AT BE CH DE DK ES FR GB GR IT LI LU NL SE**

(71) Applicant: **SANKYO COMPANY LIMITED**
**5-1 Nihonbashi Honcho 3-chome**
**Chuo-ku, Tokyo 103(JP)**

(72) Inventor: **FURUKAWA, Hidehiko**
**Sankyo Company, Limited, 2-58, Hiromachi 1-chome**
**Shinagawa-ku, Tokyo 140(JP)**
Inventor: **MOMOTA, Kenji**
**Sankyo Company, Limited, 2-58, Hiromachi 1-chome**
**Shinagawa-ku, Tokyo 140(JP)**
Inventor: **TAKIGUCHI, Yo**
**Sankyo Company, Limited, 2-58, Hiromachi 1-chome**
**Shinagawa-ku, Tokyo 140(JP)**
Inventor: **HOTODA, Hitoshi**
**Sankyo Company, Limited, 2-58, Hiromachi 1-chome**
**Shinagawa-ku, Tokyo 140(JP)**
Inventor: **KANEKO, Masakatsu**
**Sankyo Company, Limited, 2-58, Hiromachi 1-chome**
**Shinagawa-ku, Tokyo 140(JP)**

(74) Representative: **Gibson, Christian John Robert et al**
**MARKS & CLERK 57/60 Lincoln's Inn Fields**
**London WC2A 3LS (GB)**

(54) **ANTISENSE NUCLEIC ACID DERIVATIVE.**

(57) A compound represented by general formula (1), its salt, and antiviral and antitumor drugs containing the same, wherein k is 0 to 20; m is 0 or 1; n is 4 to 29; X represents OH, methyl, sulfhydryl, $C_1$ to $C_4$ alkoxy or $C_1$ to $C_6$ monoalkylamino; Y and Z represent each O or S; $R^1$, $R^2$ and $R^3$ may be the same or different from each other and each represents H, $C_1$ to $C_6$ alkyl, or $C_6$ to $C_{10}$ aryl which may have the substitutent(s) of group $\alpha$; and D and B represent each independently a residue of any of the compounds of group $\beta$; provided that m is 0 when k is 0, and a base sequence containing B is complementary to a tumor gene or a virus gene: group $\alpha$: OH, $C_1$ to $C_6$ alkyl, $C_1$ to $C_6$ alkoxy, methylenedioxy, nitro, azido, halogen, $C_6$ to $C_{10}$ aryl, $C_6$ to $C_{10}$ aryloxy, and aralkyloxy composed of a $C_6$ to $C_{10}$ aryl moiety and a $C_1$ to $C_2$ alkyl moiety, group $\beta$: adenine guanine, cytosine and thymine.

EP 0 558 749 A1

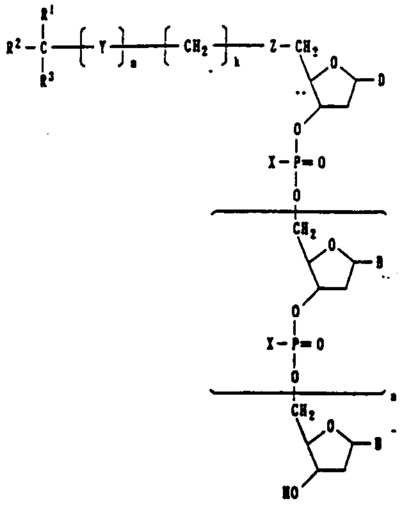

(1)

[TECHNICAL FIELD]

The present invention relates to a compound which inhibits activation or expression of virus gene or tumor gene originating in a virus or present in tumor cell, as well as an antiviral agent and an antitumor agent having said compound as its active ingredient.

[BACKGROUND ART]

Virus genes or tumor genes can be broadly classified into those that are present in cells and those from the outside that originate in viruses. It has been verified from previous research that the expression of these genes is one of the factors that induce viral disease or the tumorization of cells.

Specifically, when the genes in normal cells are expressed abnormally, regardless of whether the virus gene or tumor gene is internal or external, morphological changes, changes in adhesiveness or abnormal proliferation occur that are characteristic phenomena associated with viral diseases or tumorization.

Thus, drugs that suppress these kinds of gene activation or expression are expected to be developed as being effective in the prevention and treatment of viral diseases or cancer.

Oligonucleotides complementary to the coding sequence of a certain gene, or mRNA transcribed from said gene (hereinafter to be referred to as "anti-sense oligonucleotide") is known to inhibit the expression or that gene. In particular, extensive research has been performed on the action of anti-sense oligonucleotide of virus and tumor genes. For example, it has been reported that anti-sense oligonucleotide demonstrates anti-HIV action. In addition, it has also been reported that an 18 base oligonucleotide complementary to the mRNA transcribed for c-myb gene inhibits proliferation of cells having said gene (Proc. Natl. Acad. Sci. USA, 86, 3379-3383(1989)).

However, for reasons including naturally-occurring oligonucleotides having a low efficiency of being taken up into cells as well as being susceptible to decomposition by nucleases, there are problems with their effects and the duration of those effects when used as antiviral agents and antitumor agents.

To overcome these limitations, various derivatives of these naturally-occurring oligonucleotides have been synthesized , and research has been conducted on their effects against virus-infected cells or cells having tumor genes (hereinafter to be simply referred to as "tumor cells"). In particular, it has been discovered that anti-sense methylphosphonate oligonucleotides, in which hydroxyl groups of phosphodiester bond portions are substituted with methyl groups (U.S. Patent No. 4,511,713) and anti-sense phosphorothioate oligonucleotides, in which oxygen atoms of phosphodiester bond portions are substituted with sulfur atoms (Japanese Patent Application No. Hei-1-503302) bring about decreased colony formation rate and proliferation inhibition against virus-infected cells and tumor cells, thereby confirming their efficacy as antiviral agents or antitumor agents.

[DISCLOSURE OF THE INVENTION]

As a result of intensive research conducted over an extended period of intensive, the inventors of the present invention discovered that, when studies were conducted on the effects on virus-infected cells and tumor cells of anti-sense oligonucleotide derivatives obtained by chemical modification that completely differs from methods of the prior art, or in other words, anti-sense oligonucleotides wherein the hydroxyl group of the 5'-position of a 5' terminal nucleotide is modified with various substituents, these compounds have activity that suppresses viral proliferation or activity that suppresses tumor cell proliferation, suppress the occurrence of viral diseases or the characteristic morphological changes of tumor cells, and demonstrate effects such as those that specifically suppress proliferation, thereby leading to completion of the present invention.

Incidentally, in the present specification, adenine is abbreviated as A, guanine as G, cytosine as C, thymine as T, adenine nucleotide as $\underline{A}$, guanine nucleotide as $\underline{G}$, cytosine nucleotide as $\underline{C}$, and thymine nucleotide as $\underline{T}$.

In the present invention, viruses are to include either DNA virus or RNA virus.

Examples of DNA virus include adenovirus, hepatitis A virus, hepatitis B virus and herpes virus.

While examples of RNA virus include AIDS virus (hereinafter to be referred to as "HIV"), adult T-type leukemia virus, influenza virus and hepatitis C virus, HIV is preferable.

In the present invention, "virus genes" and "tumor genes" generically refer to genes whose activity induce normal cells to undergo viral lesions or tumorization regardless of whether they are internal or external. More specifically, examples of genes resulting in tumorization of normal cells include c-Ha-ras-(Nature, 302, 33-37, (1983), Nature, 300, 149-152, (1982), Proc. Natl. Acad. Sci. U.S.A., 81, 4771-4775,

Proc. Natl. Acad. Sci. U.S.A., 81, 5384-5388, (1984)), K-ras (Proc. Natl. Acad. Sci. U.S.A., 81, 71-75, (1984)), N-ras (EMBO.J., 3, 1321-1326 (1984), c-cis (Mol. Cell. Biol., 6, 3018-3022, (1986)), c-myc (Mol. Cell. Biol., 8, 124-129, (1988)), myb (Proc. Natl. Acad. Sci. U.S.A., 83, 9636-9640, (1986)), erb B (Nature, 319, 230-234, (1986), c-jun (Proc. Natl. Acad. Sci. U.S.A., 85, 9148-9152, (1988)), src (Mol. Cell. Biol., 7, 1978-1983(1987)-).

In addition, examples of genes that cause viral lesions include HIV-1 (Proc. Natl. Acad. Sci. USA, 85, 5507-5511 (1988), ibid, 86, 4244-4248 (1989), ibid, 84, 7706-7710 (1987), Gene, 72, 343-347 (1988), Proc. Natl. Acad. Sci. USA, 86, 7790-7794 (1989), ibid., 85, 7079-7083 (1988), ibid., 83, 4143-4146 (1986),), HSV-1 (Proc. Natl. Acad. Sci. USA, 83, 2787-2791 (1986), ibid, 86, 6868-6872 (1989),), VSV (Biochemistry, 25, 6268-6275 (1986), Proc. Natl. Acad. Sci. USA, 84, 648-652 (1987), Nucleosides & Nucleotides, 8, 825-828 (1989), Nucleic Acids Res., 18, 3777-3783 (1990)). In particular, detailed research has been conducted regarding HIV-1, and examples of those genes include tat (Science, 229, 69-73 (1985)) and rev (Nature, 321, 412-417 (1986)).

"To be complementary" means that thymine nucleotide is complementary to adenine nucleotide or uracil nucleotide; cytosine nucleotide, to guanine nucleotide; guanine nucleotide, to cytosine nucleotide; and adenine nucleotide, to thymine nucleotide of a virus gene or tumor gene of DNA itself or RNA.

"Being complementary to a virus gene or tumor gene" refers to being complementary to the nucleotide sequence of the code chain side of a tumor gene, or being complementary to the nucleotide sequence of mRNA, either before or after splicing, transcribed by a virus gene or tumor gene.

"A single base site" refers to a point mutation site in a tumor gene that causes activation of a tumor gene, at which 1 or 2 nucleotides of the original nucleotide sequence are substituted.

The compound of the present invention is the compound represented by the general formula (1).

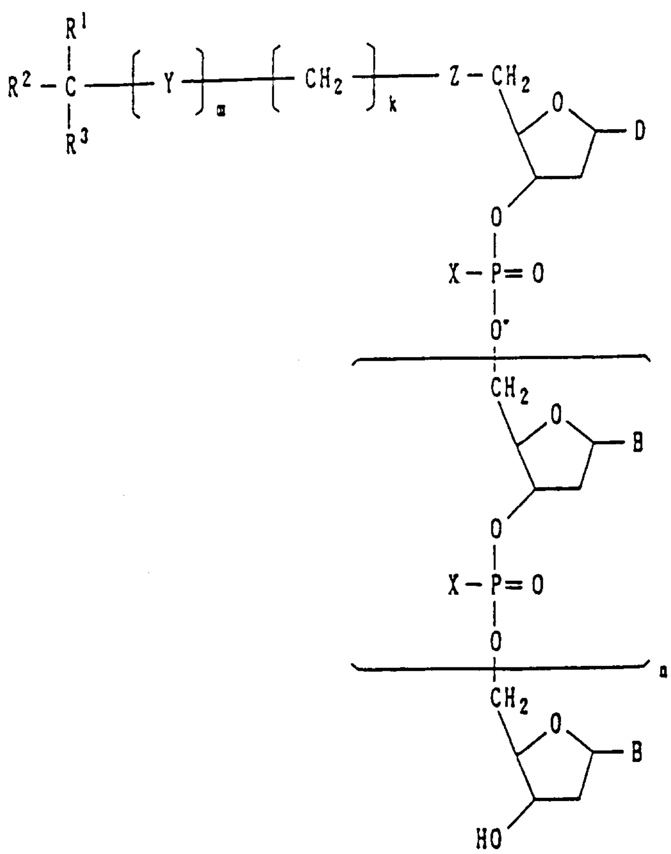

(1)

In the above formula (1), k represents 0 or an integer of 1 to 20; m represents 0 or 1; n represents an integer of 4 to 29; X represents a hydroxyl group, a methyl group, a mercapto group, an alkoxy group having 1 to 4 carbon atoms or a monoalkylamino group having 1 to 6 carbon atoms; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substitutent selected from the following group $\alpha$; and D and B each

represent a residue independently selected from the following group β in respective nucleotide units. However, m = 0 when k = 0, and the base sequence containing B is the base sequence complementary to a partial tumor gene or virus gene.

[Group α]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group β]

Adenine (A), guanine (G), cytosine (C) and thymine (T).

In addition, the present invention relates to an antiviral agent and antitumor agent having as its active ingredient the compound indicated in the above-mentioned general formula (1) and its salt.

The "base sequence containing B" refers to the following formula:

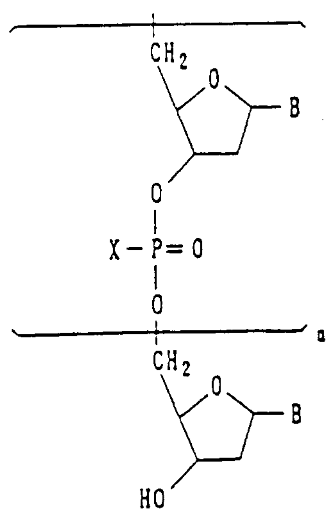

wherein Bs may be different from each other in the case where said sequence repeats n times.

In the above-mentioned general formula (1), examples of X in the form of an alkoxy group having 1 to 4 carbon atoms include methoxy, ethoxy, n-propoxy, isopropoxy, n-butoxy, isobutoxy, s-butoxy and t-butoxy groups, and preferably methoxy and ethoxy groups.

In the above-mentioned general formula (1), examples of X in the form of a monoalkylamino group having 1 to 6 carbon atoms include methylamino, ethylamino. n-propylamino, isopropylamino, n-butylamino, isobutylamino, s-butylamino, t-butylamino, n-pentylamino, isopentylamino, 2-methylbutylamino, neopentylamino, 1-ethylpropylamino, n-hexylamino, 4-methylpentylamino, 3-methylpentylamino, 2-methylpentylamino, 1-methylpentylamino, 3,3-dimethylbutylamino, 2,2-dimethylbutylamino, 1,1-dimethylbutylamino, 1,2-dimethylbutylamino, 1,3-dimethylbutylamino, 2,3-dimethylbutylamino and 2-ethylbutylamino groups, preferably those having 1 to 4 carbon atoms, and more preferably ethylamino propylamino and butylamino groups.

In the above-mentioned general formula (1), examples of $R^1$, $R^2$ and $R^3$ in the form of alkyl groups having 1 to 6 carbon atoms include methyl, ethyl, n-propyl, isopropyl, n-butyl, isobutyl, s-butyl, t-butyl, n-pentyl, isopentyl, 2-methylbutyl, neopentyl, 1-ethylpropyl, n-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl and 2-ethylbutyl groups, and preferably those having 1 to 4 carbon atoms.

In the above-mentioned general formula (1), examples of $R^1$, $R^2$ and $R^3$ in the form of aryl groups include phenyl, 1-naphthyl, 2-naphthyl, 1-anthryl and 2-anthryl 69oups, preferably phenyl and naphthyl groups, and more preferably phenyl group.

In the above-mentioned general formula (1), examples of $R^1$, $R^2$ and $R^3$ in the form of alkyl groups having 1 to 6 carbon atoms as substituted portions of aryl groups include methyl, ethyl, n-propyl, isopropyl,

n-butyl, isobutyl, s-butyl, t-butyl, n-pentyl, isopentyl, 2-methylbutyl, neopentyl, 1-ethylpropyl, n-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl and 2-ethylbutyl groups, preferably those having 1 to 4 carbon atoms, and more preferably isopropyl and t-butyl groups.

In the above-mentioned general formula (1), examples of $R^1$, $R^2$ and $R^3$ in the form of alkoxy groups having 1 to 6 carbon atoms as substituted portions of aryl groups include methoxy, ethoxy, n-propoxy, isopropoxy, n-butoxy, isobutoxy, s-butoxy, t-butoxy, n-pentoxy, isopentoxy, 2-methylbutoxy, neopentoxy, n-hexyloxy, 4-methylpentoxy, 3-methylpentoxy, 2-methylpentoxy, 3,3-dimethylbutoxy, 2,2-dimethylbutoxy, 1,1-dimethylbutoxy, 1,2-dimethylbutoxy, 1,3-dimethylbutoxy and 2,3-dimethylbutoxy groups, preferably those having 1 to 4 carbon atoms, and more preferably methoxy and ethoxy groups.

In the above-mentioned general formula (1), examples of $R^1$, $R^2$ and $R^3$ in the form of halogen atoms that are substituents of aryl groups include fluorine, chlorine, bromine and iodine, and preferably fluorine, chlorine and bromine.

In the above-mentioned general formula (1), examples of $R^1$, $R^2$ and $R^3$ in the form of aryl groups having 6 to 10 carbon atoms that are substituents of aryl groups include phenyl, 1-naphthyl, 2-naphthyl, 1-anthryl and 2-anthryl groups, preferably phenyl and naphthyl groups, and more preferably phenyl groups.

In the above-mentioned general formula (1), examples of $R^1$, $R^2$ and $R^3$ in the form of aryloxy groups having 6 to 10 carbon atoms that are substituents of aryl groups include phenyloxy, 1-naphthyloxy, 2-naphthyloxy, 1-anthryloxy and 2-anthryloxy groups, and preferably phenyloxy and naphthyloxy groups.

In the above-mentioned general formula (1), examples of $R^1$, $R^2$ and $R^3$ in the form of aralkyloxy groups with an aryl moiety having 6 to 10 carbon atoms and an alkyl moiety having 1 or 2 carbon atoms, that are substituents of aryl groups, include phenylmethyloxy, phenethyloxy, 1-naphthylmethyloxy, 2-naphthylethyloxy and 2-anthrylmethyloxy groups, and preferably phenylmethyloxy and naphthylmethyloxy groups.

In the above-mentioned general formula (1), n is preferably 8 to 29, and more preferably 13 to 18.

In the above-mentioned general formula (1), k is preferably 0 to 15, and more preferably 0 to 12.

In the above-mentioned general formula (1), while the base sequence containing B includes all base sequences provided they are sequences complementary to a virus gene or tumor gene, preferable examples of said sequence are as listed below.

(1) A base sequence of 9 to 30 bases complementary to a portion or all of a nucleotide of base no. 7947 (Nature 313, 450-458 (1985)) to base no. 7975 in HIV gene; and

(2) A base sequence of 14 to 19 bases complementary to a portion or all of a nucleotide of base no. 7947 to base no. 7975 in HIV gene.

In addition,

(3) A base sequence of 9 to 30 bases that is complementary to all or a portion of a region of 30 nucleotides in front and in back based on no more than 30 nucleotides in front of and in back of the translation initiation site in a tumor gene;

(4) A base sequence of 14 to 19 bases that is complementary to a portion of a region of 30 nucleotides in front and in back based on no more than 30 nucleotides in front of and in back of the translation initiation site in a tumor gene;

(5) A base sequence of 9 to 30 bases that is complementary to all or a portion of a region of 30 nucleotides in front and in back based on more than 5 nucleotides in front of and in back of the translation initiation site in a tumor gene;

(6) A base sequence of 14 to 19 bases that is complementary to a portion of a region of 30 nucleotides in front and in back based on no more than 5 nucleotides in front of and in back of the translation initiation site in a tumore gene;

(7) A base sequence of 9 to 30 bases that is complementary to all or a portion of a region of 30 nucleotides in front and in back based on no more than 30 nucleotides in front of and in back of a single base site in a tumor gene;

(8) A base sequence of 14 to 19 bases that is complementary to a portion of a region of 30 nucleotides in front and in back based on no more than 30 nucleotides in front of and in back of a single base site in a tumor gene;

(9) A base sequence of 9 to 30 bases that is complementary to all or a portion of a region of 30 nucleotides in front and in back based on no more than 5 nucleotides in front of and in back of a single base site in ras and c-myb tumor genes; and,

(10) A base sequence of 14 to 19 bases that is complementary to a portion of a region of 30 nucleotides in front and in back based on no more than 5 nucleotides in front of and in back of a single base site in ras and c-myb tumor genes.

In the above-mentioned general formula (1), preferred compounds include:

2) a compound wherein:

k is 0 to 20,

m is 0 or 1,

n is 4 to 29,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms or an aryl group having 6 to 10 carbon atoms that may have a substituent selected from the following group $\alpha$ and,

the base sequence containing B is a base sequence complementary to a tumor gene or virus gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy groups with an aryl moiety having 6 to 10 carbon atoms and an alkyl moiety having 1 or 2 carbon atoms.

3) a compound wherein:

k is 0 to 15,

m is 0 or 1,

n is 8 to 29,

X is a hydroxyl group, a methyl group, a mercapto group, an alkoxy group having 1 to 4 carbon atoms or a monoalkylamino group having 1 to 6 carbon atoms,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence complementary to a tumor gene or virus gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

4) a compound wherein:

k is 0 to 15,

m is 0 or 1,

n is 8 to 29,

X is a hydroxyl group.

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence complementary to a tumor gene or virus gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

5) a compound wherein:

k is 0 to 12,

m is 0,
n is 13 to 18,
X is a hydroxyl group, a methyl group, a mercapto group, an alkoxy group having 1 to 4 carbon atoms or a monoalkylamino group having 1 to 6 carbon atoms,
Y is an oxygen or sulfur atom,
Z is an oxygen or sulfur atom,
$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence complementary to a tumor gene or virus gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.
6) a compound wherein:
k is 0 to 12,
m is 0 or 1,
n is 13 to 18,
X is a hydroxyl group,
Y is an oxygen or sulfur atom,
Z is an oxygen or sulfur atom.
$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence complementary to a tumor gene or virus gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.
7) a compound wherein:
k is 0 to 12,
m is 0 or 1,
n is 13 to 18,
X is a hydroxyl group,
Y is an oxygen atom,
Z is an oxygen atom,
$R^1$, $R^2$ and $R^3$ may be the same or different from one another and each represent a hydrogen atom, an alkyl group having 1 to 4 carbon atoms, or an aryl group having 6 to 10 carbon atoms that may have a substituent selected from the following group $\alpha'$ and,
the base sequence containing B is a base sequence complementary to a tumor gene or virus gene.

[Group $\alpha'$]

Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.
8) a compound wherein:
k is 0 to 12,
m is 0 or 1,
n is 13 to 18,
X is a hydroxyl group,
Y is an oxygen atom,
Z is an oxygen atom,

$R^1$, $R^2$ and $R^3$ may be the same or different from one another and each represent a hydrogen atom, an alkyl group having 1 to 4 carbon atoms, or a phenyl group that may have a substituent selected from the following group α'' and,

the base sequence containing B is a base sequence complementary to a tumor gene or virus gene.

[Group α'']

Methyl group, ethyl group, propyl group, t-butyl group, methoxy group, ethoxy group, propoxy group, t-butoxy group and phenylmethyloxy group.

9) a compound wherein:

k is 0 to 20,

m is 0 or 1,

n is 4 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to a portion or all of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy groups with an aryl moiety having 6 to 10 carbon atoms and an alkyl moiety having 1 or 2 carbon atoms.

10) a compound wherein:

k is 0 to 20,

m is 0 or 1,

n is 4 to 29,

X is a hydroxyl group.

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl groups having 6 to 10 carbon atoms that may have a substituent selected from the following group α and,

the base sequence containing B is a base sequence of 9 to 30 bases complementary to a portion or all of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

11) a compound wherein:

k is 0 to 15,

m is 0 or 1,

n is 8 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a

substituent selected from the following group $\alpha$ and,

the base sequence containing B is a base sequence of 9 to 30 bases complementary to a portion or all of a nucleotide of base number 7947 to base number 7975 in HIV gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

12) a compound wherein:

k is 0 to 15,

m is 0 or 1,

n is 8 to 29,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence of 9 to 30 bases complementary to a portion or all of a nucleotide of base number 7947 to base number 7975 in HIV gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

13) a compound wherein:

k is 0 to 12,

m is 0,

n is 13 to 18,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence of 9 to 30 bases complementary to a portion or all of a nucleotide of base number 7947 to base number 7975 in HIV gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

14) a compound wherein:

k is 0 to 12,

m is 0 or 1,

n is 13 to 18,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a

substituent selected from the following group α and,

the base sequence containing B is a base sequence of 9 to 30 bases complementary to a portion or all of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy groups with an aryl moiety having 6 to 10 carbon atoms and an alkyl moiety having 1 or 2 carbon atoms.

15) a compound wherein:

k is 0 to 12,

m is 0 or 1,

n is 13 to 18,

X is a hydroxyl group,

Y is an oxygen atom,

Z is an oxygen atom,

$R^1$, $R^2$ and $R^3$ may be the same or different from one another and each represent a hydrogen atom, an alkyl group having 1 to 4 carbon atoms, or an aryl group having 6 to 10 carbon atoms that may have a substituent selected from the following group α' and,

the base sequence containing B is a base sequence of 9 to 30 bases complementary to a portion or all of a nucleotide of base number 7947 to base number 7975 in HIV gene.

[Group α']

Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.

16) a compound wherein:

k is 0 to 12,

m is 0 or 1,

n is 13 to 18,

X is a hydroxyl group,

Y is an oxygen atom,

Z is an oxygen atom,

$R^1$, $R^2$ and $R^3$ may be the same or different from one another and each represent a hydrogen atom, an alkyl group having 1 to 4 carbon atoms, or a phenyl group that may have a substituent selected from the following group α'' and,

the base sequence containing B is a base sequence of 9 to 30 bases complementary to a portion or all of a nucleotide of base number 7947 to base number 7975 in HIV gene.

[Group α'']

Methyl group, ethyl group, propyl group, t-butyl group; methoxy group, ethoxy group, propoxy group, t-butoxy group and phenylmethyloxy group.

17) a compound wherein:

k is 0 to 20,

m is 0 or 1,

n is 4 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

18) a compound wherein:

k is 0 to 20,

m is 0 or 1,

n is 4 to 29,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

19) a compound wherein:

k is 0 to 15,

m is 0 or 1,

n is 8 to 29,

X is a hydroxyl group, a methyl group, a mercapto group, an alkoxy group having 1 to 6 carbon atoms, or a monoalkylamino group having 1 to 6 carbon atoms,

Y is an oxygen or sulfur atom,

Z is an oxygen or sulfur atom,

$R^1$ $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

20) a compound wherein:

k is 0 to 15,

m is 0 or 1,

n is 8 to 29,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

21) a compound wherein:

k is 0 to 12,

m is 0,

n is 13 to 18,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

22) a compound wherein:

k is 0 to 12,

m is 0 or 1,

n is 13 to 18,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

23) a compound wherein:

k is 0 to 12,

m is 0 or 1,

n is 13 to 18,

X is a hydroxyl group,

Y is an oxygen atom,

Z is an oxygen atom,

$R^1$, $R^2$ and $R^3$ may be the same or different from one another and each represent a hydrogen atom, an alkyl group having 1 to 4 carbon atoms or an aryl group having 6 to 10 carbon atoms that may have a substituent selected from the following group α' and,

the base sequence containing B is a base sequence of 14 to 19 bases complementary to all or a part of a nucleotide of base number 7947 to base number 7975 in HIV gene.

[Group α']

Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.

24) a compound wherein:

k is 0 to 12,

m is 0 or 1,

n is 13 to 18,

X is a hydroxyl group,

Y is an oxygen atom,

Z is an oxygen atom,

$R^1$, $R^2$ and $R^3$ may be the same or different from one another and each represent a hydrogen atom, an alkyl group having 1 to 4 carbon atoms, or a phenyl group that may have a substituent selected from the following group α'' and,

the base sequence containing B is a base sequence of 14 to 19 bases complementary to all or a part of a nucleotide of base number 7947 to base number 7975 in HIV gene.

[Group α'']

Methyl group, ethyl group, propyl group, t-butyl group, methoxy group, ethoxy group, propoxy group, t-butoxy group and phenylmethyloxy group.

25) a compound wherein:

k is 0 to 20.

m is 0 or 1,

n is 4 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a region of 30 nucleotides which is around a criterional nucleotide positioning less than 5 nucleotides from the translation initiation site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

26) a compound wherein:

k is 0 to 20,

m is 0 or 1,

n is 4 to 29,

X is a hydroxyl group,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a region of 30 nucleotides which is around a criterional nucleotide positioning less than 5 nucleotide from the translation initiation site in a tumor gene.

[Group α]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

27) a compound wherein:

k is 0 to 15,

m is 0 or 1,

n is 8 to 29,

X is a hydroxyl group, a methyl group, a mercapto group, an alkoxy group having 1 to 4 carbon atoms, or a monoalkylamino group having 1 to 6 carbon atoms.

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a region of 30 nucleotides which is around a criterional nucleotide positioning less than 5 nucleotides from the translation initiation site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

28) a compound wherein:

k is 0 to 15,

m is 0 or 1,

n is 8 to 29,

X is a hydroxyl group,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a region of 30 nucleotides which is around a criterional nucleotide positioning less than 5 nucleotides from the translation initiation site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

29) a compound wherein:

k is 0 to 12,

m is 0,

n is 13 to 18,

X is a hydroxyl group, methyl group, mercapto group, alkoxy group having 1 to 4 carbon atoms, or monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a region of 30 nucleotides which is around a criterional nucleotide positioning less than 5 nucleotides from the translation initiation site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

30) a compound wherein:

k is 0 to 12,
m is 0 or 1,
n is 13 to 18,
X is a hydroxyl group,
Y is an oxygen or sulfur atom,
Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a region of 30 nucleotides which is around a criterional nucleotide positioning less than 5 nucleotides from the translation initiation site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

31) a compound wherein:

k is 0 to 12,
m is 0 or 1,
n is 13 to 18,
X is a hydroxyl group,
Y is an oxygen atom,
Z is an oxygen atom,

$R^1$, $R^2$, and $R^3$ may be the same or different from one another and each represent a hydrogen atom, an alkyl group having 1 to 4 carbon atoms, or an aryl group having 6 to 10 carbon atoms that may have a substituent selected from the following group α' and,

the base sequence containing B is a base sequence of 9 to 30 bases complementary to all or a part of a region of 5 nucleotides which is around a criterional nucleotide positioning less than 30 nucleotides from the translation initiation site in a tumor gene.

[α' group]

Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.

32) a compound wherein:

k is 0 to 12,
m is 0 or 1,
n is 13 to 18,
X is a hydroxyl group,
Y is an oxygen atom,
Z is an oxygen atom,

$R^1$, $R^2$, and $R^3$ may be the same or different from one another and each represent a hydrogen atom, an alkyl group having 1 to 4 carbon atoms, or a phenyl group that may have a substituent selected from the following group α'' and,

the base sequence containing B is a base sequence of 9 to 30 bases complementary to all or a part of a region of 30 nucleotides which is around a criterional nucleotide positioning less than 5 nucleotides from the translation initiation site in a tumor gene.

[Group α'']

Methyl group, ethyl group, propyl group, t-butyl group, methoxy group, ethoxy group, propoxy group, t-butoxy group, and phenylmethyloxy group.

33) a compound wherein:

k is 0 to 20,

m is 0 or 1,

n is 4 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to a part of a region of 30 nucleotides which is around a criterional nucleotide positioning less than 5 nucleotides from the translation initiation site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

34) a compound wherein:

k is 0 to 20,

m is 0 or 1,

n is 4 to 29,

X is a hydroxyl group,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to a part of a region of 30 nucleotides which is around a criterional nucleotide positioning less than 5 nucleotides from the translation initiation site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

35) a compound wherein:

k is 0 to 15,

m is 0 or 1,

n is 8 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an

alkyl group having 1 to 6 carbon atoms, or an aryl group having 6 to 10 carbon atoms that may have a substituent selected from the following group α and,

the base sequence containing B is a base sequence of 14 to 19 bases complementary to a part of a region of 30 nucleotides which is around a criterional nucleotide positioning less than 5 nucleoties from the translation initiation site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

36) a compound wherein:

k is 0 to 15,

m is 0 or 1,

n is 8 to 29,

X is a hydroxyl group,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to a part of a region of 30 nucleotides which is around a criterional nucleotide positioning less than 5 nucleotides from the translation initiation site in a tumor gene.

[Group α]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

37) a compound wherein:

k is 0 to 12,

m is 0,

n is 13 to 18,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to a part of a region of 30 nucleotides which is around a criterional nucleotide positioning less than 5 nucleotides from the translation initiation site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

38) a compound wherein:

k is 0 to 12,

m is 0 or 1,

n is 13 to 18,

X is a hydroxyl group,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to a part of a region of 30 nucleotides which is around a criterional nucleotide positioning less than 5 nucleotides from the translation initiation site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

39) a compound wherein:

k is 0 to 12,

m is 0 or 1,

n is 13 to 18,

X is a hydroxyl group,

Y is an oxygen atom,

Z is an oxygen atom,

$R^1$ $R^2$ and $R^3$ may be the same or different from one another and each represent a hydrogen atom, an alkyl group having 1 to 4 carbon atoms, or an aryl group having 6 to 10 carbon atoms that may have a substituent selected from the following group α' and,

the base sequence containing B is a base sequence of 14 to 19 bases complementary to a part of a region of 30 nucleotides which is around a criterional nucleotide positioning less than 5 nucleotides from the translation initiation site in a tumor gene.

[Group α']

Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.

40) a compound wherein:

k is 0 to 12,

m is 0 or 1,

n is 13 to 18,

X is a hydroxyl group,

Y is an oxygen atom,

Z is an oxygen atom,

$R^1$, $R^2$, and $R^3$ may be the same or different from one another and each represent a hydrogen atom, an alkyl group having 1 to 4 carbon atoms, or a phenyl group that may have a substituent selected from the following group α'' and,

the base sequence containing B is a base sequence of 14 to 19 bases complementary to a part of a region of 30 nucleotides which is around a criterional nucleotide positioning less than 5 nucleotides from the translation initiation site in a tumor gene.

[Group α'']

Methyl group, ethyl group, propyl group, t-butyl group, methoxy group, ethoxy group, propoxy group, t-butoxy group and phenylmethyloxy group.

41) a compound wherein:

k is 0 to 20,

m is 0 or 1,

n is 4 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a region of 30 nucleotides which is around a criterional nucleotide positioning less than 5 nucleotides from the single base site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

42) a compound wherein:

k is 0 to 20,

m is 0 or 1,

n is 4 to 29,

X is a hydroxyl group,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a region of 30 nucleotides which is around a criterional nucleotide positioning less than 5 nucleotides from the single base site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

43) a compound wherein:

k is 0 to 15,

m is 0 or 1,

n is 8 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a region of 30 nucleotides which is around a criterional nucleotide Positioning less than 5 nucleotides from the single base site in a tumor gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

44) a compound wherein:

k is 0 to 15,

m is 0 or 1,

n is 8 to 29,

X is a hydroxyl group,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence of 9 to 30 bases complementary to all or a part of a region of 30 nucleotides which is around a criterional nucleotide positioning less than 5 nucleotides from the single base site in a tumor gene.

[Group $\alpha$]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

45) a compound wherein:

k is 0 to 12,

m is 0,

n is 13 to 18,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence of 9 to 30 bases complementary to all or a part of a region of 30 nucleotides which is around a criterional nucleotide positioning less than 5 nucleotides from the single base site in a tumor gene.

[Group $\alpha$]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

46) a compound wherein:

k is 0 to 12,

m is 0 or 1,

n is 13 to 18,

X is a hydroxyl group,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence of 9 to 30 bases complementary to all or a part of a region of 30 nucleotides which is around a criterional nucleotide positioning less than 5 nucleotide from the single base site in a tumor gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

47) a compound wherein:
k is 0 to 12,
m is 0 or 1,
n is 13 to 18,
X is a hydroxyl group,
Y is an oxygen atom,
Z is an oxygen atom,
$R^1$, $R^2$, and $R^3$ may be the same or different from one another and each represent a hydrogen atom, an alkyl group having 1 to 4 carbon atoms, or an aryl group having 6 to 10 carbon atoms that may have a substituent selected from the following group α' and,
the base sequence containing B is a base sequence of 9 to 30 bases complementary to all or a part of a region of 30 nucleotides which is around a criterional nucleotide positioning less than 5 nucleotides from the single base site in a tumor gene.

[Group α']

Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.
48) a compound wherein:
k is 0 to 12,
m is 0 or 1,
n is 13 to 18,
X is a hydroxyl group,
Y is an oxygen atom,
Z is an oxygen atom,
$R^1$, $R^2$, and $R^3$ may be the same or different from one another and each represent a hydrogen atom, an alkyl group having 1 to 4 carbon atoms, or a phenyl group that may have a substituent selected from the following group α'' and,
the base sequence containing B is a base sequence of 9 to 30 bases complementary to all or a part of a region of 30 nucleotides which is around a criterional nucleotide positioning less than 5 nucleotides from the single base site in a tumor gene.

[Group α'']

Methyl group, ethyl group, propyl group, t-butyl group, methoxy group, ethoxy group, propoxy group, t-butoxy group and phenylmethyloxy group.
49) a compound wherein:
k is 0 to 20,
m is 0 or 1,
n is 4 to 29,
X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 14 to 19 bases complementary to a part of a region of 30 nucleotides which is around a criterional nucleotide positioning less than 5 nucleotides from the single base site in ras and c-myb tumor genes.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.
50) a compound wherein:

k is 0 to 20,
m is 0 or 1,
n is 4 to 29,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 14 to 19 bases complementary to a part of a region of 30 nucleotides which is around a criterional nucleotide positioning less than 5 nucleotides from the single base site in ras and c-myb tumor genes.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.
51) a compound wherein:
k is 0 to 15,
m is 0 or 1,
n is 8 to 29,
X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 14 to 19 bases complementary to a part of a region of 30 nucleotides which is around a criterional nucleotide positioning less than 5 nucleotides from the single base site in ras and c-myb tumor genes.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.
52) a compound wherein:
k is 0 to 15,
m is 0 or 1,
n is 8 to 29,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 14 to 19 bases complementary to a part of a region of 30 nucleotides which is around a criterional nucleotide positioning less than 5 nucleotides from the single base site in ras and c-myb tumor genes.

[Group $\alpha$]

Hydroxyl group, alkyl group having 1 to 6 carbon atoms, alkoxy group having 1 to 6 carbon atoms. methylenedioxy group, nitro group, azide group, halogen atom, aryl group having 6 to 10 carbon atoms,

aryloxy group having 6 to 10 carbon atoms and aralkyloxy group with an aryl moiety having 6 to 10 carbon atoms and an alkyl moiety having 1 or 2 carbon atoms.

53) a compound wherein:

k is 0 to 12,

m is 0,

n is 13 to 18,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence of 14 to 19 bases complementary to a part of a region of 30 nucleotides which is around a criterional nucleotide positioning less than 5 nucleotides from the single base site in ras and c-myb tumor genes.

[Group $\alpha$]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

54) a compound wherein:

k is 0 to 12,

m is 0 or 1,

n is 13 to 18,

X is a hydroxyl group,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence of 14 to 19 bases complementary to a part of a region of 30 nucleotides which is around a criterional nucleotide positioning less than 5 nucleotides from the single base site in ras and c-myb tumor genes.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

55) a compound wherein:

k is 0 to 12,

m is 0 or 1,

n is 13 to 18,

X is a hydroxyl group,

Y is an oxygen atom,

Z is an oxygen atom,

$R^1$, $R^2$, and $R^3$ may be the same or different from one another and each represent a hydrogen atom, an alkyl group having 1 to 4 carbon atoms, or an aryl group having 6 to 10 carbon atoms that may have a substituent selected from the following group $\alpha'$ and,

the base sequence containing B is a base sequence of 14 to 19 bases complementary to a portion of a region of 30 nucleotides which is around a criterional nucleotide positioning less than 5 nucleotides from the single base site in ras and c-myb tumor genes.

[Group α']

Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.

56) a compound wherein:
k is 0 to 12,
m is 0 or 1,
n is 13 to 18,
X is a hydroxyl group,
Y is an oxygen atom,
Z is an oxygen atom,
$R^1$, $R^2$, and $R^3$ may be the same or different from one another and each represent a hydrogen atom, an alkyl group having 1 to 4 carbon atoms, or a phenyl group that may have a substituent selected from the following group α'' and,
the base sequence containing B is a base sequence of 14 to 19 bases complementary to a part of a region of 30 nucleotides which is around a criterional nucleotide positioning less than 5 nucleotides from the single base site in ras and c-myb tumor genes.

[Group α'']

Methyl group, ethyl group, propyl group, t-butyl group, methoxy group, ethoxy group, propoxy group, t-butoxy group and phenylmethyloxy group.

57) a compound wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is AGGTGGGTCTGAAAC.

[Group α''']

Methoxy group, ethoxy group, propoxy group and t-butoxy group.

58) a compound wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group a''' and,
the base sequence is TCGGGGTTGGGAGGT.

[Group α''']

Methoxy group, ethoxy group, propoxy group and t-butoxy group.

59) a compound wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TTGGGAGGTGGGTCT.

[Group α''']

Methoxy group, ethoxy group, propoxy group and t-butoxy group.

60) a compound wherein:

k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is ATACTCAGTCATTTTTAGCAG.

[Group α''']

Methoxy group, ethoxy group, propoxy group and t-butoxy group.

61) a compound wherein:

k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is GTGCCGGGGTCTTCGGGC.

[Group α''']

Methoxy group.

62) a compound wherein:

k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGTCTGAAACGAT.

[Group α''']

Methoxy group, ethoxy group, propoxy group and t-butoxy group.

63) a compound wherein:

k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TAGGTGGGTCTGAAAC.

[Group α''']

Methoxy group.

64) a compound wherein:

k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,

the base sequence is GGTGGGTCTGAAACG.

[Group α''']

Methoxy group, ethoxy group, propoxy group and t-butoxy group.

65) a compound wherein:

k is 0,

m is 0,

X is a hydroxyl group,

Z is an oxygen atom,

$R^1$, $R^2$ and $R^3$ may be the same or different from one another and each represent a phenyl group that may have a substituent selected from the following group α''' and,

the base sequence is GGTGGGTTGCTTTGA.

[Group α''']

Methoxy group, ethoxy group, propoxy group and t-butoxy group.

66) a compound wherein:

k is 0,

m is 0,

X is a hydroxyl group,

Z is an oxygen atom,

$R^1$, $R^2$ and $R^3$ may be the same or different from one another and each represent a phenyl group that may have a substituent selected from the following group α''' and,

the base sequence is GGAGGTGGGTCTGAA.

[Group α''']

Methoxy group, ethoxy group, propoxy group and t-butoxy group.

67) a compound wherein:

k is 0,

m is 0,

X is a hydroxyl group,

Z is an oxygen atom,

$R^1$, $R^2$ and $R^3$ may be the same or different from one another and each represent a phenyl group that may have a substituent selected from the following group α''' and,

the base sequence is GGGAGGTGGGTCTGA.

[Group α''']

Methoxy group, ethoxy group, propoxy group and t-butoxy group.

68) a compound wherein:

k is 0,

m is 0,

X is a hydroxyl group,

Z is an oxygen atom,

$R^1$, $R^2$ and $R^3$ may be the same or different from one another and each represent a phenyl group that may have a substituent selected from the following group α''' and,

the base sequence is GTTGGGAGGTGGGTC.

[Group α''']

Methoxy group.

69) a compound wherein:

k is 0,

m is 0,

X is a hydroxyl group,

Z is an oxygen atom,

$R^1$, $R^2$ and $R^3$ may be the same or different from one another and each represent a phenyl group that may have a substituent selected from the following group α''' and,
the base sequence is GGGTTGGGAGGTGGG.

[Group α''']

Methoxy group, ethoxy group, propoxy group and t-butoxy group.
70) a compound wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGGGTCTG.

[Group α''']

Methoxy group, ethoxy group, propoxy group and t-butoxy group.
71) a compound wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGGGTCT.

[Group α''']

Methoxy group, ethoxy group, propoxy group and t-butoxy group.
72) a compound wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGGGTC.

[Group α''']

Methoxy group, ethoxy group, propoxy group and t-butoxy group.
73) a compound wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGGGT.

[Group α''']

Methoxy group, ethoxy group, propoxy group and t-butoxy group.
74) a compound wherein:
k is 0,
m is 0,

X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGGG.

[Group α''']

Methoxy group, ethoxy group, propoxy group and t-butoxy group.

75) a compound wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGG.

[Group α''']

Methoxy group, ethoxy group, propoxy group and t-butoxy group.

76) a compound wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTG.

[Group α''']

Methoxy group, ethoxy group, propoxy group and t-butoxy group.

Preferred compounds of the present invention specifically include those listed in the following Table.

In the Table, Ph represents a phenyl group, Me represents a methyl group, Et represents an ethyl group, Pr represents a propyl group, ipr represents an isopropyl group, tBu represents a tert-butyl group, MDO represents a 3,4-methylenedioxy group, By represents a phenylmethyl group, NaPh represents a naphthyl group, NaPhme represents a naphthylmethyl group, and dByoBy represents a (3,5-dibenzyloxy)-benzyl group.

In the nucleotide sequence portion in the formula (I),
[1] represents AGGTGGGTCTGAAAC (in the text, it is abbreviated as ODN-1),
[2] represents TCGGGGTTGGGAGGT (in the text, it is abbreviated as ODN-2)
[3] represents TTGGGAGGTGGGTCT (in the text, it is abbreviated as ODN-3)
[4] represents ATACTCAGTCATTTTTAGCAG (in the text, it is abbreviated as ODN-4)
[5] represents GTGCCGGGGTCTTCGGGC (in the text, it is abbreviated as ODN-5)
[6] represents TGGGTCTGAAACGAT (in the text, it is abbreviated as ODN-6)
[7] represents TAGGTGGGTCTGAAAC (in the text, it is abbreviated as ODN-7)
[8] represents GGTGGGTCTGAAACG (in the text, it is abbreviated as ODN-8)
[9] represents GGTGGGTTGCTTTGA (in the text, it is abbreviated as ODN-9)
[10] represents GGAGGTGGGTCTGAA (in the text, it is abbreviated as ODN-10)
[11] represents GGGAGGTGGGTCTGA (in the text, it is abbreviated as ODN-11)
[12] represents GTTGGGAGGTGGGTC (in the text, it is abbreviated as ODN-12)
[13] represents GGGTTGGGAGGTGGG (in the text, it is abbreviated as ODN-13)
[14] represents TGGGAGGTGGGTCTG (in the text, it is abbreviated as ODN-14)
[15] represents TGGGAGGTGGGTCT (in the text, it is abbreviated as ODN-15)
[a] represents TGGGAGGTGGGTC (in the text, it is abbreviated as ODN-16)
[b] represents TGGGAGGTGGGT (in the text, it is abbreviated as ODN-17)
[c] represents TGGGAGGTGGG (in the text, it is abbreviated as ODN-18)

[d] represents TGGGAGGTGG (in the text, it is abbreviated as ODN-19)
[e] represents TGGGAGGTG (in the text, it is abbreviated as ODN-20)

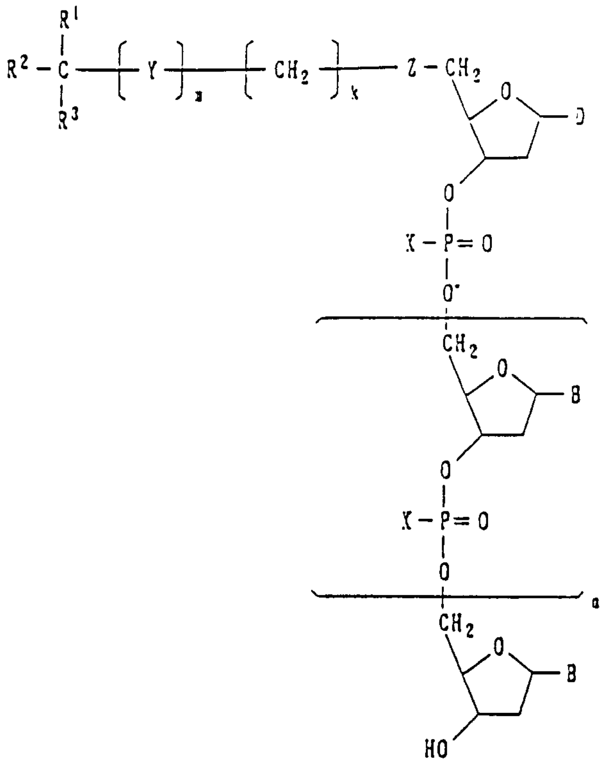

| No. Sequence | k | m | n | X | Y | Z | $R^1$ | $R^2$ | $R^3$ | |
|---|---|---|---|---|---|---|---|---|---|---|
| 1 | 0 | 0 | 13 | OH | - | O | 4-MeOPh | 4-MeOPh | Ph | [14] |
| 2 | 0 | 0 | 13 | OH | - | O | 3-MeOPh | 3-MeOPh | Ph | [14] |
| 3 | 0 | 0 | 13 | OH | - | O | 4-MeOPh | Ph | Ph | [14] |
| 4 | 0 | 0 | 13 | OH | - | O | 3-MeOPh | Ph | Ph | [14] |
| 5 | 0 | 0 | 13 | OH | - | O | 2-MeOPh | Ph | Ph | [14] |
| 6 | 0 | 0 | 13 | OH | - | O | Ph | Ph | Ph | [14] |
| 7 | 0 | 0 | 13 | OH | - | O | 4-EtOPh | 4-EtOPh | Ph | [14] |
| 8 | 0 | 0 | 13 | OH | - | O | 3-EtOPh | 3-EtOPh | Ph | [14] |
| 9 | 0 | 0 | 13 | OH | - | O | 2-EtOPh | 2-EtOPh | Ph | [14] |
| 10 | 0 | 0 | 13 | OH | - | O | 4-tBuOPh | Ph | Ph | [14] |
| 11 | 0 | 0 | 13 | OH | - | O | 3-tBuOPh | PH | Ph | [14] |
| 12 | 0 | 0 | 13 | OH | - | O | 2-tBuOPh | Ph | Ph | [14] |

| | | | | | | | | | | |
|---|---|---|---|---|---|---|---|---|---|---|
| 13 | 0 | 0 | 13 | OH | - | O | 4-tBuOPh | 4-tBuOPh | Ph | [14] |
| 14 | 0 | 0 | 13 | OH | - | O | 3-tBuOPh | 4-tBuOPh | Ph | [14] |
| 15 | 0 | 0 | 13 | OH | - | O | 2-tBuOPh | 4-tBuOPh | Ph | [14] |
| 16 | 0 | 0 | 13 | OH | - | O | 4-EtPh | 4-EtPh | 4-EtPh | [14] |
| 17 | 0 | 0 | 13 | OH | - | O | 3-EtPh | 3-EtPh | 3-EtPh | [14] |
| 18 | 0 | 0 | 13 | OH | - | O | 2-EtPh | 2-EtPh | 2-EtPh | [14] |
| 19 | 0 | 0 | 13 | OH | - | O | 4-iPrOPh | 4-iPrOPh | 4-iPrOPh | [14] |
| 20 | 0 | 0 | 13 | OH | - | O | 3-iPrOPh | 3-iPrOPh | 3-iPrOPh | [14] |
| 21 | 0 | 0 | 13 | OH | - | O | 4-tBuPh | 4-tBuPh | Ph | [14] |
| 22 | 0 | 0 | 13 | OH | - | O | 2-tBuPh | 2-tBuPh | Ph | [14] |
| 23 | 0 | 0 | 13 | OH | - | O | MDOPh | MDOPh | Ph | [14] |
| 24 | 0 | 0 | 13 | OH | - | O | 4-$NO_2$Ph | 4-$NO_2$Ph | Ph | [14] |
| 25 | 0 | 0 | 13 | OH | - | O | 3-$NO_2$Ph | 3-$NO_2$Ph | Ph | [14] |
| 26 | 0 | 0 | 13 | OH | - | O | 4-$N_3$Ph | Ph | Ph | [14] |
| 27 | 0 | 0 | 13 | OH | - | O | 2-NaPh | Ph | Ph | [14] |
| 28 | 0 | 0 | 13 | OH | - | O | 4-$N_3$Ph | 4-$N_3$Ph | Ph | [14] |
| 29 | 0 | 0 | 13 | OH | - | O | 3-$N_3$Ph | 4-$N_3$Ph | Ph | [14] |
| 30 | 0 | 0 | 13 | OH | - | O | 4-BrPh | 4-BrPh | 4-BrPh | [14] |
| 31 | 0 | 0 | 13 | OH | - | O | 3-BrPh | 3-BrPh | 3-BrPh | [14] |
| 32 | 0 | 0 | 13 | OH | - | O | Ph | Ph | H | [14] |
| 33 | 0 | 0 | 13 | OH | - | O | Ph | H | H | [14] |
| 34 | 0 | 0 | 13 | OH | - | O | 1-NaPhme | H | H | [14] |
| 35 | 0 | 0 | 13 | OH | - | O | 2-NaPhme | H | H | [14] |
| 36 | 0 | 0 | 13 | OH | - | O | 4-BrPh | Ph | H | [14] |
| 37 | 0 | 0 | 13 | OH | - | O | 3-BrPh | Ph | H | [14] |
| 38 | 0 | 0 | 13 | OH | - | O | 4-IPh | 4-IPh | Ph | [14] |
| 39 | 0 | 0 | 13 | OH | - | O | 2-IPh | 2-IPh | Ph | [14] |

| | | | | | | | | | | |
|---|---|---|---|---|---|---|---|---|---|---|
| 40 | 0 | 0 | 13 | OH | - | O | 4-ByOPh | 4-ByOPh | 4-ByOPh | [14] |
| 41 | 0 | 0 | 13 | OH | - | O | 3-ByOPh | 3-ByOPh | 3-ByOPh | [14] |
| 42 | 0 | 0 | 13 | OH | - | O | 2-ByOPh | 2-ByOPh | 2-ByOPh | [14] |
| 43 | 0 | 0 | 13 | OH | - | O | dByOBy | H | H | [14] |
| 44 | 0 | 0 | 13 | OH | - | O | dByOBy | dByOBy | H | [14] |
| 45 | 0 | 0 | 13 | OH | - | O | Bu | $CH_3$ | $CH_3$ | [14] |
| 46 | 0 | 0 | 13 | OH | - | O | Bu | Pr | $CH_3$ | [14] |
| 47 | 0 | 0 | 13 | OH | - | O | Bu | Bu | Et | [14] |
| 48 | 0 | 0 | 13 | OH | - | S | 4-MeOPh | 4-MeOPh | Ph | [14] |
| 49 | 0 | 0 | 13 | OH | - | S | 3-MeOPh | 3-MeOPh | Ph | [14] |
| 50 | 0 | 0 | 13 | OH | - | S | 2-MeOPh | 2-MeOPh | Ph | [14] |
| 51 | 0 | 0 | 13 | OH | - | S | 4-MeOPh | Ph | Ph | [14] |
| 52 | 0 | 0 | 13 | OH | - | S | 3-MeOPh | Ph | Ph | [14] |
| 53 | 0 | 0 | 13 | OH | - | S | 3-MeOPh | Ph | Ph | [14] |
| 54 | 0 | 0 | 13 | OH | - | S | Ph | Ph | Ph | [14] |
| 55 | 0 | 0 | 13 | OH | - | S | 4-PrOPh | 4-PrOPh | Ph | [14] |
| 56 | 0 | 0 | 13 | OH | - | S | 3-PrOPh | 3-PrOPh | Ph | [14] |
| 57 | 0 | 0 | 13 | OH | - | S | 2-PrOPh | 2-PrOPh | Ph | [14] |
| 58 | 0 | 0 | 13 | OH | - | S | 4-tBuPh | 4-tBuPh | Ph | [14] |
| 59 | 0 | 0 | 13 | OH | - | S | 3-tBuPh | 4-tBuPh | Ph | [14] |
| 60 | 0 | 0 | 13 | OH | - | S | 2-tBuPh | 2-tBuPh | Ph | [14] |
| 61 | 0 | 0 | 13 | OH | - | S | MDOPh | MDOPh | Ph | [14] |
| 62 | 0 | 0 | 13 | OH | - | S | Ph | Ph | H | [14] |
| 63 | 0 | 0 | 13 | OH | - | S | Ph | H | H | [14] |
| 64 | 0 | 0 | 13 | OH | - | S | 4-$NO_2$Ph | 4-$NO_2$Ph | Ph | [14] |
| 65 | 0 | 0 | 13 | OH | - | S | 3-$NO_2$Ph | 3-$NO_2$Ph | Ph | [14] |
| 66 | 0 | 0 | 13 | OH | - | S | 2-$NO_2$Ph | 2-$NO_2$Ph | Ph | [14] |

| | | | | | | | | | | |
|---|---|---|---|---|---|---|---|---|---|---|
| 67 | 0 | 0 | 13 | OH | - | S | 4-IPh | 4-IPh | Ph | [14] |
| 68 | 0 | 0 | 13 | OH | - | S | 3-IPh | 3-IPh | Ph | [14] |
| 69 | 0 | 0 | 13 | OH | - | S | tBu | tBu | $CH_3$ | [14] |
| 70 | 0 | 0 | 13 | SH | - | O | 4-MePh | 4-MePh | Ph | [14] |
| 71 | 0 | 0 | 13 | SH | - | O | 3-MePh | 3-MePh | Ph | [14] |
| 72 | 0 | 0 | 13 | SH | - | O | 4-MeOPh | Ph | Ph | [14] |
| 73 | 0 | 0 | 13 | SH | - | O | 3-MeOPh | Ph | Ph | [14] |
| 74 | 0 | 0 | 13 | SH | - | O | 2-MeOPh | Ph | Ph | [14] |
| 75 | 0 | 0 | 13 | SH | - | O | Ph | Ph | Ph | [14] |
| 76 | 0 | 0 | 13 | SH | - | O | 4-PrOPh | 4-PrOPh | Ph | [14] |
| 77 | 0 | 0 | 13 | SH | - | O | 2-PrOPh | 2-PrOPh | Ph | [14] |
| 78 | 0 | 0 | 13 | SH | - | O | 4-tBuPh | 4-tBuPh | Ph | [14] |
| 79 | 0 | 0 | 13 | SH | - | O | 2-tBuPh | 2-tBuPh | Ph | [14] |
| 80 | 0 | 0 | 13 | SH | - | O | MDOPh | MDOPh | Ph | [14] |
| 81 | 0 | 0 | 13 | SH | - | O | Ph | Ph | H | [14] |
| 82 | 0 | 0 | 13 | SH | - | O | Ph | H | H | [14] |
| 83 | 0 | 0 | 13 | SH | - | O | 4-$NO_2$Ph | 4-$NO_2$Ph | Ph | [14] |
| 84 | 0 | 0 | 13 | SH | - | O | 3-$NO_2$Ph | 3-$NO_2$Ph | Ph | [14] |
| 85 | 0 | 0 | 13 | SH | - | O | 4-IPh | 4-IPh | Ph | [14] |
| 86 | 0 | 0 | 13 | SH | - | O | 2-IPh | 2-IPh | Ph | [14] |
| 87 | 0 | 0 | 13 | SH | - | O | tBu | tBu | $CH_3$ | [14] |
| 88 | 0 | 0 | 13 | $CH_3$ | - | O | 4-MeOPh | 4-MePh | Ph | [14] |
| 89 | 0 | 0 | 13 | $CH_3$ | - | O | 3-MeOPh | 3-MePh | Ph | [14] |
| 90 | 0 | 0 | 13 | $CH_3$ | - | O | Ph | Ph | 4-MeOPh | [14] |
| 91 | 0 | 0 | 13 | $CH_3$ | - | O | Ph | Ph | Ph | [14] |
| 92 | 0 | 0 | 13 | $CH_3$ | - | O | 4-EtOPh | 4-EtOPh | Ph | [14] |
| 93 | 0 | 0 | 13 | $CH_3$ | - | O | 2-EtOPh | 2-EtOph | Ph | [14] |

| | | | | | | | | | | |
|---|---|---|---|---|---|---|---|---|---|---|
| 94 | 0 | 0 | 13 | $CH_3$ | - | O | 4-tBuOPh | Ph | Ph | [14] |
| 95 | 0 | 0 | 13 | $CH_3$ | - | O | 3-tBuOPh | Ph | Ph | [14] |
| 96 | 0 | 0 | 13 | $CH_3$ | - | O | 4-tBuOPh | 4-tBuOPh | Ph | [14] |
| 97 | 0 | 0 | 13 | $CH_3$ | - | O | 3-tBuOPh | 3-tBuOPh | Ph | [14] |
| 98 | 0 | 0 | 13 | $CH_3$ | - | O | 4-EtPh | 4-EtPh | 4-EtPh | [14] |
| 99 | 0 | 0 | 13 | $CH_3$ | - | O | 3-EtPh | 3-EtPh | 4-EtPh | [14] |
| 100 | 0 | 0 | 13 | $CH_3$ | - | O | 2-EtPh | 2-EtPh | 4-EtPh | [14] |
| 101 | 0 | 0 | 13 | $CH_3$ | - | O | 4-iPrOPh | 4-iPrOPh | 4-iPrOPh | [14] |
| 102 | 0 | 0 | 13 | $CH_3$ | - | O | 2-iPrOPh | 2-iPrOPh | 4-iPrOPh | [14] |
| 103 | 0 | 0 | 13 | PrO | - | O | 4-tBuPh | 4-tBuPh | Ph | [14] |
| 104 | 0 | 0 | 13 | PrO | - | O | 2-tBuPh | 2-tBuPh | Ph | [14] |
| 105 | 0 | 0 | 13 | PrO | - | O | MDOPh | MDOPh | Ph | [14] |
| 106 | 0 | 0 | 13 | PrO | - | O | 4-$NO_2$Ph | 4-$NO_2$Ph | Ph | [14] |
| 107 | 0 | 0 | 13 | PrO | - | O | 3-$NO_2$Ph | 3-$NO_2$Ph | Ph | [14] |
| 108 | 0 | 0 | 13 | PrO | - | O | 4-$N_3$Ph | Ph | Ph | [14] |
| 109 | 0 | 0 | 13 | PrO | - | O | 2-$N_3$Ph | Ph | Ph | [14] |
| 110 | 0 | 0 | 13 | PrO | - | O | 4-$N_3$Ph | 4-$N_3$Ph | Ph | [14] |
| 111 | 0 | 0 | 13 | PrO | - | O | 2-$N_3$Ph | 2-$N_3$Ph | Ph | [14] |
| 112 | 0 | 0 | 13 | PrO | - | O | 3-$N_3$Ph | 3-$N_3$Ph | Ph | [14] |
| 113 | 0 | 0 | 13 | PrO | - | O | 4-BrPh | 4-BrPh | 4-BrPh | [14] |
| 114 | 0 | 0 | 13 | PrO | - | O | 3-BrPh | 3-BrPh | 4-BrPh | [14] |
| 115 | 0 | 0 | 13 | PrO | - | O | 2-BrPh | 2-BrPh | 4-BrPh | [14] |
| 116 | 0 | 0 | 13 | PrO | - | O | Ph | Ph | H | [14] |
| 117 | 0 | 0 | 13 | PrO | - | O | Ph | H | H | [14] |
| 118 | 0 | 0 | 13 | BuO | - | O | 4-ClPh | Ph | H | [14] |
| 119 | 0 | 0 | 13 | BuO | - | O | 3-ClPh | Ph | H | [14] |
| 120 | 0 | 0 | 13 | BuO | - | O | 2-ClPh | Ph | H | [14] |

| | | | | | | | | | | |
|---|---|---|---|---|---|---|---|---|---|---|
| 121 | 0 | 0 | 13 | BuO | - | O | 4-BrPh | Ph | H | [14] |
| 122 | 0 | 0 | 13 | BuO | - | O | 3-BrPh | Ph | H | [14] |
| 123 | 0 | 0 | 13 | BuO | - | O | 4-IPh | 4-IPh | Ph | [14] |
| 124 | 0 | 0 | 13 | BuO | - | O | 2-IPh | 2-IPh | Ph | [14] |
| 125 | 0 | 0 | 13 | BuO | - | O | 4-ByOPh | 4-ByOPh | 4-ByOPh | [14] |
| 126 | 0 | 0 | 13 | BuO | - | O | 3-ByOPh | 3-ByOPh | 3-ByOPh | [14] |
| 127 | 0 | 0 | 13 | BuO | - | O | 2-ByOPh | 2-ByOPh | 2-ByOPh | [14] |
| 128 | 0 | 0 | 13 | EtNH | - | O | 4-MeOPh | 4-MeOPh | Ph | [14] |
| 129 | 0 | 0 | 13 | EtNH | - | O | 2-MeOPh | 2-MeOPh | Ph | [14] |
| 130 | 0 | 0 | 13 | EtNH | - | O | 4-MeOPh | Ph | Ph | [14] |
| 131 | 0 | 0 | 13 | EtNH | - | O | 3-MeOPh | Ph | Ph | [14] |
| 132 | 0 | 0 | 13 | EtNH | - | O | Ph | Ph | Ph | [14] |
| 133 | 0 | 0 | 13 | EtNH | - | O | 4-PrOPh | 4-PrOPh | Ph | [14] |
| 134 | 0 | 0 | 13 | EtNH | - | O | 3-PrOPh | 3-PrOPh | Ph | [14] |
| 135 | 0 | 0 | 13 | EtNH | - | O | 4-tBuOPh | 4-tBuOPh | Ph | [14] |
| 136 | 0 | 0 | 13 | EtNH | - | O | 2-tBuOPh | 2-tBuOPh | Ph | [14] |
| 137 | 0 | 0 | 13 | EtNH | - | O | MDOPh | MDOPh | Ph | [14] |
| 138 | 0 | 0 | 13 | EtNH | - | O | Ph | Ph | H | [14] |
| 139 | 0 | 0 | 13 | EtNH | - | O | Ph | H | H | [14] |
| 140 | 0 | 0 | 13 | PrNH | - | O | 4-$NO_2$Ph | 4-$NO_2$Ph | Ph | [14] |
| 141 | 0 | 0 | 13 | PrNH | - | O | 3-$NO_2$Ph | 3-$NO_2$Ph | Ph | [14] |
| 142 | 0 | 0 | 13 | PrNH | - | O | 4-IPh | 4-IPh | Ph | [14] |
| 143 | 0 | 0 | 13 | PrNH | - | O | 2-IPh | 2-IPh | Ph | [14] |
| 144 | 0 | 0 | 13 | PrNH | - | O | 4-tBuPh | tBu | $CH_3$ | [14] |
| 145 | 0 | 0 | 13 | PrNH | - | O | 3-tBuPh | tBu | $CH_3$ | [14] |
| 146 | 15 | 0 | 13 | OH | - | S | H | H | H | [14] |
| 147 | 12 | 0 | 13 | OH | - | S | Ph | Ph | Ph | [14] |

| | | | | | | | | | | |
|---|---|---|---|---|---|---|---|---|---|---|
| 148 | 6 | 0 | 13 | OH | – | S | Ph | Ph | H | [14] |
| 149 | 6 | 0 | 13 | OH | – | S | PH | Ph | 4-MeOPh | [14] |
| 150 | 6 | 0 | 13 | OH | – | S | Ph | Ph | 2-MeOPh | [14] |
| 151 | 15 | 0 | 13 | SH | – | S | H | H | H | [14] |
| 152 | 12 | 1 | 13 | OH | O | S | Ph | Ph | Ph | [14] |
| 153 | 12 | 1 | 13 | OH | O | S | 4-MeOPh | 4-MeOPh | Ph | [14] |
| 154 | 12 | 1 | 13 | OH | O | S | 3-MeOPh | 3-MeOPh | Ph | [14] |
| 155 | 10 | 1 | 13 | OH | O | S | Ph | Ph | H | [14] |
| 156 | 10 | 1 | 13 | SH | O | S | Ph | Ph | Ph | [14] |
| 157 | 6 | 1 | 13 | SH | O | S | Ph | Ph | 4-MeOPh | [14] |
| 158 | 6 | 1 | 13 | SH | O | S | Ph | Ph | 3-MeOPh | [14] |
| 159 | 6 | 1 | 13 | SH | S | S | Ph | Ph | H | [14] |
| 160 | 12 | 1 | 13 | $CH_3$ | O | S | Ph | Ph | Ph | [14] |
| 161 | 12 | 1 | 13 | $CH_3$ | O | S | 4-BuPh | 4-BuPh | Ph | [14] |
| 162 | 12 | 1 | 13 | $CH_3$ | O | S | 3-BuPh | 3-BuPh | Ph | [14] |
| 163 | 12 | 1 | 13 | OBu | O | S | Ph | Ph | Ph | [14] |
| 164 | 12 | 1 | 13 | OMe | O | S | Ph | Ph | H | [14] |
| 165 | 12 | 1 | 13 | OMe | O | S | Ph | Ph | Ph | [14] |
| 166 | 6 | 1 | 13 | NHBu | O | S | Ph | Ph | Ph | [14] |
| 167 | 6 | 1 | 13 | NHBu | O | S | Ph | Ph | Ph | [14] |
| 168 | 6 | 1 | 13 | SH | O | S | 4-MeOPh | 4-MeOPh | Ph | [14] |
| 169 | 6 | 1 | 13 | SH | O | S | 2-MeOPh | 2-MeOPh | Ph | [14] |
| 170 | 0 | 0 | 13 | OH | – | O | 4-MeOPh | 4-MeOPh | Ph | [1] |
| 171 | 0 | 0 | 13 | OH | – | O | 4-MeOPh | 4-MeOPh | Ph | [2] |
| 172 | 0 | 0 | 13 | OH | – | O | 4-MeOPh | 4-MeOPh | Ph | [3] |
| 173 | 0 | 0 | 13 | OH | – | O | 4-MeOPh | 4-MeOPh | Ph | [4] |
| 174 | 0 | 0 | 13 | OH | – | O | 4-MeOPh | 4-MeOPh | Ph | [5] |

| | | | | | | | | | | |
|---|---|---|---|---|---|---|---|---|---|---|
| 175 | 0 | 0 | 13 | OH | – | O | 4-MeOPh | 4-MeOPh | Ph | [6] |
| 176 | 0 | 0 | 13 | OH | – | O | 4-MeOPh | 4-MeOPh | Ph | [7] |
| 177 | 0 | 0 | 13 | OH | – | O | 4-MeOPh | 4-MeOPh | Ph | [8] |
| 178 | 0 | 0 | 13 | OH | – | O | 4-MeOPh | 4-MeOPh | Ph | [9] |
| 179 | 0 | 0 | 13 | OH | – | O | 4-MeOPh | 4-MeOPh | Ph | [10] |
| 180 | 0 | 0 | 13 | OH | – | O | 4-MeOPh | 4-MeOPh | Ph | [11] |
| 181 | 0 | 0 | 13 | OH | – | O | 4-MeOPh | 4-MeOPh | Ph | [12] |
| 182 | 0 | 0 | 13 | OH | – | O | 4-MeOPh | 4-MeOPh | Ph | [13] |
| 183 | 0 | 0 | 13 | OH | – | O | Ph | Ph | Ph | [1] |
| 184 | 0 | 0 | 13 | OH | – | O | Ph | Ph | Ph | [2] |
| 185 | 0 | 0 | 13 | OH | – | O | Ph | Ph | Ph | [3] |
| 186 | 0 | 0 | 13 | OH | – | O | Ph | Ph | Ph | [4] |
| 187 | 0 | 0 | 13 | OH | – | O | Ph | Ph | Ph | [5] |
| 188 | 0 | 0 | 13 | OH | – | O | Ph | Ph | Ph | [6] |
| 189 | 0 | 0 | 13 | OH | – | O | Ph | Ph | Ph | [7] |
| 190 | 0 | 0 | 13 | OH | – | O | Ph | Ph | Ph | [8] |
| 191 | 0 | 0 | 13 | OH | – | O | Ph | Ph | Ph | [9] |
| 192 | 0 | 0 | 13 | OH | – | O | Ph | Ph | Ph | [10] |
| 193 | 0 | 0 | 13 | OH | – | O | Ph | Ph | Ph | [11] |
| 194 | 0 | 0 | 13 | OH | – | O | Ph | Ph | Ph | [12] |
| 195 | 0 | 0 | 13 | OH | – | O | Ph | Ph | Ph | [13] |
| 196 | 0 | 0 | 13 | OH | – | O | 4-MeOPh | 4-MeOPh | Ph | [15] |
| 197 | 0 | 0 | 13 | OH | – | O | 4-MeOPh | 4-MeOPh | Ph | [a] |
| 198 | 0 | 0 | 13 | OH | – | O | 4-MeOPh | 4-MeOPh | Ph | [b] |
| 199 | 0 | 0 | 13 | OH | – | O | 4-MeOPh | 4-MeOPh | Ph | [c] |
| 200 | 0 | 0 | 13 | OH | – | O | 4-MeOPh | 4-MeOPh | Ph | [d] |
| 201 | 0 | 0 | 13 | OH | – | O | 4-MeOPh | 4-MeOPh | Ph | [e] |

| | | | | | | | | | | |
|---|---|---|---|---|---|---|---|---|---|---|
| 202 | 0 | 0 | 13 | OH | - | O | Ph | Ph | Ph | [15] |
| 203 | 0 | 0 | 13 | OH | - | O | Ph | Ph | Ph | [a] |
| 204 | 0 | 0 | 13 | OH | - | O | Ph | Ph | Ph | [b] |
| 205 | 0 | 0 | 13 | OH | - | O | Ph | Ph | Ph | [c] |
| 206 | 0 | 0 | 13 | OH | - | O | Ph | Ph | Ph | [d] |
| 207 | 0 | 0 | 13 | OH | - | O | Ph | Ph | Ph | [e] |

More preferred compounds are 1, 3, 6, 20 32, 33, 35, 43, 45, 46, 47, 48, 54, 62, 69, 70, 75, 81, 82, 87, 88, 91, 128, 132, 147, 153, 157, 159, 160, 165, 166, 168, 170, 171, 172, 173, 175, 180, 181, 182, 183, 196, 197, 198, 199, 200, and 201.

The most preferred compounds are:
5'-O-(4,4'-dimethoxytrityl)-TGGGAGGTGGGTCTG (Exemplary compound 1),
5'-O-(4-methoxytrityl)-TGGGAGGTGGGTCTG (Exemplary compound 3),
5'-O-trityl-TGGGAGGTGGGTCTG (Exemplary compound 6),
5'-O-diphenylmethyl-TGGGAGGTGGGTCTG (Exemplary compound 32),
5'-O-phenylmethyl-TGGGAGGTGGGTCTG Exemplary compound 33),
5'-O-(2-naphthylmethyl)-TGGGAGGTGGGTCTG (Exemplary compound 35),
5'-O-[(3,5-dibenzyloxy)benzyl]-TGGGAGGTGGGTCTG (Exemplary compound 43),
5'-S-diphenylmethyl-TGGGAGGTGGGTCTG (Exemplary compound 62)
5'-S-(4,4'-dimethoxytrityl)-TGGGAGGTGGGTCTG (Exemplary compound 70),
5'-S-trityl-TGGGAGGTGGGTCTG (Exemplary compound 75),
5'-O-(4,4'-dimethoxytrityl)-AGGTGGGTCTGAAAC (Exemplary compound 170),
5'-O-(4,4'-dimethoxytrityl)-TCGGGGTTGGGAGGT (Exemplary compound 171),
5'-O-(4,4'-dimethoxytrityl)-TTGGGAGGTGGGTCT (Exemplary compound 172),
5'-O-(4,4'-dimethoxytrityl)-ATACTCAGTCATTTTTAGCAG (Exemplary compound 173),
5'-O-(4,4'-dimethoxytrityl)-TGGGTCTGAAACGAT (Exemplary compound 175),
5'-O-(4,4'dimethoxytrityl)-GGGAGGTGGGTCTGA (Exemplary compound 180),
5'-O-(4,4'-dimethoxytrityl)-GTTGGGAGGTGGGTC (Exemplary compound 181),
5'-O-(4,4'-dimethoxytrityl)-GGGTTGGGAGTGGGG (Exemplary compound 182), and
5'-O-trityl-TGGGAGGTGGGTCTG (Exemplary compound 183),
5'-O-(4,4'-dimethoxytrityl)-TGGGAGGTGGGTCT (Exemplary compound 196),
5'-O-(4,4'-dimethoxytrityl)-TGGGAGGTGGGTC (Exemplary compound 197),
5'-O-(4,4'-dimethoxytrityl)-TGGGAGGTGGGT (Exemplary compound 198),
5'-O-(4,4'-dimethoxytrityl)-TGGGAGGTGGG (Exemplary compound 199),
5'-O-(4,4'-dimethoxytrityl)-TGGGAGGTGG (Exemplary compound 200),
5'-O-(4,4'-dimethoxytrityl)-TGGGAGGTG (Exemplary compound 201).

The compound having the above-mentioned general formula (1) of the present invention can be used in the form of a salt. Examples of said salts include inorganic or organic salts including alkali metals such as sodium and potassium; alkaline earth metals such as calcium; ammonia; basic amino acids such as lysine and arginine; and, alkylamines such as triethylamine. Preferable examples of said salts are alkaline metals such as sodium and potassium.

The compound of general formula (1) of the present invention can be manufactured using Compound (2)

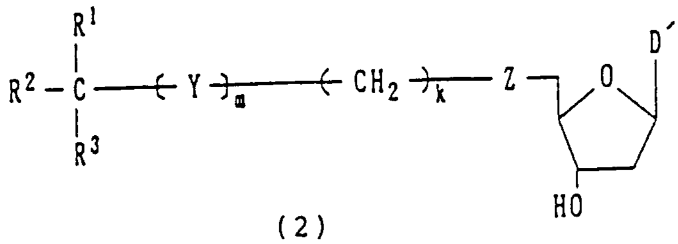

(2)

which is prepared according to Processes A-1, A-2, A-3 and A-4, Process B, C or D in the case where X in the above-mentioned general formula (1) is a hydroxyl group, Process E in the case where X in the above-mentioned general formula (1) is a methyl group, Process F in the case where X in the above-mentioned general formula (1) is a sulfhydryl group, Process G in the case where X in the above-mentioned general formula (1) is an alkoxy group having 1 to 6 carbon atoms, and Process H in the case where X in the above-mentioned general formula (1) is a monoalkylamino group having 1 to 6 carbon atoms.

In Compound (2), $R^1$, $R^2$, $R^3$, Y, Z, m and k have the same meanings as defined in the general formula (1), while D' represents a base selected from the following group $\beta$ or protected homologous base.

[Group $\beta$]

Adenine (A), guanine (G), cytosine (C) and thymine (T).

In Process B, (a) a 3'-phosphorous acid derivative prepared by reacting phosphitylating agent with Compound (2), and (b) [1] an oligodeoxynucleotide (hereinafter to be abbreviated as "ODN") coupled to controlled pore glass (hereinafter to be abbreviated as "CPG") from which only the 5'-terminal dimethoxytrityl group is eliminated following synthesis with a DNA synthesizer using a nucleotide wherein the basic portion and phosphate portion are protected with protecting group, or [2] ODN having a free hydroxyl group on its 5'-terminal, synthesized by liquid phase technique using a nucleotide wherein the basic portion and phosphate portion are protected with protecting groups, are (c) condensed using a condensing agent to form triphosphite bonds, followed by (d) oxidizing to triphosphate using an oxidizing agent or severing the ODN using ordinary techniques in the case of being coupled to CPG to eliminate the protecting groups, and (e) purifying by ordinary purification techniques to obtain the desired compound.

In Process C, (a) a 3' phosphorylating derivative prepared by reacting a phosphating agent with Compound (2), and (b) [1] an ODN coupled to CPG from which only the 5'-terminal dimethoxytrityl group is eliminated following synthesis with a DNA synthesizer using a nucleotide wherein the basic portion is protected with a protecting group, or [2] ODN having a free hydroxyl group on its 5'-terminal, synthesized by liquid phase technique using a nucleotide wherein the basic portion is protected with a protecting group, are (c) condensed using a condensing agent to form phosphodiester or phosphotriester bonds, and (d) eliminating the protecting groups other than the substituent bonded to the 5' carbon atoms of the 5'-terminal after severing the ODN using ordinary techniques in the case of being coupled to CPG, followed by (e) purifying by ordinary purification techniques to obtain the desired compound.

In Process D, (a) a 2'-deoxyphosphonic acid derivative, wherein if an amino group is present in the basic portion of the 2'-deoxynucleotide, the amino group is protected with a protecting group, the desired substituent is introduced to the 5'-position, and a phosphonate group is introduced to the 3'-position, and, (b) [1] an ODN coupled to CPG from which only the 5'-terminal dimethoxytrityl group is eliminated following synthesis with a DNA synthesizer using a nucleotide wherein the basic portion is protected with protecting groups such as an acyl group, or [2] ODN having a free hydroxyl group on its 5'-terminal is synthesized by liquid phase technique using a nucleotide wherein the basic portion is protected with protecting groups, are (c) reacted with acid halide in the presence of a base to form phosphodiester or phosphotriester bonds by using an oxidizing agent, followed by (d) eliminating the protecting groups after severing the ODN using ordinary techniques in the case of being coupled to CPG, and (e) purifying by ordinary purification techniques to obtain the desired compound.

The following provides a detailed description of Processes A to H.

In the reaction schemes of Processes A to H, $R^1$, $R^2$, $R^3$, Y, Z, m, k, B, D, n and D' have the same meanings as defined above, while Hal represents a halogen atom, B' represents a base selected from the following group $\beta$ or protected homologous base, $A^1$ represents a protecting group of a hydroxyl group or mercepto group, and $A^2$ represents a protecting group of a hydroxyl group.

[Group β]

Adenine (A), guanine (G), cytosine (C) and thymine (T).

[Process A]

This process is for preparing a nucleotide intermediate (2) to serve as the 5'-terminal of the compound of the present invention.

Process A-1

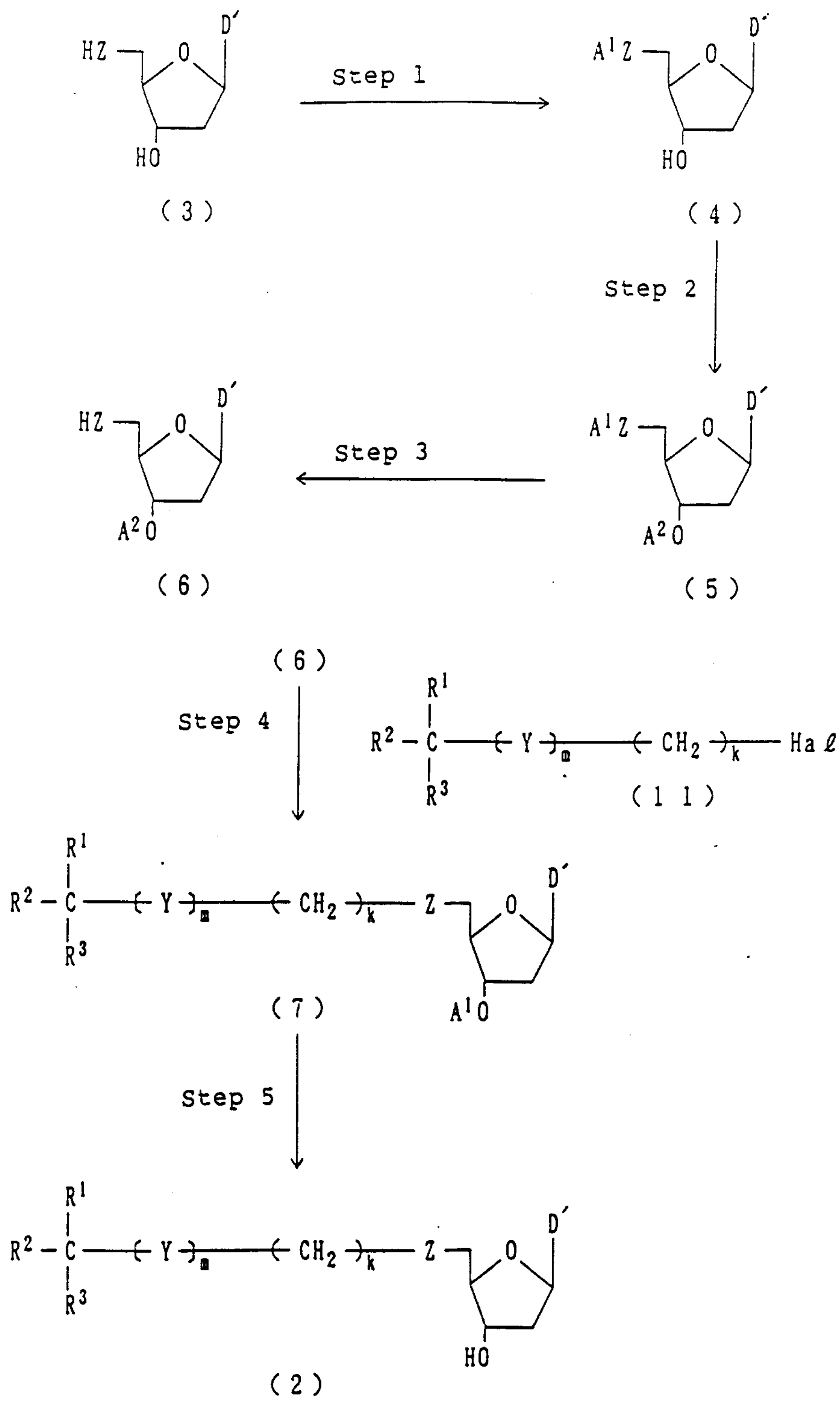


(3) (4) (5) (6) (6) (11) (7) (2)

Process A-1 is composed of Step 1 to Step 5.

(Step 1)

This step is for preparing Compound (4), in which only the hydroxyl group at the 5'-position is selectively protected by reacting a hydroxyl group protecting reagent with Compound (3) in an inactive

solvent (provided that the acylation protecting process for those amino groups present is included in the previous stage in the case the base portions are A, G and C. The protecting process can be easily carried out according to known method (J. Am. Chem. Soc., 104, 1316 (1982)). Lower aliphatic acyl or aromatic acyl groups are generally used for the protecting groups of the amino groups. Examples of those lower aliphatic acyl groups used include formyl, acetyl, propionyl, butyryl, isobutyryl, pentanoyl, pivaloyl, valeryl and isovaleryl groups, examples of those aromatic acyl groups include benzoyl, 4-acetoxybenzoyl, 4-methoxybenzoyl, 4-methylbenzoyl and 1-naphthoyl groups, while preferable examples include benzoyl groups in the case the base portion is A or C, and isobutyryl groups in the case the base portion is G).

The solvent employable includes preferably aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; ketones such as acetone, methyl ethyl ketone, methyl isobutyl ketone, isophorone and cyclohexanone; nitro compounds such as nitroethane and nitrobenzene; nitriles such as acetonitrile and isobutyronitrile; amides such as formamide, dimethylformamide, dimethylacetamide and hexamethylphosphorotriamide; sulfoxides such as dimethyl sulfoxide and sulfolane; aliphatic tertiary amines such as trimethylamine, triethylamine and N-methylmorpholine; and aromatic amines such as pyridine and picoline, more preferably halogenated hydrocarbons (particularly methylene chloride) and amides (particularly DMF).

Although there are no particular restrictions on the protecting reagent used, provided that it is able to selectively protect the 5'-position only and can be eliminated under acidic or neutral conditions, preferable examples include triarylmethyl halides such as trityl chloride, monomethoxytrityl chloride and dimethoxytrityl chloride.

Base is usually used in the case of using a triarylmethyl halide for the protecting reagent.

Examples of bases that are used include heterocyclic amines such as pyridine, dimethylaminopyridine and pyrrolidinopyridine, and aliphatic tertiary amines such as trimethylamine and triethylamine, preferably organic bases (particularly pyridine, dimethylaminopyridine and pyrrolidinopyridine).

In the case where organic amines are used as the solvent, there is no need to add another acid acceptor, since the organic amine itself functions as a acid acceptor.

While the reaction temperature varies depending on the starting material, reagent, solvent, etc. employed, it is usually 0 to 150 °C, and preferably 20 to 100 °C.

While the reaction time varies depending on the starting material, solvent and reaction temperature, it is usually 1 to 100 hours, and preferably 2 to 24 hours.

After completion of the reaction, for example, the solvent is distilled off and the reaction mixture is poured in water, made acidic with inorganic acids such as hydrochloric acid and sulfuric acid, extracted with a water-immiscible solvent such as benzene, ether and ethyl acetate, followed by evaporation of the solvent from the extract. The thus obtained product is usually used as such in the subsequent step. If desired, the product can be purified by isolation by various chromatographies or recrystallization.

(Step 2)

This step is for preparing Compound (5) by reacting Compound (4) with a protecting reagent for hydroxyl group in an inert solvent.

The solvent employable includes preferably aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; alcohols such as methanol, ethanol, n-propanol, isopropanol, n-butanol, isobutanol, t-butanol, isoamyl alcohol, diethylene glycol, glycerol, octanol, cyclohexanol and methyl cellosolve; ketones such as acetone, methyl ethyl ketone, methyl isobutyl ketone, isophorone and cyclohexanone; nitro compounds such as nitroethane and nitrobenzene; nitriles such as acetonitrile and isobutyronitrile; amides such as formamide, dimethylformamide, dimethylacetamide and hexamethylphosphorotriamide; sulfoxides such as dimethyl sulfoxide and sulfolane, and more preferably halogenated hydrocarbons (particularly methylene chloride), aromatic hydrocarbons (particularly toluene) and amides (particularly DMF).

The protecting reagent employed is not particularly limited as long as it can deprotect except the 5'-position, and includes preferably silyl halides such as t-butyldimethylsilyl chloride; haloalkoxycarbonyl halides such as trichloroethoxycarbonyl chloride; and aralkyloxycarbonyl halides such as benzyloxycarbonyl

chloride.

In the case where the silyl halides, haloalkoxycarbonyl halides and aralkyloxycarbonyl halides are used as the protecting reagent, bases are usually used.

The base employable includes preferably organic bases (particularly triethylamine, pyridine, N-methylmorpholine, DBU, etc.).

While the reaction temperature varies depending on the reagent, starting material, solvent, etc. employed, it is usually -20 to 150 ° C, and preferably -10 to 50 ° C.

While the reaction time varies depending on the starting material, solvent and reaction temperature, it is usually 1 to 100 hours, and preferably 1 to 24 hours.

After completion of the reaction, for example, the solvent is distilled off and the reaction mixture is poured in water, made acidic with inorganic acids such as hydrochloric acid and sulfuric acid, extracted with a water-immiscible solvent such as benzene, ether and ethyl acetate, followed by evaporation of the solvent from the extract. The thus obtained product is usually used as such in the subsequent step. If desired, the product can be purified by isolation by various chromatographies or recrystallization.

(Step 3)

This step is for preparing compound (6) by reacting a deprotecting reagent with Compound (5) in an inert solvent to eliminate selectively the hydroxyl group at the 5'-position.

The solvent employable includes preferably aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; alcohols such as methanol, ethanol, n-propanol, isopropanol, n-butanol, isobutanol, t-butanol, isoamyl alcohol, diethylene glycol, glycerol, octanol, cyclohexanol and methyl cellosolve; ketones such as acetone, methyl ethyl ketone, methyl isobutyl ketone, isophorone and cyclohexanone; nitro compounds such as nitroethane and nitrobenzene; nitriles such as acetonitrile and isobutyronitrile; amides such as formamide, dimethylformamide, dimethylacetamide and hexamethylphosphorotriamide; and sulfoxides such as dimethyl sulfoxide and sulfolane, and more preferably alcohols (particularly methanol and ethanol).

The deprotecting reagent employable is not particularly limited as long as it is usually used, and includes, for example acetic acid, trifluoroacetic acid and hydrochloric acid methanol when the protecting group is a triarylmethyl group, and preferably acetic acid and trifluoroacetic acid.

While the reaction temperature varies depending on the reagent, starting material, solvent, etc. employed, it is usually -10 to 100 ° C, and preferably 0 to 50 ° C.

While the reaction time varies depending on the starting material, solvent and reaction temperature employed, it is usually 1 to 50 hours, and preferably 1 to 24 hours.

After completion of the reaction, for example, the solvent is distilled off and the reaction mixture is poured in water, made acidic with inorganic acids such as hydrochloric acid and sulfuric acid, extracted with a water-immiscible solvent such as benzene, ether and ethyl acetate, followed by evaporation of the solvent from the extract. The thus obtained product is usually used as such in the subsequent step. If desired, the product can be purified by isolation by various chromatographies or recrystallization.

(Step 4)

This step is for preparing Compound (7) by reacting Compound (6) with Compound (11) (in the formula, Hal represents a halogen atom) in an inert solvent in the presence of a base.

The halide portion of Compound (11) employed includes chlorine, bromine and iodine, and preferably chlorine and bromine.

The base employable preferably includes organic bases (particularly triethylamine, pyridine, N-methylmorpholine, DBU, etc.)

While the reaction temperature is not particularly limited, it is usually 0 ° C to 100 ° C, and the reaction is carried out preferably at 50 ° C.

While the reaction time is usually from 5 minutes to 30 hours, the reaction completes in 10 hours when it is carried out at 50 ° C.

For example, after completion of the reaction, the reaction mixture is appropriately neutralized, or if insolubles exist therein, they are removed by filtration. Then, an organic water-immiscible solvent such as ethyl acetate is added to the filtrate, followed by washing with water. The organic layer containing the

desired compound is separated and dried over anhydrous magnesium sulfate, followed by evaporation of the solvent to give the desired compound.

The thus obtained desired compound can further be purified by conventional procedure such as recrystallization, reprecipitation, chromatography, etc., if desired.

(Step 5)

This step is for preparing Compound (2) by reacting a deprotecting agent with Compound (7) in an inert solvent.

1) In the case where a silyl is used as the 3-position protecting group, it is usually removed by treating with a compound which forms a fluorine anion such as tetrabutylammonium fluoride.

While the solvent employable is not particularly limilted unless it inhibits the reaction, it preferably includes ethers such as tetrahydrofuran and dioxane.

While the reaction temperature is not particularly limited, it is usually -30°C to 100°C, and the reaction is carried out preferably at 0°C to 30°C.

While the reaction time is usually from 5 minutes to 30 hours, the reaction completes in 10 hours when it is carried out at 20°C.

For example, after completion of the reaction, the reaction mixture is appropriately neutralized, or if insolubles exist therein, they are removed by filtration. Then, an organic water-immiscible solvent such as ethyl acetate is added to the filtrate, followed by washing with water. The organic layer containing the desired compound is separated and dried over anhydrous magnesium sulfate, followed by evaporation of the solvent to give the desired compound.

The thus obtained desired compound can further be purified by conventional procedure such as recrystallization, reprecipitation, chromatography, etc., if desired.

2) In the case where a haloalkoxycarbonyl group is used as the 3'-position protecting group, zinc powder is usually used.

While the solvent employable is not particularly limilted unless it inhibits the reaction, it preferably includes acetic acid, alcohols and a mixture of water and these solvents.

While the reaction temperature is not particularly limited, it is usually 0°C to 100°C, and the reaction is carried out preferably at room temperature.

While the reaction time is usually from 5 minutes to 30 hours, the reaction completes in 10 hours when it is carried out at room temperature.

For example, after completion of the reaction, the reaction mixture is appropriately neutralized, or if insolubles exist therein, they are removed by filtration. Then, an organic water-immiscible solvent such as ethyl acetate is added to the filtrate, followed by washing with water. The organic layer containing the desired compound is separated and dried over anhydrous magnesium sulfate, followed by evaporation of the solvent to give the desired compound.

The thus obtained desired compound can further be purified by conventional procedure such as recrystallization, reprecipitation, chromatography, etc., if desired.

3) In the case where an aralkyloxycarbonyl group is used as the 3'-position protecting group, the deprotection is usually carried out by catalytic reduction or oxidization.

While the catalyst employable when the catalytic reduction is carried out is not particularly limited as long as it is usually used for the catalytic reduction reaction, it preferably inlcudes palladium carbon, Raney nickel, platinum oxide, platinum black, rhodium-aluminum oxide, triphenylphosphine-rhodium chloride and palladium-barium sulfate.

While the pressure is not particularly limited, the reaction is usually carried out at 1 to 10 atm.

While the reaction temperature and the reaction time vary depending on the starting material and the type of solvent and catalyst, the reaction is usually carried out at 0 to 100°C for 5 minutes to 24 hours.

While the solvent employable in the elimination by oxidization is not particularly limited unless it participates in the present reaction, it is preferably water-containing organic solvents.

Such organic solvents preferably include ketones such as acetone; halogenated hydrocarbons such as methylene chloride, chloroform and carbon tetrachloride; nitriles such as acetonitrile; ethers such as diethyl ether, tetrahydrofuran and dioxane; amides such as dimethylformamide, dimethylacetamide and hexamethylphosphorotriamide; and sulfoxides such as dimethyl sulfoxide.

While the oxidizing agent employable is not particularly limited as long as it is a compound to be used for oxidization, potassium persulfate, sodium persulfate, ammonium cerium nitrate (CAN), 2,3-dichloro-5,6-dicyano-p-benzoquinone (DDQ) are preferably used.

While the reaction temperature and the reaction time vary depending on the starting material, and the type of solvent and catalyst, the reaction is usually carried out at 0 to 150 ° C for 10 minutes to 24 hours.

Also, the 3'-position protecting group can be eliminated by reacting an alkali metal such as metallic lithium and metallic sodium in liquid ammonia or an alcohol such as methanol and ethanol at -78 to -20 ° C.

Further, the 3'-position protecting group can be eliminated by using aluminum chloride-sodium iodide or an alkylsilyl halide such as trimethylsilyl iodide in a solvent.

While the solvent employable is not particularly limited unless it particiates in the present reaction, it preferably includes nitriles such as acetonitrile, halogenated hydrocarbons such as methylene chloride and chloroform, and the mixture of these solvents.

While the reaction temperature and the reaction time vary depending on the type of starting material and solvent, the reaction is usually carried out at 0 to 50 ° C for 5 minutes to 3 days.

Incidentally, if the reaction substrate has a sulfur atom, aluminum chloride-sodium iodide is preferably employed.

For example, the reaction mixture is appropriately neutralized, or if insolubles exist therein, they are removed by filtration. Then, an organic water-immiscible solvent such as ethyl acetate is added to the filtrate, followed by washing with water. The organic layer containing the desired compound is separated and dried over anhydrous magnesium sulfate, followed by evaporation of the solvent to give the desired compound.

The thus obtained desired compound can further be purified by conventional procedure such as recrystallization, reprecipitation, chromatography, etc., if desired.

Process A-2

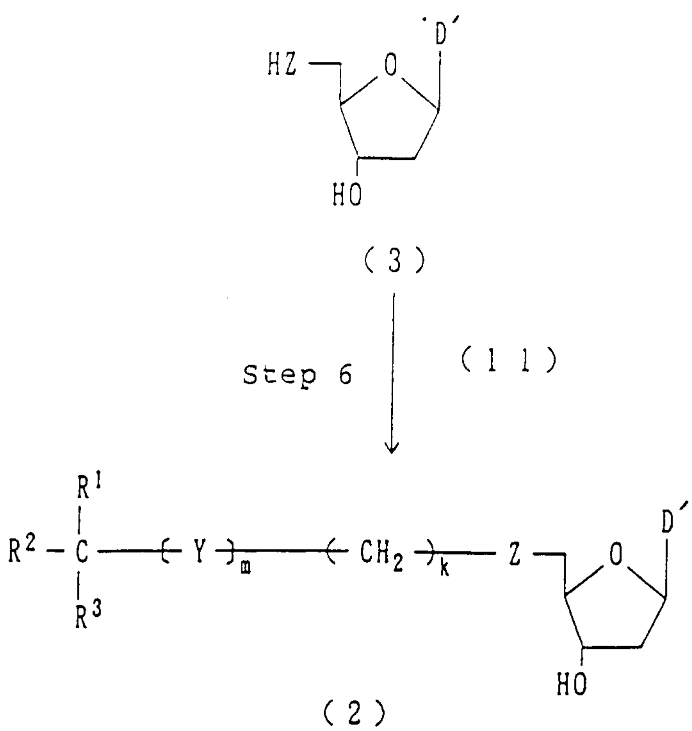


( 3 )

( 2 )

Process A-3

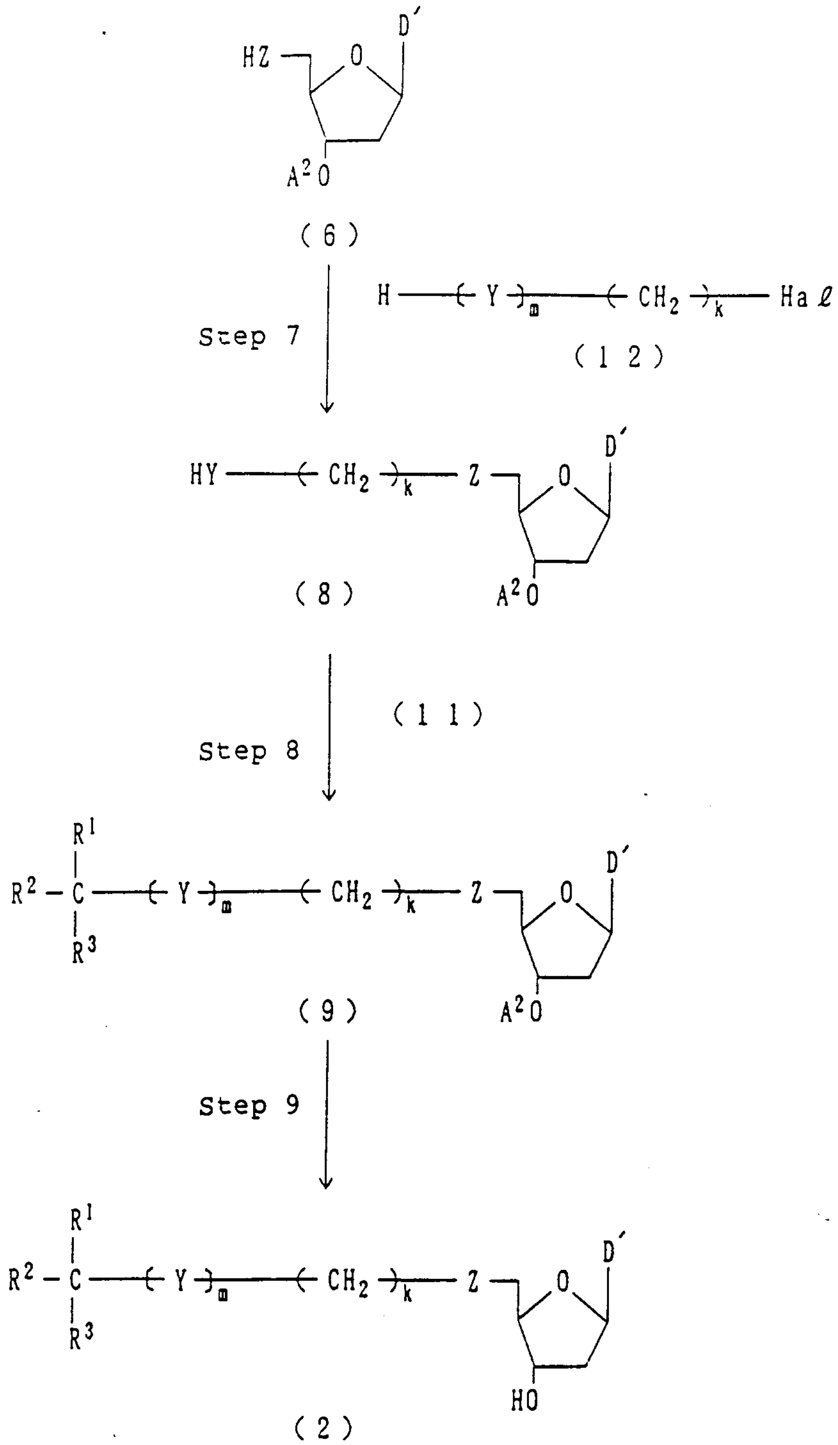


(6)

(12)

(8)

(11)

(9)

(2)

Process A-3 consists of Step 7 to Step 9.

(Step 7)

This step is for preparing Compound (8) by reacting Compound (6) with Compound (12) in an inert solvent in the presence of a base.

The halide portion of Compound (11) includes chlorine, bromine and iodine, preferably chlorine and bromine.

The solvent employable includes preferably aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; ketones such as acetone, methyl ethyl ketone, methyl isobutyl ketone, isophorone and cyclohexanone; nitro compounds such as nitroethane and nitrobenzene; nitriles such as acetonitrile and isobutyronitrile; amides such as formamide, dimethylformamide, dimethylacetamide and hexamethylphosphorotriamide; sulfoxides such as dimethyl sulfoxide and sulfolane, more preferably ketones (particularly acetone), halogenated hydrocarbons (particularly methylene chloride) and amides (particularly DMF).

The base employable preferably includes organic bases (particularly triethylamine, pyridine, N-methylmorpholine, DBU, etc.) and alkali metal carbonates (particularly sodium carbonate and lithium carbonate).

While the reaction temperature is not particularly limited, it is usually 0 °C to 100 °C, and the reaction is carried out preferably at 50 °C.

While the reaction time is usually from 5 minutes to 30 hours, the reaction completes in 10 hours when the reaction is carried out at 50 °C.

For example, after completion of the reaction, the reaction mixture is appropriately neutralized, or if insolubles exist therein, they are removed by filtration. Then, an organic water-immiscible solvent such as ethyl acetate is added to the filtrate, followed by washing with water. The organic layer containing the desired compound is separated and dried over anhydrous magnesium sulfate, followed by evaporation of the solvent to give the desired compound.

The thus obtained desired compound can further be purified by conventional procedure such as recrystallization, reprecipitation, chromatography, etc., if desired.

(Step 8)

This step is for preparing Compound (9) by reacting Compound (8) with Compound (11) in an inert solvent in the presence of a base. The base employable preferably includes organic bases (particularly triethylamine, pyridine, N-methylmorpholine, DBU, etc.) and alkali metal carbonates (particularly sodium carbonate and lithium carbonate).

While the reaction temperature is not particularly limited, it is usually 0 °C to 100 °C, and the reaction is carried out preferably at 50 °C.

While the reaction time is usually from 5 minutes to 30 hours, the reaction completes in 10 hours when the reaction is carried out at 50 °C.

For example, after completion of the reaction, the reaction mixture is appropriately neutralized, or if insolubles exist therein, they are removed by filtration. Then, an organic water-immiscible solvent such as ethyl acetate is added to the filtrate, followed by washing with water. The organic layer containing the desired compound is separated and dried over anhydrous magnesium sulfate, followed by evaporation of the solvent to give the desired compound.

The thus obtained desired compound can further be purified by conventional procedure such as recrystallization, reprecipitation, chromatography, etc., if desired.

(Step 9)

This step is for preparing Compound (2) by reacting a deprotecting agent with Compound (9) in an inert solvent, and is carried out in the same manner as in Step 5 of Process A-1.

Process A-4

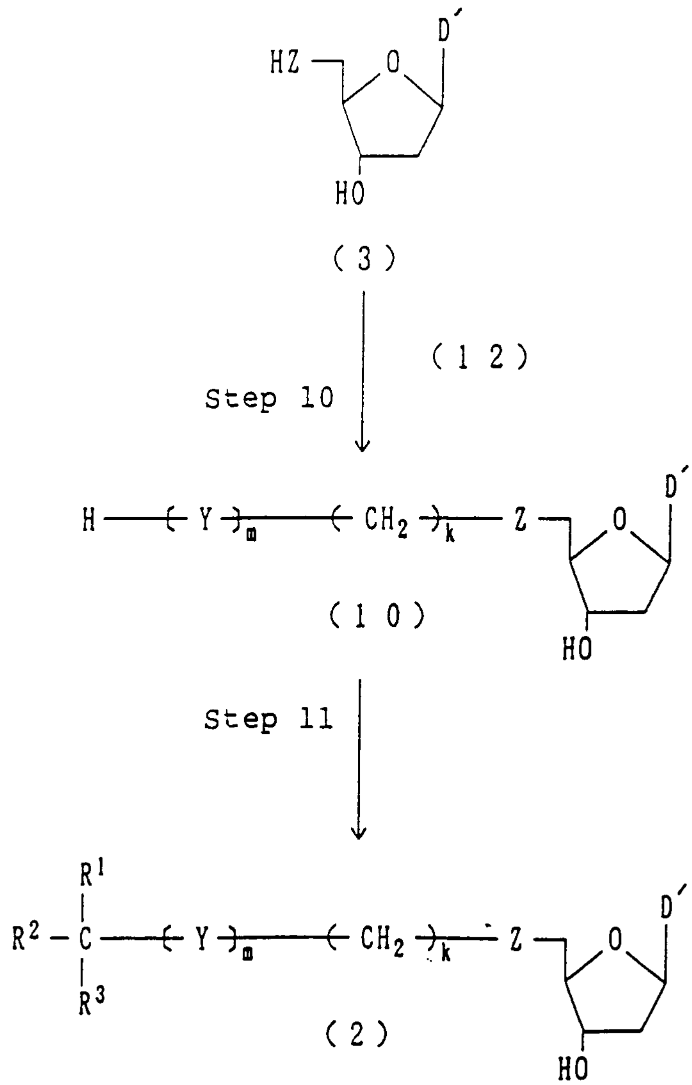


(3)

(10)

(2)

Process A-4 consists of Step 10 and Step 11.

(Step 10)

This step is for preparing Compound (10) by reacting Compound (12) (in the formula Hal represents a halogen atom) with Compound (3) in an inert solvent in the presence of a base. This step is carried out in the same manner as in Step 7 of Process A-3.

(Step 11)

This step is for preparing Compound (2) by reacting Compound (12) (wherein Hal represents a halogen atom) with Compound (10) in an inert solvent in the presence of a base. This step is carried out in the same manner as in Step 8 of Process A-3.

Process B

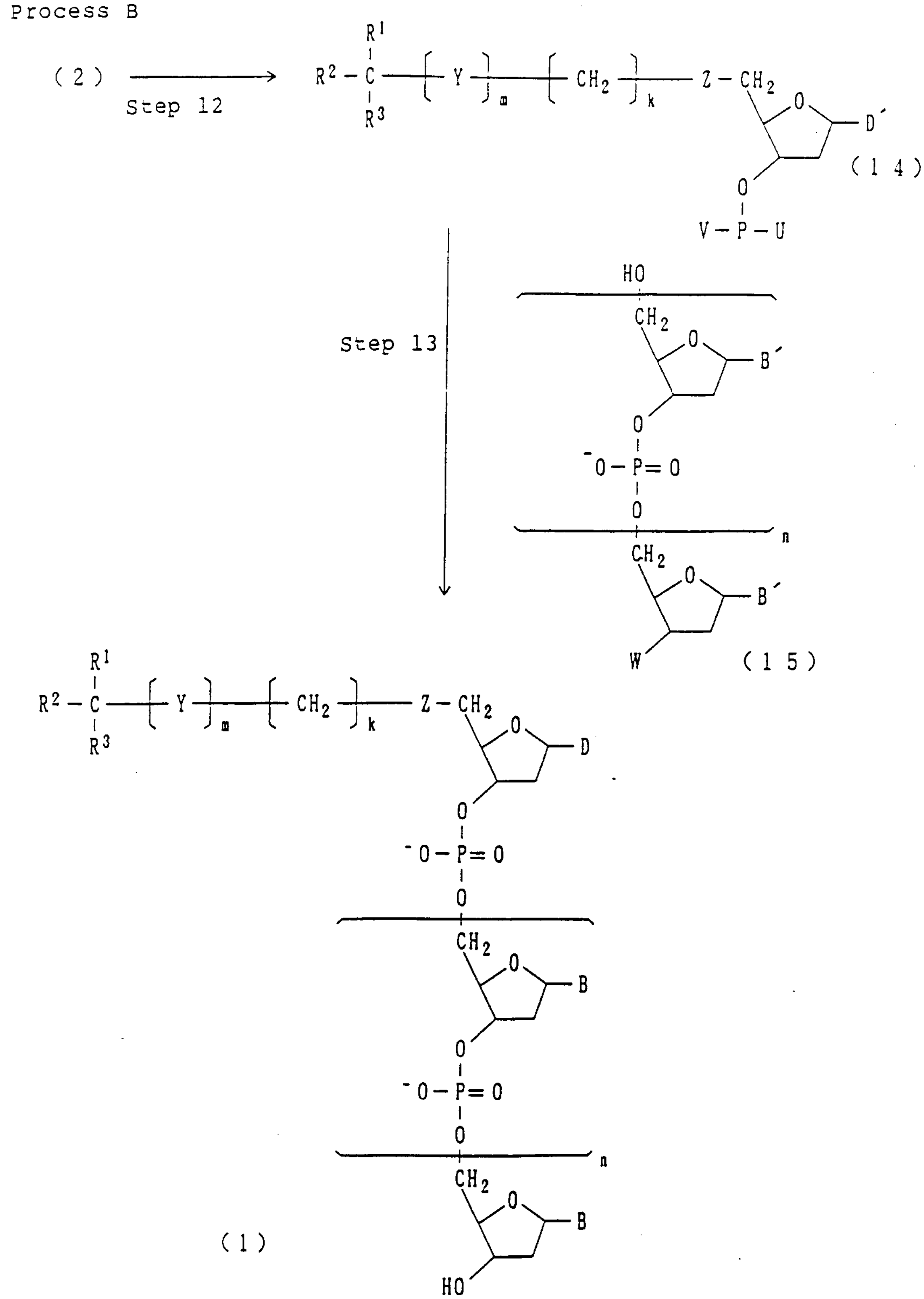


(2) (14) (15) (1)

Process B can be performed according to Step 12 and Step 13 indicated below. An explanation of each of these steps is given below.

(Step 12)

This step is for preparing a 3'-phosphorous acid derivative (Compound (14)) by reacting chlorophosphoramidite (Compound (13)), a phosphitylating agent with Compound (2) in an inactive solvent in the presence of a acid acceptor. A dialkylamino group such as a dimethylamino group and diisopropylamino group, or a heterocyclic group having 1 or 2 oxygen atoms and/or nitrogen atoms in its ring, such as a morpholino group, is used for U of Compound (13). While any group can be used for V of Compound (13) provided that it can be removed following formation of a phosphodiester group in Step 13, lower alkyl groups such as a methyl group, and cyanoalkyl groups such as a cyanoethyl group, are used preferably. Examples of Compound (13) include phosphines such as chloromorpholinomethoxyphosphine, chloromorpholinocyanoethoxyphosphine, chlorodimethylaminomethoxyphosphine, chlorodimethylaminoethoxyphosphine, chlorodiisopropylaminomethoxyphosphine and chlorodiisopropylaminoethoxyphosphine, preferably chloromorpholinomethoxyphosphine, chloromorpholinocyanoethoxyphosphine, chlorodiisopropylaminomethoxyphosphine and chlorodiisopropylaminocyanoethoxyphosphine.

While the solvent used is not particularly limited, provided that it does not affect the reaction, preferable examples include ethers such as tetrahydrofuran, diethyl ether and dioxane.

While examples of the acid acceptor used include heterocyclic amines such as pyridine and dimethylaminopyridine, and aliphatic amines such as trimethylamine, triethylamine and diisopropylethylamine, aliphatic amines (particularly diisopropylethylamine) are used preferably.

While the reaction temperature is not particularly limited, it is usually -50 °C to 50 °C, preferably room temperature.

While the reaction time varies depending on the starting material, reagent and temperature employed, it is usually 5 minutes to 30 hours, preferably 30 minutes when the reaction is carried out at room temperature. For example, the reaction mixture is appropriately neutralized or if insolubles exist, they are removed by filtration. Then, an organic water-immiscible solvent such as ethyl acetate is added thereto, followed by washing with water. The organic layer containing the desired compound is separated and dried over anhydrous magnesium sulfate, followed by evaporation of the solvent to give the desired compound.

The thus obtained desired compound can further be purified by conventional procedure such as recrystallization, reprecipitation, chromatography, etc., if desired.

(Step 13)

In this step, (i) Compound (14) obtained in Step (12), and (ii) [1] an ODN synthesized with a DNA synthesizer and coupled to CPG wherein only the 5'-terminal dimethoxytrityl group is eliminated and the base portion is protected with a protecting group such as an acyl group, or [2] an ODN synthesized by liquid phase technique having a free hydroxyl group at its 5'-terminal wherein the base portion is protected with a protecting group such as an acyl group, (iii) are [1] condensed using a suitable condensing agent to form a triphosphite bond, followed by [2] oxidation using a suitable oxidizing agent to convert to phosphotriester and severing the ODN from the CPG in the case where the ODN is coupled to CPG followed by elimination of the protecting group to (iv) obtain the final product after going through a purification procedure.

The ODN of the desired nucleotide sequence, in which a free hydroxyl group at the 5'-terminal is protected, can be synthesized using a DNA synthesizer such as the Model 380-B of Applied Biosystems Inc. applying the phosphoroamidite method, according to any variation of the stec method described in the J. Am. Chem. Soc., 106, 6077-6089 (1984).

In addition, a base protected with an acyl group is used for base in the material of ODN synthesis. A benzoyl group is preferably used for the acyl group in the case the base portion is A or C, while an isobutyryl group is used for the acyl group in the case the base is G.

Furthermore, in the case of free ODN not coupled to CPG, it is preferred that said ODN be purified in order to be used in the following reaction. Said ODN can be purified with a purification procedure used in ordinary purification of nucleic acids, including various types of chromatography such as reverse phase and ion exchange chromatography (including high-performance liquid chromatography).

While examples of acidic substances used for the catalyst in the condensation reaction of this step include acidic substances such as mesitylene sulfonylnitrotriazolide and tetrazole, tetrazole is used preferably.

While the solvent employable is not particularly limited unless it inhibits the reaction, it preferably includes nitriles.

While the reaction temperature may be in the range of -30 to 50 ° C, the reaction is usually carried out at room temperature.

While the reaction time is in the range of 1 minutes to 20 hours and it varies depending on the reaction temperature, the reaction completes in 10 minutes when the reaction is carried out at room temperature.

While there are no particular limitations on the oxidizing agent used in the oxidization reaction of this process provided that it is an oxidizing agent usually used in oxidization reactions, it can be preferably exemplified by inorganic metal oxidizing agents including manganese oxides such as potassium permanganate and manganese dioxide; ruthenium oxides such as ruthenium tetraoxide; selenium compounds such as selenium dioxide; iron compounds such as iron chloride; osmium compounds such as osmium tetraoxide; silver compounds such as silver oxide; mercury compounds such as mercury acetate; lead oxide compounds such as lead oxide and lead tetraoxide; chromic acid compounds such as potassium chromate, chromic acid-sulfuric acid complex and chromic acid-pyridine complex; and, cerium compounds such as cerium ammonium nitrate (CAN); inorganic oxidizing agents including halogen molecules such as chlorine molecules, bromine molecules and iodine molecules; periodic acids such as sodium periodate; ozone; aqueous hydrogen peroxide; nitrous acid compounds such as nitrous acid; chlorous acid compounds such as potassium chlorite and sodium chlorite; perchlorate compounds such as potassium perchlorate and sodium perchlorate; and persulfuric acid compounds such as potassium persulfate and sodium persulfate as well as organic oxidizing agents including reagents used in DMSO oxidization (complexes of dimethyl sulfoxide and dicyclohexylcarbodiimide, oxalyl chloride, acetic anhydride or phosphorus pentoxide, and complexes of pyridine and sulfuric anhydride); peroxides such as t-butylhydroperoxide; stable cations such as triphenylmethyl cation; succinimides such as N-bromosuccinimide; hypochlorous acid compounds such as t-butylhypochlorite; azo-dicarboxylic acid compounds such as azo-dicarboxylic acid esters; disulfides and triphenyl phosphines such as dimethyl disulfide, diphenyl disulfide and dipyridyl disulfide; sulfites such as methyl sulfite; tetrahalogenated carbon such as methane tetrabromide; and quinone compounds such as 2,3-dichloro-5,6-dicyano-p-benzoquinone (DDQ), particularly preferably iodine.

The solvent to be used in the reaction is not particularly limited, provided that it does not interfere with the reaction and can dissolve the starting materials to some extent, and it preferably includes aromatic hydrocarbons such as benzene, toluene and xylene; halogenated hydrocarbons such as methylene chloride and chloroform; ethers such as ether, tetrahydrofuran, dioxane and dimethoxyethane; amides such as dimethylformamide, dimethylacetamide and hexamethylphosphorotriamide; sulfoxides such as dimethyl sulfoxide; alcohols such as methanol, ethanol, n-propanol, isopropanol, n-butanol, isobutanol and isoamyl alcohol; diluted acids such as aqueous sulfuric acid; diluted bases such as aqueous sodium hydroxide; water; acetone; ketones such as methyl ethyl ketone; heterocyclic amines such as pyridine; and nitriles such as acetonitrile, preferably nitriles (particularly acetonitrile), ethers (particularly tetrahydrofuran) and halogenated hydrocarbons (particularly methylene chloride).

The reaction is carried out at a temperature of -50 to 100 ° C. while the reaction time varies depending on the reaction temperature, raw material compounds and type of solvent used, it is usually 30 minutes to 15 hours. Furthermore, in the above-mentioned oxidization reaction, the reaction is accelerated by the addition of a layer migrating catalyst such as triethylbenzylammonium chloride and tributylbenzylammonium bromide.

Severing ODN from CPG in the case where ODN is coupled to CPG as well as removal of the protecting groups other than those substituted at the 5'-terminal can be carried out according to known methods (J. Am. Chem. Soc., 103, 3185 (1981)).

By purifying the compound of the general formula (1) obtained in this manner with a purification procedure usually used for purification of nucleic acids including various types of chromatography such as reverse phase and ion exchange chromatography (including high-performance liquid chromatography), the compound having the above-mentioned general formula (1) can be obtained.

Process C

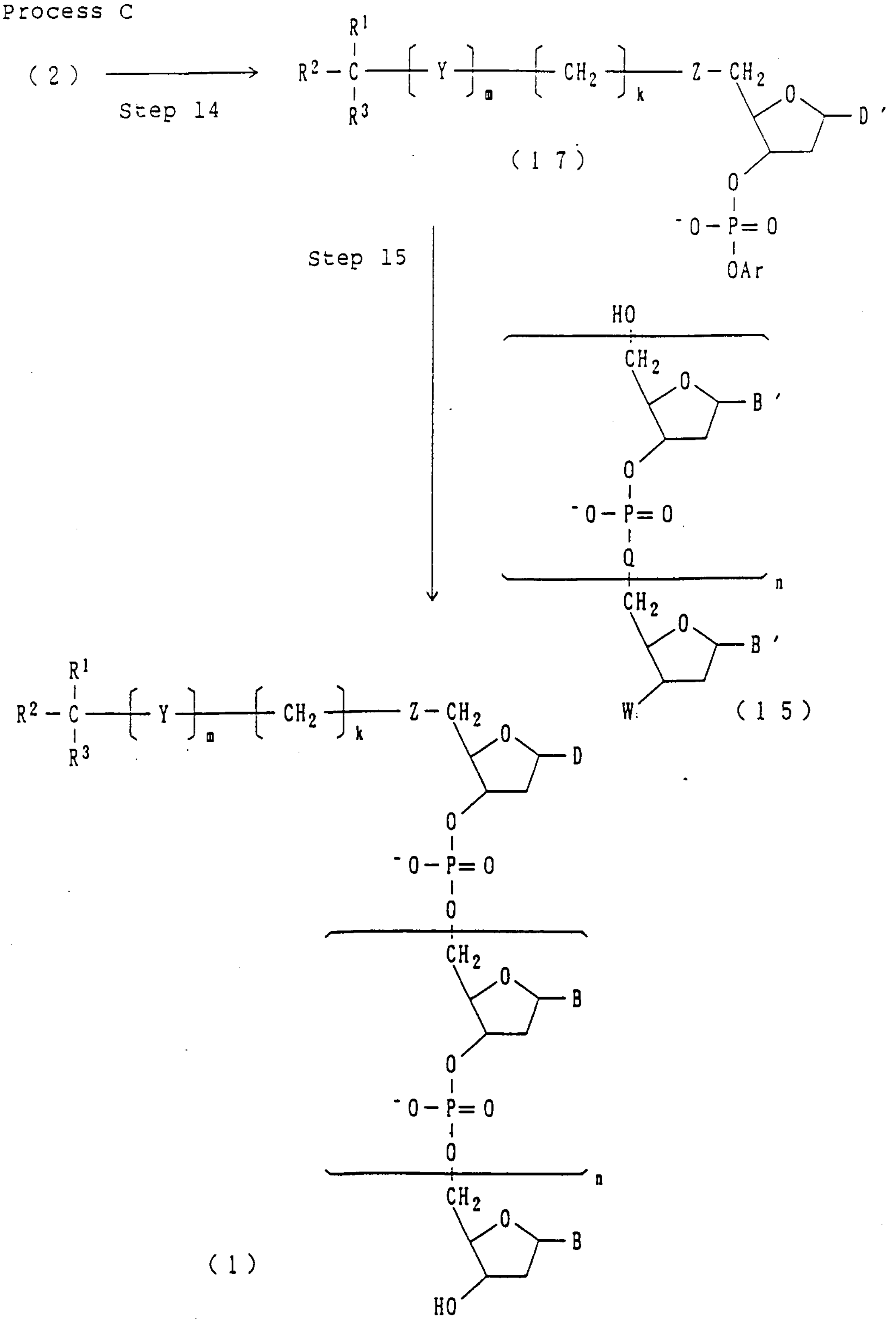


(2)

(17)

(15)

(1)

Process C can be performed according to Step 14 and Step 15 indicated below. An explanation of each of these steps is given below.

(Step 14)

In this step, after reacting a phosphating reagent such as ditriazolide (16) with Compound (2) in an inactive solvent, the reaction mixture is treated following addition of water to obtain mononucleotide (17) to serve as the intermediate.

While the solvent is not paticularly limited provided that it does not interfere with the reaction, it usually includes aromatic amines such as pyridine. While there are also no particular limitations on the Ar group of the phosphating reagent provided that it can be eliminated under conditions for eliminating the protecting group of the base portion following completion of the condensation reaction of Step 15, an ortho-chlorophenyl group is usually used.

While the reaction temperature is not particularly limited provided that it is within a range of -20 to 100°C, the reaction is usually carried out at room temperature. While the reaction time varies depending on the solvent used and the reaction temperature, the reaction time is 1 hour in the case of using pyridine for the reaction solvent and carrying out the reaction at room temperature.

(Step 15)

In this step, mononucleotide (17) obtained in Step 14, and [1] an ODN synthesized with a DNA synthesizer and coupled to CPG wherein only the 5'-terminal dimethoxytrityl group is eliminated and the base portion and phosphate portion are protected with protecting groups, or [2] an ODN synthesized with a liquid phase technique and having a free hydroxyl group at the 5'-terminal wherein the base portion and phosphate portion are protected with protecting groups, are condensed using a condensing agent to form phosphotriester bonds, followed by severing the ODN from the CPG in the case where the ODN is coupled to CPG and elimination of the protecting groups to obtain final product (1) after going through a purification procedure. While the solvent used in this step is not particularly limited provided that it does not interfere with the reaction, it preferably includes aromatic amines such as pyridine.

While examples of the condensing agent used for condensation include dicyclohexylcarbodiimide (DCC), mesitylene sulfonyl chloride (Ms-Cl), triisopropylbenzenesulfonyl chloride, mesitylene sulfonyl triazolide (MST), mesitylene sulfonyl-3-nitrotriazolide (MSNT), triisopropylbenzenesulfonyl tetrazolide (TPS-Te), triisopropylbenzenesulfonyl nitroimidazolide (TPS-NI) and triisopropylbenzenesulfonyl pyridyltetrazolide, MSNT, TPS-Te and TPS-NI are used preferably.

While the reaction temperature is not particularly limited as long as it is in the range of -10 to 100°C, the reaction is usually carried out at room temperature. While the reaction time varies depending on the solvent and the reaction temperature employed, it is 30 minutes when pyridine is used as the reactive solvent and the reaction is carried out at room temperature.

After completion of the reaction, for example, the reaction mixture is appropriately neutralized, or if insolubles exist therein, they are removed by filtration. Then, an organic water-immiscible solvent such as ethyl acetate is added to the filtrate, followed by washing with water. The organic layer containing the desired compound is separated and dried over anhydrous magnesium sulfate, followed by evaporation of the solvent to give the desired compound.

The thus obtained desired compound can further be purified by conventional procedure such as recrystallization, reprecipitation, chromatography, etc., if desired.

Process D

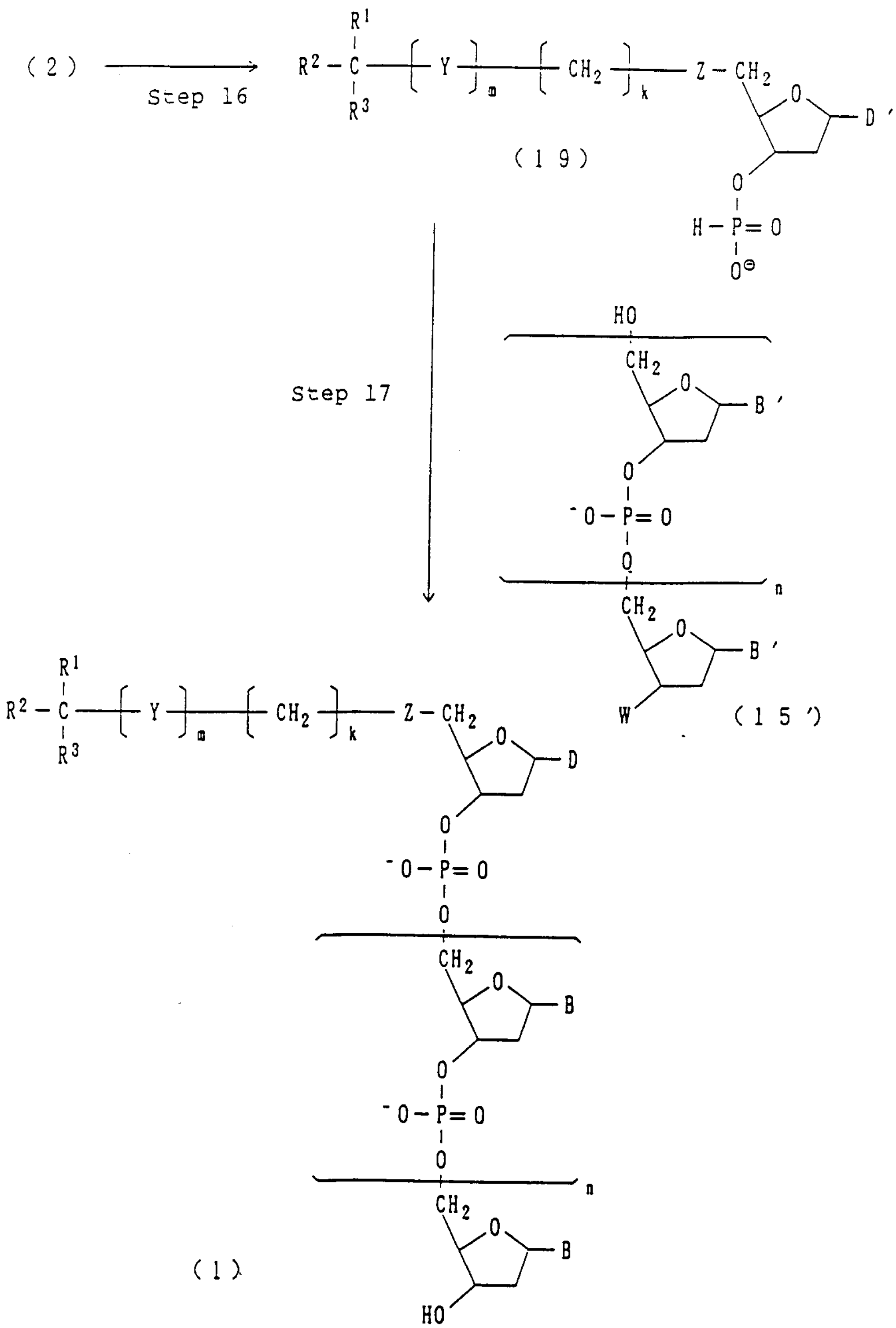


Process D is indicated below. It can be performed according to Step 16 and Step 17. An explanation of each of these steps is given below.

(Step 16)

In Step 16, after reacting, for example, tris-(1,2,4-triazolyl)phosphite (18), prepared in advance from phosphorus trichloride and 1,2,4-triazole according to the literature (B.C. Freohler, P.G. Ng and M.D. Matteucci, Nucleic Acid Res., 14, 5399 (1986)), with Compound (2) in an inactive solvent, water is added to the reaction mixture to stop the reaction followed by post-treatment to obtain 3'-H-phosphonate nucleoside (19). While the solvent used is not particularly limited provided that it does not interfere with the reaction, a preferable example is halogenated hydrocarbon such as methylene chloride. While there are no particular limitations on the reaction temperature provided that it is within a range of -20 to 100 °C, the reaction is usually carried out at room temperature.

While the reaction time varies depending on the solvent and reaction temperature employed, it is 30 minutes in the case where the reaction is carried out in methylene chloride at room temperature.

(Step 17)

In this step, the 3'-H-phosphonate nucleoside (19) obtained in Step 16, and an ODN synthesized with a DNA synthesizer and connected with CPG and wherein only the 5'-terminal dimethoxytrityl group is eliminated and the base portion is protected with a protecting group (B.C. Froehler, P.C. Ng and M.D. Matteucci, Nucleic Acid Res., 14, 5399 (1986)), are condensed in the presence of a condensing agent such as pivaloyl chloride and a acid acceptor to form H-phosphonic diester bonds, followed by conversion of H-phosphonate bonds to phosphodiester bonds using an oxidizing agent and removing the protecting group of the base portion simultaneous to severing the ODN from the CPG under basic conditions to obtain the final product (1) after going through a purification procedure. While the solvent used in this process is not particularly limited provided that it does not interfere with the reaction, anhydrous acetonitrile is used preferably. While acid chlorides of carboxylic acids or phosphoric acid are used for the reagent used as the condensing agent, pivaloyl chloride is used preferably.

While there are no particular limitations on the oxidizing agent used for oxidizing the H-phosphonic acid ODN to the ODN of phosphodiester type provided that it is an oxidizing agent usually used in oxidation reactions, it can be exemplified by iorganic metal oxidizing agents including manganese oxides such as potassium permanganate and manganese dioxide; ruthenium oxides such as ruthenium tetraoxide; selenium compounds such as selenium dioxide; iron compounds such as iron chloride; osmium compounds such as osmium tetraoxide; silver compounds such as silver oxide; mercury compounds such as mercury acetate; lead oxide compounds such as lead oxide and lead tetraoxide; chromic acid compounds such as potassium chromate, chromic acid-sulfuric acid complex and chromic acid-pyridine complex; and, cerium compounds such as cerium ammonium nitrate (CAN); inorganic oxidizing agents including halogen molecules such as chlorine molecules, bromine molecules and iodine molecules; periodic acids such as sodium periodate; ozone; aqueous hydrogen peroxide; nitrous acid compounds such as nitrous acid; chlorite compounds such as potassium chlorite and sodium chlorite; and persulfuric acid compounds such as potassium per sulfate and sodium persulfate as well as organic oxidizing agents including reagents used in DMSO oxidization (complexes of dimethyl sulfoxide and dicyclohexylcarbodiimide, oxalyl chloride, acetic anhydride or phosphorus pentoxide, and complexes of pyridine and sulfuric anhydride); peroxides such as t-butylhydroperoxide; stable cations such as triphenylmethyl cation; succinimides such as N-bromosuccinimide; hypochlorous acid compounds such as t-butylhypochlorite; azo-dicarboxylic acid compounds such as methyl azodicarboxylate; disulfides and triphenyl phosphines such as dimethyl disulfide, diphenyl disulfide and dipyridyl disulfide; sulfites such as methyl sulfite; tetrahalogenated carbons such as methane tetrabromide; and quinone compounds such as 2,3-dichloro-5,6-dicyano-p-benzoquinone (DDQ), and particularly preferably iodine molecule.

While examples of the acid acceptor used include heterocyclic amines such as pyridine and dimethylaminopyridine, and aliphatic amines such as trimethylamine, triethylamine and diisopropylethylamine, aliphatic amines (particularly diisopropylethylamine) are used preferably.

While the reaction temperature is not particularly limited, it is usually -50 to 50 °C, preferably room temperature.

While the reaction time varies depending on the starting material, reagent and temperature employed, it is usually 5 minutes to 30 hours. When the reaction is carried out at room temperature, the reaction time is preferably 30 minutes. After completion of the reaction, for example, the reaction mixture is appropriately neutralized, or if insolubles exist therein, they are removed by filtration. Then, an organic water-immiscible solvent such as ethyl acetate is added to the filtrate, followed by washing with water. The organic layer containing the desired compound is separated and dried over anhydrous magnesium sulfate, followed by

evaporation of the solvent to give the desired compound.

The thus obtained desired compound can further be purified by conventional procedure such as recrystallization, reprecipitation, chromatography, etc., if desired.

Process E

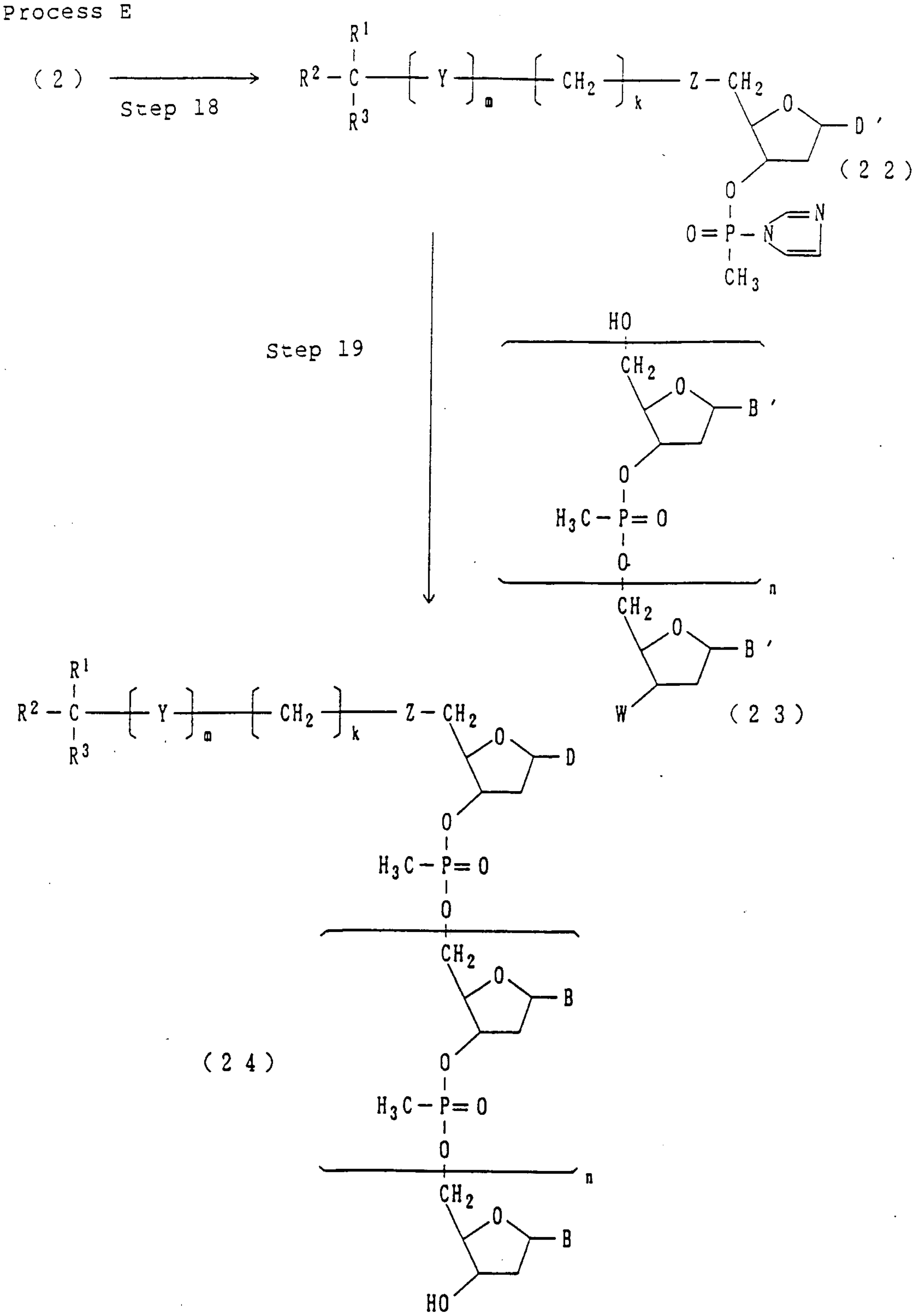

(2)
(22)
(23)
(24)

Process E can be performed according to Step 18 and Step 19 indicated below.

(Step 18)

In this step, methylphosphonic bis-imidazolide (22), synthesized in advance by reacting methylphosphonic dichloride and imidazole in the presence of, for example, imidazole, is reacted with Compound (2) in an inert solvent to obtain 3'-methylphosphonic imidazolide (22). (Reference: P.S. Miller, M.P. Reddy, A. Murakami, K.R. Blake, S.B. Lin and C.H. Agris, Biochem., 25, 5092 (1986)).

While the solvent employable is not particularly limited provided that it does not interfere with the reaction, tetrahydrofuran is preferably used. While the reaction temperature is not particularly limited and it is in the range of -20 to 100°C, the reaction is preferably carried out at room temperature. The reaction time varies depending on the solvent and reaction temperature employed, it is 6 hours when the reaction is carried out at room temperature in tetrahydrofuran.

This step can be also carried out using secondary amines such as diisopropylamine in place of imidazole to synthesize 3'-methylphosphonic diisopropylamidite (22') of Compound (2) according to the reference (S. Agrawal and J. Goodchild, Tetrahedron Lett., 28 3539 (1987)), and the resulting compound can be used in the subsequent Step 19. Reference [P. Bhan and P.S. Miller, Bioconjugate chem., 1 82 (1990)]

(Step 19)

In this step, Compound (22) obtained in Step 18, and a methylphosphonate ODN (23) synthesized according to the above-mentioned reference and coupled to CPG wherein only the 5'-terminal dimethyoxytrityl group is eliminated and the base portion is protected with a protecting group, are condensed in the presence of tetrazole to form methylphosphonic diester bonds, followed by eliminating the protecting group of the base portion simultaneous to severing the ODN from the CPG under basic conditions to obtain the final product (24) after going through a purification procedure. While the solvent used in this process is not particularly limited provided that it does not interfere with the reaction, acetonitrile is used preferably. While there are no particular limitations on the reagent used for the condensing agent provided that it causes protonation of the imidazole and diisopropylamino groups of Compound (22) or (22') to form methylphosphonic diester bonds between the hydroxyl groups of a compound having hydroxyl groups such as Compound (23) when said compound is present, tetrazole is used preferably.

While the reaction temperature is not particularly limited, it is usually -50 to 50°C, preferably room temperature.

While the reaction time varies depending on the starting material, reagent and temperature employed, it is usually 5 minutes to 30 hours. When the reaction is carried out at room temperature, the reaction time is preferably 30 minutes. After completion of the reaction, for example, the reaction mixture is appropriately neutralized, or if insolubles exist therein, they are removed by filtration. Then, an organic water-immiscible solvent such as ethyl acetate is added to the filtrate, followed by washing with water. The organic layer containing the desired compound is separated and dried over anhydrous magnesium sulfate, followed by evaporation of the solvent to give the desired compound.

The thus obtained desired compound can further be purified by conventional procedure such as recrystallization, reprecipitation, chromatography, etc., if desired.

Process F

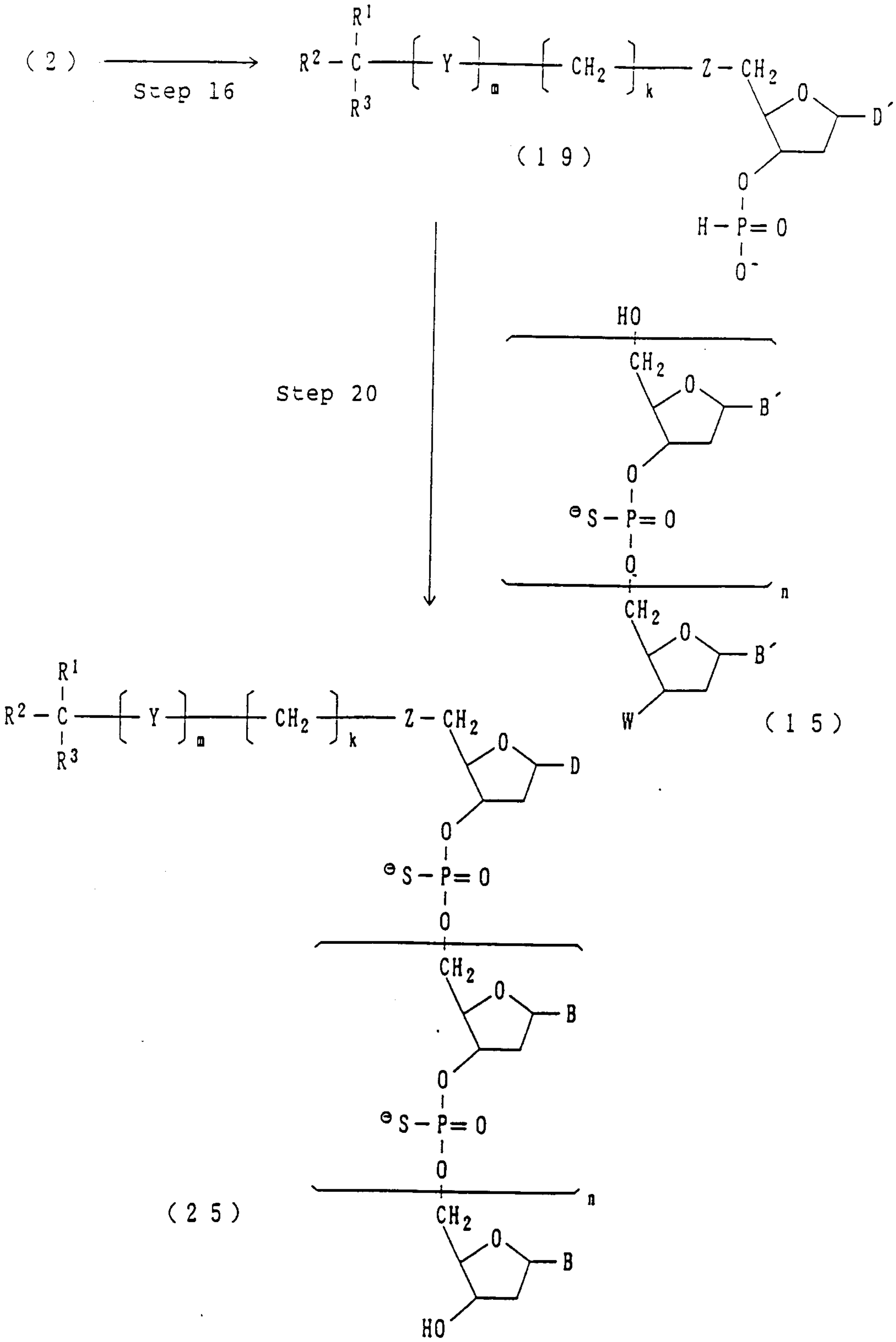


(2)

(19)

(15)

(25)

Process F is composed of Step 20 wherein thioate ODN (15), coupled to CPG and having a free hydroxyl group only at its 5'-terminal, is reacted with Compound (19) obtained in Step 16 indicated in Process D.

(Step 20)

In this step, Compound (19) obtained in Step 16 according to, for example, the method described in the literature (M. Matsukura, G. Zon, K. Shinozuka, C.A. Stein, H. Mitsuya, J.S. Cohen and S. Broder, Gene, 72, 343 (1988)), and thioate ODN (15) synthesized on a DNA synthesizer and coupled to CPG wherein the 5'-terminal dimethoxytrityl group is eliminated to form diphosphonate bonds in the same manner as in Step 17 in Process D using pivaloyl chloride or 1-adamantane carbonyl chloride as the condensing agent, followed by reacting sulfur dissolved in carbon disulfide in the presence of triethylamine using pyridine as the reaction solvent, converting the diphosphonate bonds into phosphoric thioate bonds, and eliminating the protecting group of the base portion simultaneous to severing the ODN from the CPG under basic conditions to obtain the final desired compound after going through a purification procedure. While the solvent used for thioation of this process is not particularly limited provided that it does not interfere with the reaction, pyridine is used preferably, and carbon disulfide is used preferably as the sulfur solvent.

In addition, there are no particular limitations on the amine used, tertiary amines such as triethylamine are preferable.

Severing of the thioate ODN from the CPG as well as elimination of the protecting group of the base portion can be performed according to User Bulletin of Model 381A (1987) available from Applied Biosystems Inc.

Process G

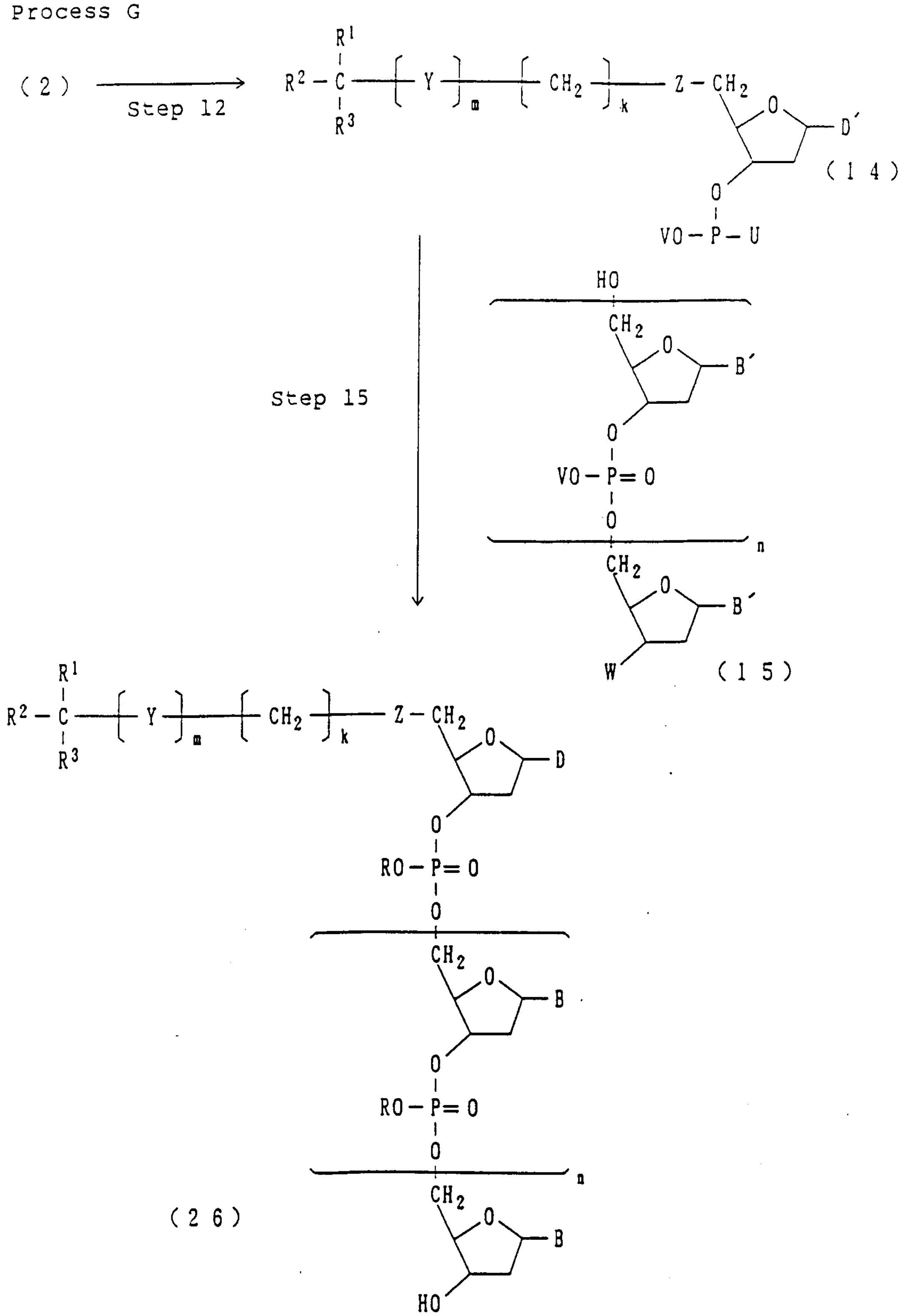


(2) (14) (15) (26)

Process G is the sulfiting agent employed in Step 12 of Process B. Triester ODN derivative (26) is obtained by carrying out Step 12 and Step 13 using a reagent (13) including an alkyl group such as a lower alkyl group, e.g. ethyl, propyl and butyl that is not eliminated during severing of ODN, synthesized with a DNA synthesizer, wherein V is a methyl group or cyanoethyl group, from CPG, as well as under basic conditions for elimination of the protecting group from the base portions.

Process H

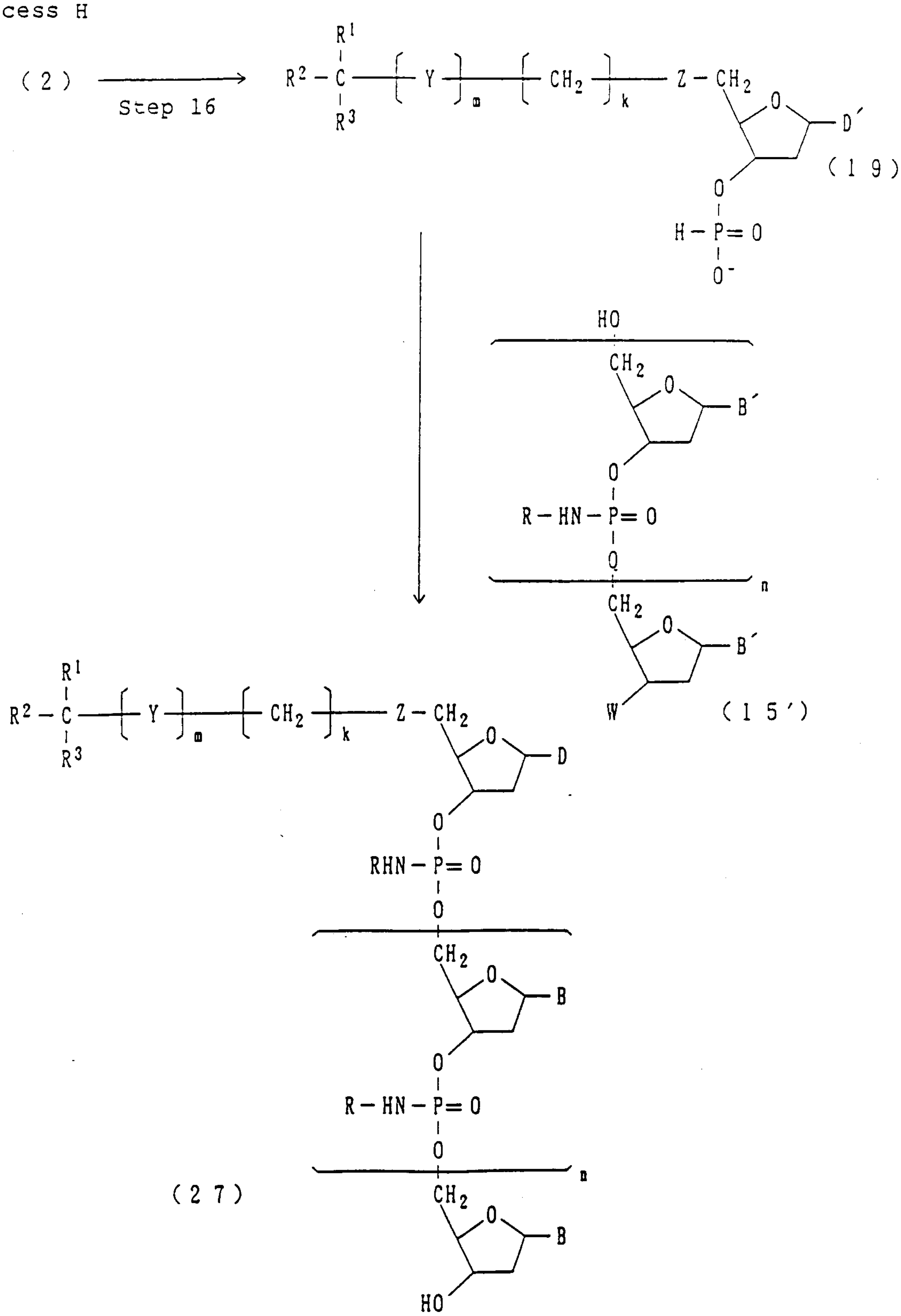


(2)

(19)

(15')

(27)

In process H, the H-phosphonate ester(19) synthesized in Step(16) of process D and an alkylphosphoramidate ODN connecting with CPG and being removed the 5'-terminal dimethoxytrityl group which was synthesized with a DNA synthesizer according to the method described in the literature( S.Agrawal, J. Good-child, M.P.Civeira, A.H.Thornton, P.S.Sarin and P.C.Zamecnik, Proc. Natl. Acad. Sci., 85, 7079(1988) ) were condensed in the presence of pivalic chloride in the same manner as in the condensation reaction of

step 17 and followed by treating the resulted ODN having 5'-terminal hydrogen phosphonate diester with appropriate amine in tetrachloromethane at room temperature for 1.5 hr. and ODN was severed from the CPG and treated with aqueous conc. ammonia to eliminate the protecting group of the base portion either at 55°C for 5.5 hours or at room temperature for 2 days or more to obtain compound(27).

An antiviral agent or antitumor agent having the compound represented by the general formula (1) of the present invention as its active ingredient can be prepared by using said compound in the form of a pharmacologically acceptable atoxic salt.

In the present invention, viral infection diseases or tumor generically refers to all types of diseases that are induced by cellular changes due to the action of internal or external virus or tumor genes. Antiviral agents or antitumor agents refer to all drugs that are used to prevent and/or treat these diseases.

Pharmaceutical preparations of these drugs can be manufactured by well known methods in said field. Said preparations can be administered by, for example, oral administration in the form of tablets, capsules, granules, powders and syrups, or parenteral administration in the form of injection preparations (intravenous, intramuscular and subcutaneous), intravenous drip preparations and suppositories. Water-soluble solvents such as physiological saline, sterilized water and Ringer's solution, water-insoluble solvents, isotonic agents, stabilizers, preservatives, suspending agents, buffers and emulsifiers and so forth can be arbitrarily used for preparing injection preparations and intravenous drip preparations. Each of these types of preparations can be prepared using known adjuvants that can be usually used in technical fields of preparation formulation, such as excipients, binders, disintegrators, flavorings, fragrances, solubility assistants, suspending agents and coating agents, according to conventional methods. While the dose used varies according to symptoms, age, body weight and so forth, the dose given to adults is about 10 mg to 1000 mg per day in the case of oral administration, with this dose to be administered once or in several portions. In addition, in the case of parenteral administration, 10 mg to 500 mg per daily administration can be given by subcutaneous injection, intramuscular injection or intravenous injection.

The present invention is specifically described below by way of Examples, Comparative examples, Test examples and Preparation examples, but the invention is not limited thereto.

(Example 1) - Tr-AGGTGGGTCTGAAAC

(1a) 5'-O-trityl-$N^6$-benzoyladenosine

In 50 ml of dry pyridine was dissolved 2.60 g (7.31 mmol) of $N^6$-benzoyladenosine, and the resulting solution was subjected to azeotropic distillation to dryness by evaporating the pyridine under reduced pressure. The residue was dissolved in 70 ml of dry pyridine, followed by the addition of 2.24 g (8.05 mmol) of trityl chloride and stirring at 50°C in an argon atmosphere. After 3.5 hours, an additional 2.24 g of trityl chloride was added thereto, followed by stirring at 50°C for 6.5 hours. After allowing to stand overnight at room temperature, the mixture was diluted with 500 ml of methylene chloride, washed three times with 200 ml of a saturated aqueous $NaHCO_3$, and dried over anhydrous $MgSO_4$. After removing the drying agent by filtration, the residue obtained by distilling off the solvent under reduced pressure was applied to a 120 g silica gel column (70-230 mesh) and eluted with methylene chloride containing 1 to 3% methanol to obtain 2.27 g of the desired substance.

$^1$H-NMR(60MHz, $CDCl_3$) δppm:

9.72 (1H, bs, NH); 8.70 (1H, S, H-8); 8.16 (1H, s, H-2); 8.12-7.84 (2H, m, ortho-H of Bz); 7.62-6.92 (18H, m, Tr and m, p-H of Bz); 6.46 (1H, t, J=6Hz, H-1'); 4.84 (1H, bs, OH); 4.73 (1H, bs, H-3'); 4.22 (1H, bs, H-4'), 3.33 (2H, bs, H-5'); 2.84-2.30 (2H, m, H-2')

(2a) 5'-O-Trityl-$N^6$-benzoyladenosine 3'-O-(2-cyanoethyl-N,N-diisopropyl)phosphoroamidite

In 10 ml of dry pyridine was dissolved 1.195 g (2 mmol) of 5'-O-trityl-6-N-benzoyladenosine and the resulting solution was subjected to azeotropic distillation to dryness by evaporating the pyridine under reduced pressure. The residue was dissolved in 10 ml of dry tetrahydrofuran, followed by addition of 1.39 ml (8 mmol) of diisopropylethylamine and stirring at room temperature in an argon atmosphere. To the mixture was added 0.892 ml (4 mmol) of 2-cyanoethyl N,N-diisopropylchlorophosphoroamidite, followed by stirring at room temperature for 30 minutes. After reaction, the precipitate was removed by filtration, and the solvent was distilled off under reduced pressure. The residue was dissolved in 100 ml of ethyl acetate and washed twice with 50 ml of ice-cooled 10% aqueous $Na_2CO_3$. After drying the organic layer over anhydrous $Na_2CO_3$, the drying agent was removed by filtration, and the solvent was distilled off under reduced pressure. The residue was applied to a 40 g silica gel column (70-230 mesh) and the desired substance

was eluted with a solution of ethyl acetate, methylene chloride and triethylamine (45:45:10). After distilling off the solvent, the desired substance was dissolved in 4 ml of toluene. This solution was then dropped into 200 ml of vigorously stirred hexane. The resulting precipitate was then collected by filtration. The thus obtained precipitate was then dissolved in methylene chloride followed by distilling off the solvent to obtain 1.04 g of the desired product in an amorphous state.

$^1$H-NMR (60MHz, $CDCl_3$) δppm:

9.82 (1H, bs, NH); 8.66 (1H, s, H-8); 8.18 (1H, s, H-2); 8.12-7.84 (2H, m, ortho-H of Bz); 7.62-7.05 (18H, m, Tr and m, p-H of Bz); 6.48 (1H, t, J=6Hz, 1'-H); 7.80 (1H, bs, 3'-H); 4.35 (1H, bs, 4'-H); 3.90-3.20 (6H, m), 3.05-2.23 (4H, m); 1.40-0.95 (12H, m, $(CH_3)_2CH$).

(3) Tr-AGGTGGGTCTGAAAC

In 1 ml of dry pyridine was dissolved 25 mg (30 µmol) of 5'-O-trityl-6-N-benzoyladenosine and the resulting solution was subjected to azeotropic distillation to dryness by distilling off the solvent under reduced pressure. The residue was dissolved in 150 µl of dry acetonitrile. Separately, 35 mg of 1H-tetrazole was dissolved in 1 ml of dry pyridine and the resulting solution was subjected to azeotropic distillation to dryness by distilling off the solvent under reduced pressure, and the residue was dissolved in 1 ml of dry acetonitrile. 180 µl of this solution was added to the above-mentioned solution of 5'-O-trityl-6-N-benzoyladenosine in acetonitrile, and this mixture was added to a solid phase carrier (1 µmol scale) supporting a DNA derivative, having the sequence of GGTGGGTCTGAAAC from the 5' side and having a free hydroxyl group at its 5' terminal, prepared in advance using an automated DNA synthesizer. After stirring this mixture of an ununiform system for 10 minutes, the solid was collected by filtration which was then washed with acetonitrile and pyridine. At this point, 1 ml of a previously prepared solution of acetic anhydride and pyridine (1:9, v/v) containing 0.1 M dimethylaminopyridine was added followed by stirring for 1 minute. After collecting the solid by filtration and washing with pyridine and methylene chloride, 1 ml of a previouysly prepared solution of tetrahydrofuran, lutidine and water (2:2:1, v/v/v) containing 0.1 M iodine was added followed by stirring for 1 minute. After collecting the solid by filtration, washing with pyridine and methylene chloride, and drying under reduced pressure, 20 ml of concentrated aqueous ammonia was added to the residue, followed by stirring for 1 day at room temperature and then 5 hours at 50 °C. After removing the solid by filtration, concentrating the filtrate under reduced pressure, removing the ammonia and washing three times with 10 ml of diethyl ether, the resulting aqueous solution was freeze-dried. The residue was then dissolved in 5 ml of water and then applied in several separate applications to preparative reverse phase HPLC (Inertsil PREP-ODS, 20 x 250 mm, 0.1 M $NH_4OAc$ (pH 7.0), 0-50% $CH_3CN$/50 min.: linear gradient) to obtain a fraction having a primary peak that eluted at 28.5 minutes. After applying this to Sep-pak and washing with 20 ml of water, desalting procedure was performed by eluting with 10 ml of a mixture of methanol and water (1:1, v/v) to obtain the desired substance of Tr-AGGTGGGTCTGAAAC having an optical density of 28 OD (260 nm) (approximately 1 mg).

The thus obtained desired substance (Tr-AGGTGGGTCTGAAAC) was subjected to high-performance liquid chromatography resulting in the confirmation of a single peak.

The conditions are described below:

| | |
|---|---|
| Flow rate: | 1 ml/min. |
| Column: | Inertsil ODS 2 4.6 x 150 mm |
| Solvent: | 0.1 M $NH_4OAC$ (pH 7) |
| Gradient: | 0-40% $CH_3CN$/40 min. (linear) |
| Retention Time: | 29.1 min. |
| UV: | Max. 256 nm, Min. 229 nm (solvent: $H_2O$) |

(Example 2)

Dimethoxy-Tr-TCGGGGTTGGGAGGT

Dimethoxy (hereinafter abbreviated as "Dm")-Tr-TCGGGGTTGGGAGGT was synthesized following the operation manual of Applied Biosystems Inc. using model 394 according to the phosphoroamidite method of the above firm. Purification of the compound was performed with preparative C18 reverse phase HPLC.

The behavior of this compound under the analysis conditions were as indicated below.

| | |
|---|---|
| Column used | ASAHI PAC ODP-50 |
| Flow rate: | 1 ml/min. |
| Solvent: | 0.1 M triethylamine-acetic acid (pH 7.0) acetonitrile 15 to 30%/18 min. |

Retention time: 14.9 minutes

(Example 3)

Dm-Tr-TGGGAGGTGGGTCTG was obtained according to the method of Example 2. The behavior of this compound as determined by reverse phase liquid chromatography was as indicated below.

Column used: ASAHI PAC ODP-50
Flow rate: 1 ml/min.
Solvent: 0.1 M triethylamine-acetic acid (pH 7.0)
acetonitrile 15 to 30%/18 min.
Retention time: 15.4 minutes

(Example 4)

Dm-Tr-TTGGGAGGTGGGTCT was obtained according to the method of Example 2. The behavior of this compound as determined by reverse phase liquid chromatography was as indicated below.

Column used: ASAHI PAC ODP-50
Flow rate: 1 ml/min.
Solvent: 0.1 M triethylamine-acetic acid (pH 7.0)
acetonitrile 15 to 30%/18 min.
Retention time: 15.4 minutes

(Example 5)

Dm-Tr-AGGTGGGTCTGAAAC was obtained according to the method of Example 2. The behavior of this compound as determined by reverse phase liquid chromatography was as indicated below.

Column used: ASAHI PAC ODP-50
Flow rate: 1 ml/min.
Solvent: 0.1 M triethylamine-acetic acid (pH 7.0)
acetonitrile 15 to 30%/18 min.
Retention time: 13.2 minutes

(Example 6)

Dm-Tr-ATACTCAGTCATTTTTAGCAG was obtained according to the method of Example 2. The behavior of this compound as determined by reverse phase liquid chromatography was as indicated below.

Column used: ASAHI PAC ODP-50
Flow rate: 1 ml/min.
Solvent: 0.1 M triethylamine-acetic acid (pH 7.0)
acetonitrile 20 to 40%/20 min.
Retention time: 14.3 minutes

(Example 7)

Dm-Tr-GTGCCGGGGTCTTCGGGC was obtained according to the method of Example 2. The behavior of this compound as determined by reverse phase liquid chromatography was as indicated below.

Column used: Senshu pak VP-304-4251
Flow rate: 5 ml/min.
Solvent: 0.1 M triethylamine-acetic acid (pH 7.0)
acetonitrile 18 to 30%/15 min.
Retention time: 10.8 minutes

(Example 8)

Dm-Tr-TGGGTCTGAAACGAT was obtained according to the method of Example 2. The behavior of this compound as determined by reverse phase liquid chromatography was as indicated below.

Column used: Senshu pak VP-304-4251
Flow rate: 5 ml/min.

Solvent: 0.1 M triethylamine-acetic acid (pH 7.0) acetonitrile 18 to 30%/15 min.
Retention time: 11.2 minutes

(Example 9)

Dm-Tr-TAGGTGGGTCTGAAA was obtained according to the method of Example 2. The behavior of this compound as determined by reverse phase liquid chromatography was as indicated below.

Column used: Senshu pak VP-304-4251
Flow rate: 5 ml/min.
Solvent: 0.1 M triethylamine-acetic acid (pH 7.0) acetonitrile 18 to 30%/15 min.
Retention time: 12.5 minutes

(Example 10)

Dm-Tr-GGTGGGTCTGAAACG was obtained according to the method of Example 2. The behavior of this compound as determined by reverse phase liquid chromatography was as indicated below.

Column used: COSMOSIL 5C18-AR (20 mm x 250 mm)
Flow rate: 5 ml/min.
Solvent: 0.1 M triethylamine-acetic acid (pH 7.0) acetonitrile 20 to 45%/15 min.
Retention time: 11.2 minutes

(Example 11)

Dm-Tr-GGTGGGTTGCTTTGA was obtained according to the method of Example 2. The behavior of this compound as determined by reverse phase liquid chromatography was as indicated below.

Column used: COSMOSIL 5C18-AR (20 mm x 250 mm)
Flow rate: 5 ml/min.
Solvent: 0.1 M triethylamine-acetic acid (pH 7.0) acetonitrile 20 to 45%/15 min.
Retention time: 10.8 minutes

(Example 12)

Dm-Tr-GGAGGTGGGTCTGAA was obtained according to the method of Example 2. The behavior of this compound as determined by reverse phase liquid chromatography was as indicated below.

Column used: COSMOSIL 5C18-AR (20 mm x 250 mm)
Flow rate: 9 ml/min.
Solvent: 0.1 M triethylamine-acetic acid (pH 7.0) acetonitrile 20 to 45%/15 min.
Retention time: 11.2 minutes

(Example 13)

Dm-Tr-GGGAGGTGGGTCTGA was obtained according to the method of Example 2. The behavior of this compound as determined by reverse phase liquid chromatography was as indicated below.

Column used: Senshu pak VP-304-4251
Flow rate: 5 ml/min.
Solvent: 0.1 M triethylamine-acetic acid (pH 7.0) acetonitrile 18 to 30%/15 min.
Retention time: 10.9 minutes

(Example 14)

Dm-Tr-GTTGGGAGGTGGGTC was obtained according to the method of Example 2. The behavior of this compound as determined by reverse phase liquid chromatography was as indicated below.

Column used: Senshu pak VP-304-4251

Flow rate: 5 ml/min.
Solvent: 0.1 M triethylamine-acetic acid (pH 7.0)
acetonitrile 18 to 30%/15 min.
Retention time: 11.5 minutes

(Example 15)

Dm-Tr-GGGTTGGGAGGTGGG was obtained according to the method of Example 2. The behavior of this compound as determined by reverse phase liquid chromatography was as indicated below.

Column used: Senshu pak VP-304-4251
Flow rate: 5 ml/min.
Solvent: 0.1 M triethylamine-acetic acid (pH 7.0)
acetonitrile 18 to 30%/15 min.
Retention time: 12.7 minutes

(Example 16) 5'-O-Benzyl ODN-14 (16c)

It should be noted that ODN-40 represents an $Et_3N$ salt of DNA having a sequence of TGGGAGGTGGGTCTG from the 5' terminal.

5'-O-Benzylthymidine (16a)

3'-O-[(1,1-dimethylethyl)diemthylsilyl]thymidine was synthesized in the same manner as described in Can. J. Chem., 56, 2768 (1978). In 4 ml of tetrahydrofuran was dissolved 713 mg (2 mmol) of 3'-O-[(1,1-dimethylethyl)dimethylsilyl] thymidine, followed by addition of 175 mg (4 mmol) of 55% NaH in an argon atmosphere and stirring at 60 ° C for 2 hours. After allowing the temperature to return to room temperature, a solution of 0.238 ml (2 mmol) of benzyl chloride dissolved in 1 ml of tetrahydrofuran was added dropwise thereto, followed by addition of 149.9 mg (1 mmol) of NaI and stirring at room temperature. After 21 hours, the solvent was distilled off under reduced pressure, and the residue was dissolved in 50 ml of ethyl acetate. After the resulting solution was washed twice with 50 ml of 0.1N HCl, the solution was dried over anhydrous magnesium sulfate. After the solvent was distilled off under reduced pressure, the residue was applied to a 30 g (70-230 mesh) silica gel column and eluted with 1 % methanol-methylene chloride to obtain 592 mg of 3'-O-[(1,1-dimethylethyl)dimethylsilyl]-5'-O-(benzyl)thymidine.
$^1$H-NMR (270MHz, $CDCl_3$) δppm:

9.99 (1H, s), 7.59 (1H, s), 7.38-7.26 (5H, m), 6.35 (1H, t, J = 5.94Hz), 4.58 (2H, s), 4.47 (1H, m), 3.98 (1H, bs), 3.85-3.60 (2H, m), 2.32-2.08 (2H, m, H2'), 1.60 (3H, s, CH3), 0.88 (9H, s, $(CH_3)_3C$), 0.07 (6H, s, $CH_3$-Si)

Next, the entire amount of the above compound was dissolved in 2.64 ml of tetrahydrofuran, and to the resulting solution was added 2.64 ml of a solution (1M) of tetrabutylammonium fluoride in tetrahydrofuran, followed by stirring at room temperature. After 30 minutes, the solvent was distilled off under reduced pressure, and the residue was dissolved in 50 ml of ethyl acetate, followed by washing twice with 50 ml of a saturated aqueous sodium chloride. After the resulting mixture was dried over anhydrous magnesium sulfate, the solvent was distilled off under reduced pressure, and the residue was applied to a 30 g (230-400 mesh) silica gel column and eluted with 2 to 3 % methanol-methylene chloride to obtain 392 mg of (16a) as crystals.
$^1$H-NMR (270MHz, $CDCl_3$, TMS) δppm:

7.59 (1H, s, H6), 7.35-7.22 (5H, m, Ph), 6.42 (1H, t, H1'), J = 6.59Hz), 4.56 (2H, s, $PhCH_2$), 4.51 (1H, bs, H3'), 4.13 (1H, bs, H4'), 3.80-3.65 (2H, m, H5') 2.42-2.12 (2H, m, H2'), 1.58 (3H, s, $CH_3$)

5'-O-Benzylthymidine-3'-O-(2-cyanoethyl N,N-diisopropyl)phosphoramidite (16b)

In pyridine 166 mg (0.5 mmol) of 16a was subjected to azeotropic distillation three times to dryness, followed by dissolving in 2.5 ml of tetrahydrofuran. To the resulting solution were added 0.348 ml (2 mmol) of diisopropylethylamine and 0.223 ml (1 ml) of 2-cyanoethyl N,N-diisopropylchlorophosphoroamidite, followed by stirring at room temperature in an argon atmosphere. After 30 minutes, precipitates were removed by filtration and the solvent was distilled off under reduced pressure. The residue was dissolved in 50 ml of ethyl acetate and washed twice with 50 ml of ice-cooled 10% aqueous $Na_2CO_3$, followed by drying over anhydrous sodium sulfate. After the solvent was distilled off under reduced pressure, the residue was

applied to a 30 g silica gel column (70-230 mesh) and eluted with methylene chloride : ethyl acetate : triethylamine (45 : 45 : 10, v/v/v) to obtain 271 mg of 16b.

$^1$H-NMR (270MHz, $CDCl_3$, TMS) δppm:

7.59, 7.56 (1H, 2s, H6), 7.38-7.26 (5H, m, Ph), 6.40 (1H, t, H1', J = 7.32Hz), 4.68-4.55 (1H, m, H3'), 4.60, 4.59 (2H, 2s, $PhCH_2$), 4.24, 4.18 (1H, 2m, H4') 3.90-3.55 (6H, m, H5', $POCH_2$, PNCH), 2.65, 2.58 (2H, 2t, $CH_2CN$), 2.52-2.15 (2H, m, H2'), 1.63 (3H, s, $CH_3$), 1.18 (12H, d $(CH_3)_2CH$)

5'-O-benzyl ODN-12 (16c)

A completely protected oligodeoxynucleotide derivative, having the sequence of GGGAGGTGGGTCTG from the 5'-terminal and formed on a controlled pore glass (CPG), and which was synthesized using an automatic synthesizer (Cyclone™ Plus), was purchased from Nippon Millipore Limited-Milligen/Biosearch.

After immersing 135 mg (approximately 5 μmol) of the above-mentioned compound in 2 ml of a 3% solution of dichloroacetic acid and methylene chloride for 1 minute, the compound was filtered with a glass filter, and the CPG beads were collected and washed with methylene chloride. These CPG beads were then dried by azeotropic distillation in pyridine.

Separately, 0.9 ml of a solution in which 42 mg of 1H-tetrazole, dried in advance by azeotropic distillation with pyridine, were dissolved in 1.2 ml of acetonitrile, were added to a solution in which 80 mg (0.15 mmol) of 16b were subjected to azeotropic distillation to dryness with pyridine and then dissolved in 0.75 ml of acetonitrile. The resulting solution was then added to the above-mentioned dried CPG beads and stirred at room temperature in an argon atmosphere. After 30 minutes, the mixture was filtered with a glass filter and the CPG beads were collected, followed by washing with pyridine. To the thus obtained beads was added 2 ml of a tetrahydrofuran solution containing 5% acetic anhydride, 5% 2,6-lutidine and 3% N-methylimidazole, followed by stirring at room temperature. After 1 minute, the CPG beads were collected by filtering with a glass filter and then washed with pyridine. To the resulting CPG beads was added 2 ml of a tetrahydrofuran, pyridine and water (40:20:1) solution containing 0.1 M iodine, followed by additional stirring at room temperature. After 1 minute, the CPG beads were collected by filtering with a glass filter, washed with pyridine and methylene chloride, and then dried under reduced pressure. Approximately 10 ml of concentrated aqueous ammonia was added to the resulting CPG beads followed by stirring overnight at room temperature and then stirring for 3 hours at 50°C. After this reaction, the CPG beads were removed by filtration and washed twice with 10 ml of water. After combining the filtrate and washings, the mixture was washed three times with 30 ml of diethylether, and the ammonia and diethylether were removed under reduced pressure. The resulting aqueous solution was freeze dried. The residue was dissolved in about 5 ml of 0.1 M an aqueous solution of triethylammonium acetate (TEAA) (pH 7.3), and insolubles were removed with a 0.45 micron millipore filter. The resulting solution was applied in three portions to reverse phase HPLC (Intertsil PREP-ODS, 20.0 x 250 mm, 0.1 M TEEA, pH 7.3; 10-40% $CH_3CN$/30 min., linear gradient; 9 ml/min.; 254 nm). The fraction was collected that eluted at 14.1 minutes. After removing the acetonitrile under reduced pressure and freeze drying, the residue was dissolved in 50 ml of water followed again by freeze drying to obtain amorphous 16c having an optical density of 126 OD (260 nm).

UV max: 256 nm

(Example 17) 5'-O-Benzhydryl ODN-12 (17c)

5'-O-Benzhydrylthymidine (17a)

In 8 ml of tetrahydrofuran was dissolved 1.426 g (4 mmol) of 3'-O-[(1,1-dimethylethyl)dimethylsilyl] thymidine, followed by addition of 350 mg of 55% NaH in an argon atmosphere and stirring at 60°C for 2 hours. After allowing the temperature to return to room temperature, a solution of 988 mg (4 mmol) of $Ph_2CHBr$ dissolved in 2 ml of tetrahydrofuran was added dropwise thereto, followed by addition of 300 mg (2 mmol) of NaI and stirring at room temperature. After 17 hours, the solvent was distilled off under reduced pressure after which the residue was dissolved in 50 ml of ethyl acetate. After the resulting solution was washed twice with 50 ml of a saturated aqueous sodium chloride, the solution was dried over anhydrous magnesium sulfate. After the solvent was distilled off under reduced pressure, the residue was dissolved in 4 ml of tetrahydrofuran followed by addition of 4 ml of a solution (1 M) of tetrabutylammonium fluoride in tetrahydrofuran and stirring at room temperature. After 2 hours, the solvent was distilled off under reduced pressure, the residue was dissolved in 50 ml of ethyl acetate, and then washed twice with 50 ml of a saturated aqueous sodium chloride. After the organic layer was dried over anhydrous magnesium sulfate, the solvent was distilled off under reduced pressure and the residue was applied to a 60 g (230-400 mesh)

silica gel column to obtain 377.7 mg (23 %) of 17a by elution with 1 to 2.5% methanol-methylene chloride.
$^1$H-NMR (270MHz, $CDCl_3$, TMS) δppm:

9.85 (1H, bs, NH), 7.56 (1H, s, H6), 7.38-7.20 (10H, m, Ph), 6.45 (1H, t, H1', J=6.92Hz), 5.40 (1H, s, $Ph_2\underline{CH}$), 4.62-4.58 (1H, m, H3'), 4.17-4.15 (1H, m, H4'), 3.75-3.58 (2H, m, H5'), 2.47-2.22 (2H, m, H2'), 1.36 (3H, s, $CH_3$)

5'-O-Benzhydrylthymidine-3'-O-(2-cyanoethyl N,N-diisopropyl)phosphoramidite (17b)

The procedure of Example 16 was repeated analogously using 204.2 mg (0.5 mmol) of Compound 17a to give 213.4 mg (70%) of 17b.
$^1$H-NMR (270MHz, $CDCl_3$, TMS) δppm:

7.55, 7.51 (1H, 2s, H6), 7.43-7.22 (10H, m, Ph), 6.48-6.42 (1H, m, H1'), 5.44, 5.42 (1H, 2s, $Ph_2\underline{CH}$), 4.73-4.64 (1H, m, H3'), 4.25, 4.19 (1H, 2bs, H4'), 3.90-3.55 (6H, m, H5', $PO\underline{CH_2}$, $PN\underline{CH}$), 2.68-2.24 (4H, m, H2', $NC\underline{CH_2}$), 1.39 (3H, s, $CH_3$), 1.30-1.10 (12H, m, $(\underline{CH_3})_2CH$)

5'-O-Benzhydryl ODN-12 (17c)

Compound 17c was synthesized according to the same procedure as in Example 16 using 91 mg (0.15 mmol) of Compound 17b. For purification, Compound 17c was applied in three portions to reverse phase HPLC (Inertsil PREP-ODS, 20.0 x 250 mm; 0.1 M TEAA, pH 7.3; 0-40% $CH_3CN$/40 min,, linear gradient; 9 ml/min.; 254 nm), and the fraction was collected that was eluted at 29.2 minutes. After the acetonitrile was distilled off under reduced pressure, the residue was freeze-dried, and it was dissolved in 50 ml of water followed again by freeze drying to obtain amorphous 17c having an optical density of 130 OD (260 nm).

UVmax: 265 nm

(Example 18) 5'-O-Trityl ODN-12 (18c)

5'-O-Tritylthymidine-3'-O-(2-cyanoethyl N,N-diisopropyl)phosphoramidite (18b)

5'-O-tritylthymidine (18a) was synthesized in the same manner as the method described in J. Am. Chem. Soc., 80, 6212 (1958). The procedure of Example 16 was repeated analogously using 969 mg (2 mmol) of Compound 18a to obtain 1.35 g (98 %) of 18b.
$^1$H-NMR (270MHz, $CDCl_3$, TMS) δppm:

7.62, 7.57 (1H, 2s, H6), 7.46-7.20 (15H, m, Ph), 6.46-6.37 (1H, m, H1'), 4.68 (1H, bs, H3'), 4.19 and 4.15 (1H, 2bs, H4'), 3.90-3.30 (6H, m, H5', $PO\underline{CH_2}$, PNCH), 2.63-2.28 (4H, m, H2', $NC\underline{CH_2}$), 1.47 (3H, s, $CH_3$), 1.23-1.00 (12H, m, $(\underline{CH_3})CH$)

5'-O-Trityl ODN-12 (18c)

Compound 18c was synthesized in the same manner as Example 16 on a scale of 15 µmol using 308 mg (0.45 mmol) of Compound 18b. For purification, Compound 18c was applied in ten portions to reverse phase HPLC (Inertsil PREP-ODS, 20.0 x 250 mm; 0.1 M TEAA, pH 7.3; 20-50% $CH_3CN$/20 min., linear gradient; 9 ml/min.; 254 nm), and the fraction was collected that eluted at 12.6 minutes. After the acetonitrile was distilled off under reduced pressure and the residue was freeze-dried, it was dissolved in 50 ml of water followed again by freeze drying to obtain amorphous 18c having an optical density of 464 OD (260 nm).

UVmax: 256 nm

(Example 19) 5'-O-(4-Methoxytrityl) ODN-12 (19c)

5'-O-(4-Methoxytrityl)thymidine-3'-O-(2-cyanoethyl N,N-diisopropyl)phosphoramidite (19b)

5'-O-(4-Methoxytrityl)thymidine (19a) was synthesized in the same manner as the method described in J. Am. Chem. Soc., 85, 3821 (1963). The procedure of Example 16 was repeated analogously using 257.3 g (0.5 mmol) of Compound 19a to obtain 261.9 mg (73 %) of 19b.
$^1$H-NMR (270MHz, $CDCl_3$, TMS) δppm:

7.64, 7.59 (1H, 2s, H6), 7.46-7.20, 6,88-6.82 (14H, m, Ph), 6.46-6.39 (1H, m, H1'), 4.73-4.62 (1H, m, H3'), 4.19, 4.15 (1H, 2bs, H4'), 3.90-3.30 (6H, M, H5', $PO\underline{CH_2}$, $PN\underline{CH}$), 3.78 (3H, s, $\underline{CH_3}O$), 2.66-2.29 (4H, m,

H2', NCCH2), 1.46 (3H, s, $CH_3$), 1.17 (12H, d, $(CH_3)_2CH$, J = 7.26Hz)

5'-O-(4-Methoxytrityl) ODN-12 (19c)

Compound 19c was synthesized according to the same method as in Example 16 using 107 mg (0.15 mmol) of Compound 19b. For purification, Compound 19c was applied in three portions to reverse phase HPLC (Inertsil PREP-ODS 20.0 x 250 mm; 0.1 M TEAA, pH 7.3; 20-50% $CH_3CN$/30 min., linear gradient; 9 ml/min.; 254 nm), and the fraction was collected that eluted at 14.4 minutes. After the acetonitrile was distilled off under reduced pressure, the residue was freeze-dried, and it was dissolved in 50 ml of water followed again by freeze drying to obtain amorphous 19c having an optical density of 91 OD (260 nm).

UVmax: 256 nm

(Example 20) 5'-O-(3,5-Dibenzyloxy)benzyl ODN-12 (20c)

5'-O-(3,5-Dibenzyloxy)benzylthymidine (20a)

3,5-(Dibenzyloxy)benzylbromide was synthesized by the same method described in Chem. Ber., 102, 2887 (1969). The procedure of Example 17 was repeated analogously using 713 mg (2 mmol) of 3'-O-[(1,1-dimethylethyl) dimethylsilyl]thymidine and 767 mg (2 mmol) of 3,5-(dibenzyloxy)benzylbromide to obtain 258.5 mg (23%) of 20a.

$^1$H-NMR (270 MHz, $CDCl_3$, TMS) δppm:

7.87 (1H, s, NH), 7.52 (1H, s, H6), 7.43-7.27, 6.59-6.52 (13H, m, Ph), 6.37 (1H, t, H1', J = 6.75Hz), 5.03 (4H, s, $PhCH_2$), 4.51 (2H, d, $PhCH_2OCH_2$, J = 3.30Hz), 4.50-4.44 (1H, m, H3'), 4.06-4.03 (1H, m, H4'), 3.77-3.63 (2H, m, H5'), 2.32-2.12 (2H, m, H2'), 1.67 (3H, s, $CH_3$)

5'-O-(3,5-Dibenzyloxy)benzylthymidine-3'-O-(2-cyanoethyl-N,N -diisopropyl)phosphoramidite (20b)

The procedure of Example 17 was repeated analogously using 258.5 mg (0.475 mmol) of Compound 20a to give 307.7 mg (87%) of 20b.

$^1$H-NMR (270 MHz, $CDCl_3$ , TMS) δppm:

7.56, 7.53 (1H, 2s, H6), 7.42-7.28, 6.56 (13H, m, Ph), 6.40 (1H, t, H1', J = 6.60Hz), 5.02 (4H, s, $PhCH_2$), 4.67-4.58 (1H m, H3'), 4.53, 4.51 (2H, 2s, $PhCH_2OCH_2$), 4.23, 4.17 (1H, 2bs, H4'), 3.90-3.52 (6H, m, H5' $POCH_2$, PNCH), 2.68-2.53 (2H, m, $NCCH_2$), 2.49-2.12 (2H, m, H2'), 1.65 (3H, s, $CH_3$), 1.18 (12H, d, $(CH_3)_2CH$, J = 5.94Hz)

5'-O-(3,5-Dibenzyloxy)benzyl ODN-12 (20c)

Compound 20c was synthesized according to the same procedure as in Example 17 using 112 mg (0.15 mmol) of Compound 20b. For purification, Compound 20c was applied in 3 separate applications to reverse phase HPLC (Inertsil PREP-ODS, 20.0 x 250 mm; 0.1 M TEAA, pH 7.3; 20-50% $CH_3CN$/30 min., linear gradient; 9 ml/min.; 254 nm), and the fraction was collected that eluted at 15.7 minutes. After the acetonitrile was distilled off under reduced pressure, the residue was freeze-dried, and it was dissolved in 50 ml of water followed again by freeze drying to obtain amorphous 20c having an optical density of 85 OD (260 nm).

UVmax: 256 nm

(Example 19)

5'-O-[3,5-Bis§3,5-(dibenzyloxy)benzyloxy¥benzyl] ODN-12 (21c)

5'-O-[3,5-Bis§3,5-(dibenzyloxy)benzyloxy¥benzyl]thymidine (21a)

3,5-Bis§3,5-(dibenzyloxy)benzyloxy¥benzylbromide was synthesized by the same method described in Chem. Ber., 102, 2887 (1969). The procedure of Example 17 was repeated analogously using 713 mg (2 mmol) of 3'-O-[(1,1-dimethyiethyl) dimethylsilyl]thymidine and 1.61 g (2 mmol) of 3,5-bis§3,5-(dibenzyloxy)-benzyloxy¥benzylbromide to obtain 381 mg (20%) of 21a.

$^1$H-NMR (270 MHz, $CDCl_3$, TMS) δppm:

7.86 (1H, s, NH), 7. 52(1H, s, H6), 7.45-7.28, 6.68-6.50 (29H, m, Ph), 6.35 (1H, t, H1', J = 8.10Hz), 5.03

(8H, s, $PhCH_2$), 4.98 (4H, s, $PhCH_2$), 4.50 (2H, dd, $PhCH_2$), 4.42-4.38 (1H, m, H3'), 4.03-3.98 (1H, m, H4'), 3.73-3.59 (2H, m, H5'), 2.30-2.09 (2H, m, H2'), 1.70 (3H, s, $CH_3$)

5'-O-[3,5-Bis§3,5-(dibenzyloxy)benzyloxy¥benzyl]thymidine-3' -O-(2-cyanoethyl N,N-diisopropyl)-phosphoramidite (21b)

The procedure of Example 18 was repeated analogously using 242 mg (0.25 mmol) of Compound 21a to give 146 mg (50%) of 21b.
$^1$H-NMR (270 MHz, $CDCl_3$, TMS) δppm:
7.56, 7.53 (1H, 2s, H6), 7.45-7.28, 6.66, 6.58-6.52 (29H, m, Ph), 5.01 (8H, s, $PhCH_2$), 4.95 (4H s, $PhCH_2$), 4.66-4.57 (1H, m, H3'), 4.52, 4.51 (2H, 2s, $PhCH_2$), 4.22, 4.16 (1H, 2bs, H4'), 3.83-3.34 (6H, m, H5', $POCH_2$, PNCH), 2.69-1.77 (4H, m, H2', $NCCH_2$), 1.66 (3H, s, $CH_3$), 1.30-1.08 (12H, m, $(CH_3)_2CH$)

5'-O-[3,5-Bis§3,5-(dibenzyloxy)benzyloxy¥benzyl] ODN-12 (21c)

Compound 21c was synthesized according to the same procedure as in Example 18 using 146 mg (0.125 mmol) of Compound 21b. For purification, Compound 21c was applied in two portions to reverse phase HPLC (Inertsil PREP-ODS, 20.0 x 250 mm; 0.1 M TEAA, pH 7.3; 20-70% $CH_3CN$/50 min., linear gradient; 9 ml/min.; 254 nm), and the fraction was collected that eluted at 36.6 minutes. After the acetonitrile was distilled off under reduced pressure, the residue was freeze-dried, and it was dissolved in 50 ml of water followed again by freeze drying to obtain amorphous 21c having an optical density of 59 OD (260 nm).
UVmax: 256 nm

(Example 22) 5'-O-(2-Naphthylmethyl) ODN-12 (22c)

5'-O-(2-Naphthylmethyl)thymidine (22a)

The procedure of Example 17 was repeated analogously using 480.5 mg (1.35 mmol) of 3'-O-[(1,1-dimethylethyl)dimethylsilyl]thymidine and 298 mg (1.35 mmol) of 2-bromomethylnaphthalene to obtain 179.5 mg (35%) of 22a.
$^1$H-NMR (270 MHz, $CDCl_3$-$CD_3OD$, TMS) δppm:
7.88-7.77, 7.53-7.43 (7H, m, naphtyl), 7.61 (1H, s, H6), 6.35 (1H, t, H1', J = 6.60Hz), 4.76 (2H, s, $CH_2$), 4.52-4.47 (1H, m, H3'), 4.11-4.09 (1H, m, H4'), 3.89-3.71 (2H, m, H5'), 2.38-2.14 (2H, m, H2'), 1.54 (3H, s, $CH_3$)

5'-O-(2-Naphthylmethyl)thymidine-3'-O-(2-cyanoethyl N,N-diisopropyl)phosphoramidite (22b)

The procedure of Example 16 was repeated analogously using 179.5 mg (0.47 mmol) of Compound 22b to give 165 mg (60%) of 22b.
$^1$H-NMR (270 MHz, $CDCl_3$, TMS) δppm:
7.88-7.77 and 7.52-7.42 (7H, m, naphtyl), 7.60, 7.57 (1H, 2s, H6), 6.40 (1H, t, H1', J = 5.94Hz), 4.81-4.71 (2H, m, $CH_2$), 4.67-4.58 (1H, m, H3'), 4.24, 4.18 (1H, 2bs, H4'), 3.90-3.50 (6H, m, H5', $POCH_2$, PNCH), 2.68-2.15 (4H, m, H2', $NCCH_2$), 1.58, 1.56 (3H, 2s, $CH_3$) 1.17 (12H, d, $(CH_3)_2CH$, J = 6.60Hz)

5'-O-(2-Naphthylmethyl) ODN-12 (22c)

Compound 22c was synthesized according to the same procedure as in Example 16 using 87 mg (0.15 mmol) of Compound 22b. For purification, Compound 22c was applied in two portions to reverse phase HPLC (Inertsil PREP-ODS, 20.0 x 250 mm; 0.1 M TEAA, pH 7.3; 10-40% $CH_3CN$/30 min., linear gradient; 9 ml/min.; 254 nm), and the fraction was collected that eluted at 17.6 minutes. After the acetonitrile was distilled off under reduced pressure, the residue was freeze-dried, and it was dissolved in 50 ml of water followed again by freeze drying to obtain amorphous 22c having an optical density of 141.6 OD (260 nm).
UVmax: 256 nm

(Example 23) 5'-Benzylthio-5'-deoxy ODN-12 (23c)

5'-Benzylthio-5'-deoxythymidine (23a)

In acetone (20 ml) was dissolved 5'-deoxy-5'-mercaptothymidine (775 mg, 3.0 mmol), followed by addition of benzylbromide (564 mg, 3.3 mmol) and sodium carbonate (660 mg, 6.6 mmol) and stirring at room temperature for 17 hours in a drying atmosphere. After insoluble salt was removed by filtration, the solvent was distilled off under reduced pressure. The residue was dissolved in methylene chloride (40 ml), and the resulting solution was washed twice with 20 ml of water, followed by drying over anhydrous magnesium sulfate. The solvent was distilled off under reduced pressure to obtain caramelous residue. This was dissolved in 10 ml of ethanol and left to stand in a refrigerator overnight to give 451 mg of the desired compound in white prism crystals.
$^1$H-NMR (270 MHz, DMSO-$d_6$) δppm:
11.28 (1H, s, NH), 7.46 (1H, s, H6), 7.46-7.21 (5H, m, Ph) 6.61 (1H, t, J=6.8, 7.3Hz, H1'), 5.32 (1H, d, 4.4Hz, OH,), 4.15 (1H, m, H3'), 3.83 (1H, m, H4'), 3.78 (2H, s, PhC$H_2$), 2.78-2.59 (2H, m, H5'), 2.25-2.00 (2H, m, H2'), 1.77 (3H, s, C$H_3$)

5'-Benzylthio-5'-deoxythymidine-3'-O-(2-cyanoethyl N,N-diisopropyl)phosphoramidite (23b)

The procedure of Example 16 was repeated analogously using 174 mg (0.5 mmol) of Compound 23a to give 262 mg (95%) of 23b.
$^1$H-NMR (270 MHz, $CDCl_3$, TMS) δppm:
7.37 (1H, s, H6), 7.34-7.20 (5H, m, Ph), 6.32-6.25 (1H, m, H1'), 4.49-4.38 (1H, m, H3'), 4.18-4.08 (1H, m, H4'), 3.90-3.50 (6H, m, PhC$H_2$, POC$H_2$, PNCH), 2.87-2.68 (2H, m, H5'), 2.67-2.56 (2H, m, NCC$H_2$), 2.55-2.12 (2H, m, H2'), 1.91 (3H, s, C$H_3$), 1.20-1.15 (12H, m, (C$H_3$)CH)

5'-Benzylthio-5'-deoxy ODN-12 (23c)

Compound 23c was synthesized according to the same procedure as in Example 16 using 82 mg (0.15 mmol) of Compound 23b. For purification, Compound 23c was applied in three portions to reverse phase HPLC (Inertsil PREP-ODS, 20.0 x 250 mm; 0.1 M TEAA, pH 7.3; 0-40% $CH_3CN$/40 min., linear gradient; 9 ml/min.; 254 nm), and the fraction was collected that eluted at 25.8 minutes. After the acetonitrile was distilled off under reduced pressure, the residue was freeze-dried, and it was dissolved in 50 ml of water followed again by freeze drying to obtain amorphous 23c having an optical density of 46 OD (260 nm).
UVmax: 256 nm

(Example 24) 5'-Diphenylmethylthio-5'-deoxy ODN-12 (24c)

5'-Diphenylmethylthio-5'-deoxythymidine (24a)

In acetone (320 ml) was dissolved 5'-deoxy-5'-mercaptothymidine (516 mg, 2.0 mmol), followed by addition of diphenylmethyl bromide (741 mg, 3.0 mmol) and sodium carbonate (1.0 g) and refluxing for 5 hours in a drying atmosphere. After confirming the absence of the starting substance with TLC (using methylene chloride containing 10% methanol as the developer), insolubles were removed by filtration. After distilling off the solvent under reduced pressure and dissolving the residue in a small amount of methylene chloride, the solution was applied to a silica gel column and purified by allowing to flow off with methylene chloride containing 5% methanol. The residue obtained by collecting the major peak and concentrating to dryness was crystallized from ethanol to obtain 334 mg of the desired product in the form of a colorless powdery crystal.
$^1$H-NMR (270 MHz, DMOS-$d_6$) δppm:
11.28 (1H, s, NH), 7.47-7.20 (11H, m, H6, Bzh-ph), 6.15 (1H, t, J=6.8Hz, H1'), 5.37-5.32 (2H, m, OH, $Ph_2$CH), 4.16-4.12 (1H, m, H3'), 3.85-3.79 (1H, m, H4'), 2.71-2.50 (2H, m, H5'), 2.21-2.00 (2H, m, H2'), 1.79 (3H, s, C$H_3$)

5'-Diphenylmethylthio-5'-deoxythimydine-3'-O-(2-cyanoethyl N,N-diisopropyl)phosphoramidite (24b)

The procedure of Example 16 was repeated analogously using 212 mg (0.5 mmol) of Compound 24a to give 253.7 mg (81%) of 24b.

$^1$H-NMR (270 MHz, $CDCl_3$, TMS) δppm:

7.47-7.17 (11H, m, Ph, H6), 6.35-6.27 (1H, m, H1'), 5.29, 5.27 (1H, 2s, $Ph_2CH$) 4.50-4.40 (1H, m, H3'), 4.19-4.10 (1H, m, H4'), 3.92-3.50 (4H, m, $POCH_2$, PNCH), 2.80-2.10 (6H, m, H2', H5', $NCCH_2$), 1.77 (3H, s, $CH_3$), 1.20-1.15 (12H, m, $(CH_3)CH$)

5'-Diphenylmethylthio-5'-deoxy ODN-12 (24c)

Compound 24c was synthesized according to the same procedure as in Example 16 using 94 mg (0.15 mmol) of Compound 24b. For purification, Compound 24c was applied in three portions to reverse phase HPLC (Inertsil PREP-ODS, 20.0 x 250 mm; 0.1 M TEAA, pH 7.3; 0-50% $CH_3CN$/50 min., linear gradient; 9 ml/min.; 254 nm), and the fraction was collected that eluted at 30.3 minutes. After the acetonitrile was distilled off under reduced pressure, the residue was freeze-dried, and it was dissolved in 50 ml of water followed again by freeze drying to obtain amorphous 24c having an optical density of 73 OD (260 nm).

UVmax: 256 nm

(Example 25) 5'-Triphenylmethylthio-5'-deoxy ODN-12 (25c)

5'-Triphenylmethylthio-5'-deoxythymidine (25a)

In dry pyridine (20 ml) was dissolved 5'-deoxy-5'-mercaptothymidine (775 mg, 3.0 mmol), and the resulting solution was dissolved in trityl chloride (920 mg, 3.3 mmol), followed by stirring at 50°C for 2 hours. After water (1 ml) was added to the solution and the resulting mixture was stirred at room temperature for 15 minutes, the solvent was distilled off under reduced pressure. The residue was then dissolved in methylene chloride (50 ml) and then washed with 40 ml each of a saturated aqueous sodium chloride, 0.2 N HCl and a saturated aqueous sodium chloride. Each of the aqueous layers was then sequentially washed with 20 ml of methylene chloride, and then combined with the organic layer and dried over anhydrous magnesium sulfate. The solvent was then concentrated to dryness to obtain a yellowish-white residue. This residue was dissolved in a small amount of methylene chloride and applied to silica gel column chromatography packed with methylene chloride. After eluting with a mixed solvent of cyclohexane and ethyl acetate in a ratio of 2:1, the major peak was collected and concentrated to dryness to obtain 437 mg of the desired substance in the form of a caramel-like substance producing a single spot in TLC (using methylene chloride containing 5% methanol as the developer).

$^1$H-NMR (270 MHz, DMSO-$d_6$) δppm:

11.28 (1H, s, NH), 7.40-7.20 (16H, m, H6, $Ph_3C$), 6.07 (1H, dd, J=6.84, 7.32Hz, H1'), 5.26 (1H, d, J=4.40Hz, OH), 3.99-3.94 (1H, m, H3'), 3.60-3.55 (1H, m, H4'), 2.50-2.30 (2H, m, H5'), 2.18-1.94 (2H, m, H2'), 1.75 (3H, s, $CH_3$)

5'-Triphenylmethylthio-5'-deoxythimydine-3'-O-(2-cyanoethyl N,N-diisopropyl)phosphoramidite (25b)

The procedure of Example 16 was repeated analogously using 250 mg (0.5 mmol) of Compound 25b to give 231 mg (66%) of 25b.

$^1$H-NMR (270 MHz, $CDCl_3$, TMS) δppm:

7.48-7.15 (16H, m, H6, $Ph_3C$), 6.27-6.17 (1H, m, H1'), 4.22-4.19 (1H, m, H3'), 4.06, 4.00 (1H, 2bs, H4'), 3.85-3.40 (4H, m, $POCH_2$, PNCH), 2.67-1.95 (6H, m, H2', H5', $NCCH_2$), 1.83 (3H, s, $CH_3$), 1.20-1.10 (12H, m, $CH_3)_2CH$)

5'-Triphenylmethylthio-5'-deoxy ODN-12 (25c)

Compound 25c was Synthesized according to the same procedure as in Example 1 on a scale of 2 µmol using 42 mg (0.06 mmol) of Compound 25b. For purification, Compound 25c was applied in two portions to reverse phase HPLC (Inertsil PREP-ODS, 20.0 x 250 mm; 0.1 M TEAA, pH 7.3; 20-50% $CH_3CN$/20 min., linear gradient; 9 ml/min.; 254 nm), and the fraction was collected that eluted at 13.1 minutes. After the acetonitrile was distilled off under reduced pressure, the residue was freeze-dried, and it was dissolved in 50 ml of water followed again by freeze drying to obtain amorphous 25c having an optical density of 38 OD (260 nm).

UVmax: 256 nm

(Example 26) 5'-Hexadecylthio-5'-deoxy (ODN)-12 (26b)

5'-Hexadecylthio-5'-deoxythymidine (26a)

In acetone (20 ml) was dissolved 5'-deoxy-5'-mercaptothymidine (258 mg, 1 mmol), and to the resulting solution were added hexadecyl bromide (457 mg, 1.5 mmol) and sodium carbonate (500 mg), followed by refluxing for 3 hours in a drying atmosphere.

Insolubles were removed by filtration and the solvent was distilled off under reduced pressure. The residue was dissolved in a small amount of methylene chloride and the solution was applied to silica gel column chromatography packed with methylene chloride and eluted with methylene chloride containing 5% methanol. The major peak was collected and the solvent was distilled off to obtain 303 mg of the desired product in the form of a colorless caramel-like substance.

$^1$H-NMR (270 MHz, DMSO-$d_6$) δppm:

11.28 (1H, s, NH), 6.61 (1H, dd, J = 6.35, 7.82Hz, H1'), 5.31 (1H, d, J = 4.40Hz, OH), 4.19-4.12 (1H, m, H3'), 3.84-3.79 (1H, m, H4'), 2.83-2.68 (2H, m, H5'), 2.58-2.47 (2H, m, $CH_2$), 2.25-2.00 (2H, m, H2'), 1.79 (3H, s, 5$C\underline{H}_3$), 1.57-1.45 (2H, m, $CH_2$), 1.30-1.23 (26H, s, $C\underline{H}_2$), 0.85 (3H, t, J = 6.83Hz, $C\underline{H}_3$)

5'-Hexadecylthio-5'-deoxythymidine-3'-O-(2-cyanoethyl N,N-diisopropyl)phosphoramidite (26b)

The procedure of Example 16 was repeated analogously using 241 mg (0.5 mmol) of Compound 26a to give 193 mg (56%) of 26b.

$^1$H-NMR (270 MHz, $CDCl_3$, TMS) δppm:

7.46-7.41 (1H, 2s, H6), 6.29 (1H, t, H1', J = 7.26Hz), 4.55-4.45 (1H, m, H3'), 4.22-4.12 (1H, m, H4'), 3.92-3.54 (4H, m, $POC\underline{H}_2$, PNCH), 2.93-2.17 (6H, m, H2', H5', $NCCH_2$), 1.93 (3H, s, $CH_3$), 1.60 (2H, bs, $C\underline{H}_2S$), 1.26 (30H, s, $CH_2$), 1.20 (12H, d, $(C\underline{H}_3)_2CH$, J = 6.60Hz), 0.88 (3H, t, $CH_3$, J = 7.00Hz)

5'-Hexadecylthio-5'-deoxy ODN-12 (26c)

Compound 26c was synthesized according to the same procedure as in Example 16 using 102 mg (0.15 mmol) of Compound 26b. For purification, Compound 26c was applied in three portions to reverse phase HPLC (Inertsil PREP-ODS, 20.0 x 250 mm; 0.1 M TEAA, pH 7.3; 10-70% $CH_3CN$/60 min., linear gradient; 9 ml/min.; 254 nm), and the fraction was collected that eluted at 42.3 minutes. After the acetonitrile was distilled off under reduced pressure, the residue was freeze-dried, and it was dissolved in 50 ml of water followed again by freeze drying to obtain amorphous 26c having an optical density of 81 OD (260 nm).

UVmax: 256 nm

(Example 27) 5'-(12-Triphenylmethyloxydodecylthio)-5'-deoxy ODN-122 (27a)

5'-(12-Triphenylmethyloxydodecylthio)-5'-deoxythymidine (31a)

In acetone (30 ml) was dissolved 5'-deoxy-5'-mercaptothymidine (516 mg, 2.0 mmol), followed by addition of 12-hydroxydodecyl bromide (1.06 g, 4 mmol) and sodium carbonate (2 g) and refluxing for 3 hours in a drying atmosphere. After confirming that the starting substance was no longer present with TLC (using methylene chloride containing 5% methanol as the developer) and filtering out insolubles, the solvent was distilled off under reduced pressure. The residue was applied to silica gel column chromatography packed with methylene chloride and eluted with methylene chloride containing 3% methanol. The major peak was collected and the solvent was distilled off to obtain 712 mg of 5'-deoxy-5'-(12-hydroxydodecylthio)thymidine in the form of a colorless caramel-like substance. This caramel-like substance (589 mg, 1.30 mmol) was dissolved in anhydrous pyridine (30 ml) followed by addition of trityl chloride (541 mg, 1.95 mmol) and stirring at 70 °C for 4 hours in a drying atmosphere. The solvent was then distilled off under reduced pressure and the residue was dissolved in methylene chloride (30 ml), and the resulting solution was washed with 30 ml each of a saturated aqueous sodium chloride, 0.2 N HCl, a saturated aqueous sodium chloride and a saturated aqueous sodium hydrogencarbonate. The aqueous layers were then Sequentially washed with 20 ml of methylene chloride, combined with the organic layer and dried over anhydrous magnesium sulfate. The solvent was then concentrated to dryness under reduced pressure. The residue was dissolved in a small amount of methylene chloride and applied to silica gel column chromatography packed with methylene chloride and eluted with methylene chloride containing 5%

methanol. The major peak was collected and the solvent was distilled off. The residue was then freeze dried with cyclohexane to obtain 757 mg of the desired compound in the form of a yellowish-white powdery substance.

$^1$H-NMR (270 MHz, DMSO-$d_6$ ] δppm:

11.28 (1H, s, NH), 7.50 (1H, s, 6H), 7.38-7.21 (15H, m, Ph), 6.16 (1H, t, J = 6.34Hz, H1'), 5.31 (1H, d, J = 4.4Hz, OH), 4.17-4.14 (1H, m, H3'), 3.83-3.80 (1H, m, H4'), 2.95 (2H t, J = 6.35, $CH_2$), 2.77-2.73 (2H, m, H5'), 2.20-2.05 (2H, m, H2'), 1.78 (3H, s, $CH_3$),1.10-1.60 (20H, m, $CH_2$)

5'-(12-Triphenylmethyloxydodecylthio)-5'-deoxythymidine-3'-O-(2-cyanoethyl N,N-diisopropyl)-phosphoramidite (27b)

The procedure of Example 16 was repeated analogously using 342 mg (0.5 mmol) of Compound 27a to give 423.8 mg (96%) of 31b.

$^1$H-NMR (270 MHz, $CDCl_3$, TMS) δppm:

7.55-7.22 (16H, m, H6, $Ph_3C$), 6.36 (1H, t, H1', J = 6.60Hz), 4.61-4.51 (1H, m, H3), 4.30-4.12 (1H, m, H4'), 3.97-3.55 (4H, m, $POCH_2$, PNCH), 3.10 (2H, t, $CH_2$, J = 6.40Hz), 2.99-2.20 (8H, m, H2', H5', $NCCH_2$, $CH_2S$), 1.97 (3H, s, $CH_3$), 1.72-1.25 (10H, m, $CH_2$), 1.25 (12H, d, $(CH_3)_2CH$, J = 7.26Hz), 1.09 (3H, t, $CH_3$, J = 7.26Hz)

5'-(12-Triphenylmethyloxydodecylthio)-5'-deoxy ODN-12 (27c)

Compound 27c was synthesized according to the same procedure as in Example 16 using 133 mg (0.15 mmol) of Compound 27b. For purification, Compound 26b was applied in three portions to reverse phase HPLC (Inertsil PREP-ODS, 20.0 x 250 mm; 0.1 M TEAA, pH 7.3; 20-70% $CH_3CN$/50 min., linear gradient; 9 ml/min.; 254 nm), and the fraction was collected that eluted at 36.0 minutes. After the acetonitrile was distilled off under reduced pressure, the residue was freeze-dried, and it was dissolved in 50 ml of water followed again by freeze drying to obtain amorphous 27c having an optical density of 48 OD (260 nm).

UVmax: 256 nm

Example 28 Phosphorothioate type DmTrODN-12 of the compound of Example 3

The phosphorothioate type DmTrODN-12 was synthesized using the model 394 according to the phosphoroamidite method available from Applied Biosystems Inc. following the User's Manual from the above company. Purification of the compound was performed using the preparative C18 reverse phase HPLC. The conditions are as follows.

| | |
|---|---|
| Flow rate: | 9 ml |
| Column: | COSMOSIL 5C18-AR (20 x 250 mm) |
| Solvent: | 0.1 M triethylamine acetic acid (pH 7.0) acetonitrile 15 to 30 % |
| Retention time: | 14.04 min, 14.63 min. |

(Comparative example 1)

Dm-Tr-CAAAGTCTGGGTGGA (comparative compound No. 1) was obtained following the procedure of Example 2. The behavor of this compound in the reverse phase liquid chromatography was as follows.

| | |
|---|---|
| Column used: | Waters Microbondapack C18 |
| Flow rate : | 1 ml/min |
| Solvent: | 0.1 M triethylamine-acetic acid (pH 7.0) acetonitrile 15 to 30 %/20 min |
| Retention time: | 16.6 min |

(Comparative example 2)

Dm-Tr-GTCTGGGTGGAGGGT was synthesized in the same manner as in Comparative example 1.

(Comparative example 3)

Dm-Tr-ACCCTCCACCCAGAC was synthesized in the same manner as in Comparative example 1.

(Comparative example 4)

Dm-Tr-TTTTTTTTTTTTTTT was Synthesized in the same manner as in Comparative example 1.

(Test Example 1)

Inhibitory Effects on proliferation of HIV

The anti-HIV activity of unmodified naturally occurring anti-sense oligonucleotides having a homologous base sequence to the compound of the present invention, as well as corresponding nonsense oligonucleotides was determined with respect to the vicinity of the splicing acceptor site of pre-mRNA of HIV tat and rev.

Anti-human HIV activity was determined according to conventional methods (R. Pauwel et al., J Virological Methods 20, 309-321 (1988)).

More specifically, cell pellets obtained by centrifuging MT-4 cells in the logarithmic growth phase for 5 minutes at 150 x g were infected with HIV-1 at 37°C for 1 hour at a concentration of 10 $CCID_{50}$. HIV-infected MT-4 cells were then obtained by centrifuging the cells in 10% fetal bovine serum containing RPMI-1640 medium (to be referred to as "serum medium") for washing.

The HIV-infected MT-4 cells as well as mock infected MT-4 cells were suspended in serum medium to a concentration of $4 \times 10^5$ cells/ml, respectively. 100 µl each of previously stepwise-diluted solutions of the specimen compound (suspended in serum medium) were placed in each of the wells of a 96-well plastic microtiter plate. Next, 100 µl each of the above-mentioned cell suspensions was placed in each of the wells, followed by culturing while allowing the plate to stand at 37°C for 6 days in the presence of 5% carbon dioxide gas.

HIV-infected MT-4 cells to which the specimen compound had been added, as well as non-HIV-infected MT-4 cells to which the specimen compound had not been added were cultured in the same manner.

After completion of culturing, the cells were assayed based on the MTT (3-(4,5-dimethylthiazol-2-yl)-2,5-diphenyltetrazolium bromide) method (L.M. Green et al., J. Immunolo. Methods, 70, 257-268 (1984)) to determine the cytopathic activity of HIV. The concentration of specimen that inhibited 50% of the cytopathic activity of HIV-infected MT-4 cells (effective dose, $ED_{50}$) was determined by taking the cytopathic activity of HIV-infected MT-4 cells to which the specimen compound was added to be 100%, and taking the cell damaging activity of non-HIV-infected MT-4 cells to which the specimen compound was not added to be 0%.

In addition, the concentration that suppressed the proliferation of HIV-infected MT-4 cells to 50% ($CD_{50}$) was determined as an indication of the cytotoxic activity of the specimen compound, and the value of $CD_{50}/ED_{50}$ was taken to be the selectivity index (S. I.) of anti-HIV activity.

Those results are indicated in Table 1.

[Table 1] Test compounds and their HIV activity

| Example No. | Base sequence | $ED_{50}$ (µg/ml) | $CD_{50}$ (µg/ml) | Selectivity index |
|---|---|---|---|---|
| 1 | AGGTGGGTCTGAAAC | 6.5 | >100 | >15 |
| 2 | TCGGGGTTGGGAGGT | 7.0 | >100 | >14.3 |
| 3 | TGGGAGGTGGGTCTG | 4.0 | >100 | >25 |
| 4 | TTGGGAGGTGGGTCT | 2.2 | >100 | >45 |
| 5 | AGGTGGGTCTGAAAC | 7.2 | >100 | >13 |
| 8 | TGGGTCTGAAACGAT | 4.0 | >100 | >25 |
| 9 | TAGGTGGGTCTGAAAC | 12.5 | >100 | >8 |
| 10 | GGTGGGTCTGAAACG | 11.0 | >100 | >9.1 |
| 12 | GGAGGTGGGTCTGAA | 18.0 | >100 | >5.6 |
| 13 | GGGAGGTGGGTCTGA | 9.0 | >100 | >11.1 |
| 14 | GTTGGGAGGTGGGTC | 10.0 | >100 | >10 |
| 15 | GGGTTGGGAGGTGGG | 8.5 | >100 | >11.8 |
| 17 | TGGGAGGTGGGTCTG | 6.2 | >100 | >16.6 |
| 18 | TGGGAGGTGGGTCTG | 5.2 | >400 | >76.5 |
| 19 | TGGGAGGTGGGTCTG | 8.0 | >100 | >12.5 |
| 20 | TGGGAGGTGGGTCTG | 4.3 | 90 | >20.9 |

| | | | | |
|---|---|---|---|---|
| 22 | TGGGAGGTGGGTCTG | 7.4 | >100 | >13.5 |
| 24 | TGGGAGGTGGGTCTG | 2.5 | >100 | >40 |
| 25 | TGGGAGGTGGGTCTG | 7.2 | >100 | >13.8 |
| 26 | TGGGAGGTGGGTCTG | 12.5 | >100 | >8 |
| 27 | TGGGAGGTGGGTCTG | 34 | >100 | >2.9 |
| 28 | TGGGAGGTGGGTCT | 5.5 | >100 | >18.2 |
| 29 | TGGGAGGTGGGTC | 4.6 | >100 | >21.7 |
| 30 | TGGGAGGTGGGT | 4.5 | >100 | >22.2 |
| 31 | TGGGAGGTGGG | 4.6 | >100 | >21.7 |
| 32 | TGGGAGGTGG | 5.0 | >100 | >20 |
| 33 | TGGGAGGTG | 5.5 | >100 | >18.2 |

All of the compounds of the present invention were observed to demonstrate inhibitory effects on HIV proliferation at concentrations of 100 $\mu$g/ml or less. On the other hand, those corresponding compounds in which the 5' terminal was not modified were not observed to demonstrate anti-HIV activity over the range of concentrations investigated (<600 $\mu$g/ml). In addition, even when modified, those sequences not complementary to HIV gene (compounds of Comparative Examples 1 to 4) did not demonstrate inhibitory effects (<100 $\mu$g/ml). These results indicate that modifying the 5' terminal of the oligonucleotide molecule enhances the action of the anti-sense sequence.

(Test Example 2)

Action on tumor gene ras

It was also confirmed that in the method pertaining to the present invention, modified anti-sense oligonucleotides to oncogene ras is able to inhibit proliferation of DT cells at a concentration at which unmodified anti-sense oligonucleotides do not demonstrate efficacy.

After twice infecting NIH3T3 cells with Carsten's mouse sarcoma virus, DT cells consist of a non-virus-producing transformed cell strain that is cloned according to the agar colony method, incorporating two copies of activated ras gene (v-Ki-ras) in the form of chromosomes (Noda et al., Proc. Natl. Acad. Sci. USA, 80, 5602 (1988)). Consequently, DT cells differ from the parent NIH3T3 cells in that they have remarkably low adhesion, do not exhibit contact inhibition, and proliferate in multilayer, with said proliferation not limited to single layers.

The action of an anti-sense sequence (the compound of Example 6) corresponding to 21 bases from the 9th base upstream from the translation initiation site to the 12th base downstream from the initiation codon of this v-Ki-ras gene was investigated for the purpose of inhibiting expression of this gene. Those results are indicated in

Table 2

| Title: Oligonucleotide Structure (5'-3') | |
|---|---|
| DmTr-ODN-4 (Compound of Example 6) | DmTr-ATACTCAGTCATTTTTAGCAG |
| ODN-4 | -ATACTCAGTCATTTTTAGCAG |

The proliferation inhibitory action on DT cells of the above-mentioned specimen was measured in the manner described below. DT cells were suspended in DMEM medium containing 10% serum to a concentration of 5 x $10^3$ cells/ml. 500 µl each of this suspension was added to a 48-well plastic microtiter plate together with 20 µg of specimen. The plate was then cultured while allowing to stand in the presence of 5% $CO_2$. The medium was replaced with new one each three days, and the specimen was also added at that time. The number of cells was counted after 10 days, and proliferation inhibitory activity was determined by comparing with a group to which specimen was not added. As indicated in the results of Fig. 1, in contrast to strong cell proliferation inhibitory activity being recognized for only the compound of Example 6, proliferation inhibitory activity was not observed for homologous naturally occurring anti-sense oligonucleotides. In addition, changes were also observed in the morphology of cells to which the compound of Example 6 had been added. More specifically, there was an increased proportion of flat cells similar to the morphology of the parent NIH3T3 cells (Fig. 1).

(Test Example 3)

Action on tumor gene c-myb

It has been reported that expression of tumor gene c-myb is overexpressed in HL60 premyelocytic leukemia cells, and cell proliferation is interrupted by suppression of its expression with anti-sense oligonucleotide (Anfossi et al., Proc. Natl. Acad. Sci. USA, 86, 3379-3383 (1989)). The compound of Example 7 was also confirmed to demonstrate strong proliferation inhibitory effects by comparison with homologous unmodified anti-sense oligonucleotide.

The proliferation inhibitory effects on HL60 cells were measured in the manner described below for a compound in which a DmTr group was bonded to the 5' terminal of the 18mer sequence of 5'-GTGCCGGGGTCTTCGGGC-37 complementary to the mRNA of c-myb, for which inhibitory activity has been observed in the report of Anfossi et al. (DmTr-ODN-4: compound of Example 7), and corresponding unmodified oligonucleotide. After suspending the HL60 cells in serum medium to a concentration of 2 x $10^4$ cells/ml, and adding specimen to each to a concentration of 20 µg/ml, 500 µl aliquots of those cell suspensions were added to each of the wells of a 48-well plastic microtiter plate followed by culturing while allowing to stand for 5 days in the presence of 5% $CO_2$. The number of cells was counted on the 4th and 5th days of culturing. As a result, the compound of Example 7 demonstrated approximately twice the proliferation inhibitory activity of homologous unmodified compounds (Fig. 2).

(Test Example 4)

Suppression of Production of Viral Antigen in HIV-Infected Cells

It was confirmed by the following two types of experiments that treatment of HIV-1 infected cells by anti-sense oligonucleotide suppresses synthesis of viral portein.

(1) Inhibition of the Amount of Viral Reverse Transcriptase Released in Media

MT-4 cells were infected with HIV-1 in the same manner as in Test Example 1. Various concentrations of the compound of Example 3 were then added followed by culturing at 37°C for 6 days in a carbon dioxide gas incubator. Following culturing, the activity of reverse transcriptase contained in viral particles released into the culture supernatant was assayed using the method of Somogyi et al. (Somogyi et al., J. Virol. Methods, 27, 269 (1990)). Those results are indicated in Fig. 3. The activity of reverse transcriptase released by HIV-1 infected cells decreased dependently on the amount of the compound of Example 3 added to the medium. The concentration that resulted in 50% inhibition was 4.7 µg/ml, and simultaneously assayed virus-caused cell damage was nearly equal to the oligonucleotide concentration resulting in 50%

inhibition (7 μg/ml). Based on the above results, it became clear that the compound of Example 3 inhibits the production of viral reverse transcriptase in HIV-1 infected cells.

(2) Inhibition of Production of viral Antigen in HIV-1 Infected Cells

Next, the amount of viral antigen produced in HIV-1 infected cells treated with various concentrations of the compound of Example 3 was investigated in order to determine the action of said compound of Example 3 on the production of viral antigen other than reverse transcriptase. After adding the sample preparation solution described by Laemmli et al., containing SDS and the reducing agent mercaptoethanol (Laemmli, Nature, 227, 680 (1970)) to virus infected cells obtained according to the method indicated in Test Example 1 and control non-infected cells, and treating at 100°C for 5 minutes, SDS-PAGE electrophoresis was performed using 12.5% polyacrylamide gel. After the electrophoresis, the protein was transferred to a nitrocellulose filter, and the viral antigen was specifically label led using anti-HIV human serum prepared from HIV-1 infected patients and anti-human goat antibody labelled with peroxidase. The label led protein was then developed using the immunostaining kit manufactured by Konica. As indicated in lane 1 of Figs. 4A and 4B, various viral antigens including env (p160, p120) and gag (p55, p29, p24) are produced in virus infected cells (Petteway, et al., TiPS, 12, 28 (1991)). However, when the compound of Example 3 is added, production of viral antigens is inhibited dependent on its concentration. At a concentration of 12 μg/ml or more, essentially no viral antigen is observed. On the other hand, in the case of the compound corresponding to Example 3 in which the 5' terminal is not modified, despite having an identical base sequence, production of viral antigen is not inhibited at all even when added to a concentration of 100 μg/ml (Fig. 4B). Based on the above results, compounds in which the 5' terminal is modified were confirmed to strongly inhibit biosynthesis of all viral antigens in HIV-1 infected cells.

(Test Example 5)

Inhibition of Viral RNA Synthesis in HIV-1 Infected Cells

As previously described, the compound of Example 3, in which anti-HIV-1 activity was observed as shown in Table 1, is designed to inhibit the splicing of mRNA which is essential for production of TAT and REV proteins of the HIV-1 gene. Thus, it is predicted that if this compound inhibits biosynthesis of these viral proteins in virus infected cells, this would result in a shortage of TAT protein required for viral RNA synthesis, thus also resulting in inhibition of viral RNA synthesis. In order to confirm this prediction, synthesis of viral RNA in HIV-1 infected cells treated with the compound of Example 3 was analyzed by Northern blotting .

As MT-4 cells were infected with HIV-1 according to the method described in Example 1, the compound of Example 3 and a compound corresponding to Example 3 that was not modified were added to a final concentration of 20 μg/ml. The cells were then cultured at 37°C for 5 days in a carbon dioxide gas incubator. After removing the culture supernatant, the cells were washed with physiological saline solution and total cellular RNA within the cells was isolated by the guanidine thiocyanate method (Chirgwin et al., Biochemistry, 18, 5294 (1979)). 20 μg of that RNA were subjected to electrophresis on a formaldehyde/agarose gel, the resulting RNA was transferred to a nitrocellulose filter and hybridized at 42°C for 24 hours with synthetic oligonucleotide probes complementary to HIV-1 RNA label led with radioactive $^{32}P$ (5'-GAGGTGGGTCTGAAACGAT-3' and 5'-GAGGTGGGTCATTTGTACA-3'). After washing the filter twice for 15 minutes each at room temperature in 1 X SSC containing 0.1% SDS the filter was again washed twice for 15 minutes each in 0.2 X SSC containing 0.1% SDS. The signal specific for HIV-1 viral RNA was then detected by autoradiography.

As indicated in Fig. 5, in addition to a clear signal for genomic RNA of 9.2 kb coding for the gag and pol proteins, weaker signals were also detected for viral RNA of 4.3 kb coding for the splicing product of env, as well as 2.0 kb coding for the proteins tat and rev in MT-4 cells infected with HIV-1 (Fig. 5, lane 1). On the other hand, these signals were not detected in those cells not infected with HIV-1 (Fig. 5, lane 2).

No viral RNA was detected in HIV-1 infected cells treated with 20 μg/ml of the compound of Example 3 (Fig. 5, lane 4). On the other hand, there was no inhibition of viral RNA synthesis observed at all in cells to which the compound corresponding to Example 3, in which the 5' terminal was not modified, was added, despite having an identical base sequence (Fig. 5, comparison between lanes 1 and 3).

Above results suggest that compounds in which the 5' terminal is modified also strongly inhibit viral RNA synthesis in HIV-1 infected cells.

(Test Example 6)

Stabilization of Oligonucleotides by 5' Terminal Modification

All of the above test examples indicate that modification of the 5' terminal of oligonucleotides with a substituent is a prerequisite for expression of anti-HIV-1 activity. One of the reasons that can be considered for this expression of anti-HIV-1 activity is that said modification increases the stability of the oligonucleotide in the serum. In other words, although unmodified oligonucleotides are rapidly degradated due to the various kinds of nucleases contained in the serum, it is predicted that the oligonucleotides modified with substituents demonstrate higher stability. After labeling the 3' terminal of the compound of Example 3 as well as the unmodified compound corresponding to Example 3 with $^{32}P$ using the 3'-end labeling kit manufactured by Amersham, fixed amounts of those compounds were incubated at 37°C for 30 hours in either RPMI-1640 medium containing 10% fetal bovine serum which is not inactivated or the same medium without containing the serum, for the purpose of verifying this possibility. After the incubation, those oligonucleotides that were still present and had not been degradated were detected with 20% polyacrylamide gel electrophoresis. As indicated in Fig. 6, when serum was not added, both oligonucleotides were not degradated, regardless of whether modified with substituents at the 5' terminal. However, when serum was added, more than 90% of the unmodified compound corresponding to Example 3 was degradated, on the other hand the compound of Example 3 was still present and resistant to degradation(comparison of lanes 2 and 4 of Fig. 6). This result suggested that, in contrast to the unmodified compound corresponding to Example 3 being unstable and rapidly degradated in serum, oligonucleotides like the compound of Example 3, which has been modified with substituent such as DmTr, demonstrate improved stability making them less susceptible to degradation. Thus, it was strongly suggested that this is one of the factors of the improved in anti HIV-1 activity.

(Test Example 7)

Assay of Anti-HIV Activity against Fresh Clinically Isolated Strains

1) HIV-1 Separation Method (Method for Separating HIV-1 from HIV-1 Infected Peripheral Blood Monocytes (PBMC))

10 ml of blood from HIV-1 infected patients to which heparin was added was layered on a Leucoprep tube (manufactured by Becton Dikson). A white layer containing peripheral blood monocytes (PBMC) was collected after 30 minutes centrifugation at 2000 x g. To the resulting PBMC suspension was washed twice with added PBS(-) (manufactured by Nissui Seiyaku) and suspended in 0.5 ml of PBS. After counting the number of viable cells in the PBMC suspension, anti-human CD8 antibody-bound magnetic beads (Dynabeads; Dynal Inc., N.Y., USA) were added to the PBMC suspension in an amount 20 to 40 fold relative to the number of PBMC. The suspension was allowed to stand for 1 hour in an ice bath while repeating stirring 2 to 3 times. To the above-mentioned centrifuge tube was then added 5 ml of RPMI-1640 medium containing 10% inactivated fetal bovine serum, and the tube was then mounted on an MPC (Magnetic Particle Concentrator; Dynal Inc.). The Dynabeads (demonstrating a red color) was adhered to the inside of the centrifuge tube within 1 minute due to the application of magnetic force. The PBMC serum medium suspension was then collected from the centrifuge tube while being careful not to suck any of the Dynabeads.

PBMC separated from the blood of suitable healthy persons to which heparin was added was stimulated in advance for 3 days with PHA (10 µg/ml). Equal amounts of the juvenile PBMC obtained in this manner and the PBMC separated from HIV-1 infected patients, from which the above-mentioned CD8 positive cells were removed, were mixed into separation serum medium (serum medium containing the additives of 20 IU/ml of IL-2 10 µg/ml of PHA and 2 µg/ml of polybrene) to bring to a final concentration of $5 \times 10^5$ to $1 \times 10^6$ cells/ml. Mixed culturing was then performed at 37°C in a 5% carbon dioxide gas incubator. Mixed culturing was continued for approximately 14 days while replacing half the amount of juvenile PBMC with fresh juvenile PBMC of suitable healthy persons every 3 to 4 days. The amount of p24 antigen in the culture supernatant and viral titer (Focal immunoassay) were assayed when syncytium demonstrating well-defined Bloon-like forms were observed microscopically.

2) Drug Sensitivity Test on Clinically Isolated Strains by a Focus Formation Inhibition Assay (Slight Modified the Method of B. Chesbro et al.)

(Infection Procedure)

Recombinant HeLa cells (1022 cells), introduced into HeLa cell line followed by artificially expressing the CD-4 gene, were prepared to a density of 5 x $10^4$/ml in serum medium.

0.5 ml of aliquotes of the 1022 cell suspension was inoculated to each of the wells of a 48-well plate (manufactured by Coaster) with an Eppendorf pipette (so that cells are uniformly adhered to the culturing surface of each well). The 1022 cells were cultured overnight at 37°C in a 5% carbon dioxide gas incubator using a 48-well plate. On the following day, the serum medium in the 48-well plate for proliferation of 1022 cells was removed by suction with an aspirator. Next, DEAE dextran solution diluted with 250 µl of RPMI-1640 medium (8 µg/ml) was added and the plate was allowed to stand at 37°C in a 5% carbon dioxide gas incubator exactly for 20 minutes. Immediately after removing the DEAE dextran solution by suction with an aspirator, 100 µl of diluted virus solution (diluted with serum medium) was added having a titer allowing the formation of 50 to 80 foci. The plate was then allowed to stand at 37°C in a 5% carbon dioxide gas incubator for 90 minutes to infect the 1022 cells with virus. After completion of infection, 0.5 ml each of semi-logarithmically stepwise-diluted anti-HIV-containing serum medium was added to each of the wells followed by culturing at 37°C for at least 3 days in a 5% carbon dioxide gas incubator.

(Enzyme-Antibody Staining Procedure)

After completion of culturing, the serum medium in the 48-well plate for proliferation of 1022 cells was completely removed by suction with an aspirator followed by addition of 250 µl of methanol to fix the cells at room temperature for exactly 5 minutes. Immediately after 5 minutes elapsed, the methanol was completely removed by suction and the plate was washed twice with PBS(-) containing 1% FBS. Next, 100 µl of serum from an AIDS patient diluted by a factor of 800 with PBS(-) containing 1% FBS were added as the primary antibody, and allowed to react for about 30 minutes at room temperature. The thus added AIDS patient serum was then removed by suction and the plate was washed twice with PBS(-) containing 1% FBS. 100 µl of horseradish peroxidase-labelled anti-human $F(ab)^2$ diluted by a factor of 200 with PBS(-) were added, and allowed to react for at least about 45 minutes at room temperature. After washing the plate three times with PBS(-), 250 µl of enzyme substrate solution (0.2 mg/ml of 3-amino-9-ethyl-carbazole and 0.015% hydrogen peroxide/0.05 M sodium acetate buffer (pH 5.0)) was added to perform enzyme activity staining at room temperature for 20 minutes.

After completion of staining, excess substrate solution was washed from the plate with PBS(-), and the number of foci that stained red was counted microscopically (10 x 4).

(Results)

HIV-1, the pathogen of AIDS, is a hypervariable virus that demonstrates a high rate of mutation even within the same host. Thus, it is necessary to investigate the effects of the compound of the present invention against virus infected cells originating in AIDS patients actually infected with HIV at its various infection stages (AC, ARC and AIDS). A study was conducted regarding the sensitivity of HIV clinically isolated strains, isolated from HIV patients at various infection stages (AC, ARC and AIDS), to the compound of the present invention. More specifically, a focus formation inhibitory test (focus reduction assay) was performed to investigate the drug sensitivity of 12 virus strains with respect to the compound of Example 3. Sensitivity to the compound of Example 3 was confirmed for 10 standard laboratory strains and isolated strains. Moreover, this compound demonstrated uniform effects against clinically isolated strains from the various infection stages (AC, ARC and AIDS) of HIV patients.

Table 3 Various HIV-I infected clinically isolated strain
Sensitivity to the compound of Example 3

| HIV isolated strain | Clinical symptom | $IC_{50}$ (μg/ml) |
|---|---|---|
| HTLVIIIB* | - | 1.4 |
| HTLVIIIRF* | - | 2.3 |
| GOT9156 | AC | 2.8 |
| SAS91817 | AC | 7.2 |
| SAT91817 | ARC | 6.8 |
| FUJ91725 | ARC | 8.6 |
| FUJ91811 | ARC | 8.2 |
| YU6a | AIDS | 13.1 |
| ICH91513 | AIDS | 7.8 |
| SAI9166 | AIDS | 5.6 |
| IKE | AIDS | 3.3 |
| SUE | AIDS | 16.0 |

* Test standard strain

AIDS (Acquired immunodeficiency syndrome)

ARC (AIDS-related complex)

AC (Asymptomatic carrier)

(Test Example 8)

Acute toxicity was determined according to conventional methods. More specifically, 150 mg/kg of the compound of Example 3 was orally administered to five ddy mice (males). The mice were observed for 5 days, and it was found that all were viable after that time period.

(Preparation Example 1) Injectable Preparation

The injectable preparation is provided for use in the form of ampules containing a sterile solution or suspension with water or other pharmaceutically acceptable liquid, or a sterile powder preparation (preferably that resulting from freeze drying of the anti-sense compound) is filled into ampules and it may be simultaneously diluted with a pharmaceutically acceptable liquid.

(Preparation Example 2) Tablets

| | |
|---|---|
| 1) Compound of Example 3 | 200 |
| 2) Sodium salt of pyro-acid | 5 |
| 3) Aerosil 200 | 5 |
| 4) Magnesi stearate | 5 |
| 5) Lactose | 495 |
| 6) Corn starch | 154 |
| 7) Avicel | 123 |
| 8) HPL(L) | 10 |
| | 997 g |

A crushed mixture of above-mentioned ingredients 1) to 4) were added to pre-production granules consisting of a granulated mixture of above-mentioned ingredients 5) to 8). Next, the mixture was formed into tablets using a tablet making machine to obtain tablets containing 100 mg per tablet. Sugar coating can be applied to these tablets, as necessary.

(Preparation Example 3) Capsules

| | |
|---|---|
| 1) Compound of Example 3 | 200 |
| 2) Calcium hydrogenphosphate | 200 |
| 3) Aluminum silicate | 345 |
| 4) Crystal cellulose | 250 |
| 5) Magnesium stearate | 2 |
| | 997 g |

The ingredients 1) to 5) listed above were mixed and crushed. Further, after the mixture was passed through a sieve and blended well, the ingredients were formed into 200 mg capsules in accordance with conventional methods.

[INDUSTRIAL UTILIZABILITY]

The compound of the present invention possesses specific activity that damages virus infected cells and tumor cells, suppresses the characteristic morphological changes associated with the occurrence of viral diseases or tumorization, and further possesses effects of specifically inhibiting proliferation, etc.. Thus, the compound of the present invention is useful as a therapeutic and preventive drug for viral diseases and cancer.

[BRIEF DESCRIPTION OF THE DRAWINGS]

Fig. 1 indicates a microphotograph representing the action on DT cells.
Fig. 2 indicates the effects on proliferation of HL cells.
Fig. 3 indicates suppression of production of reverse transcriptase
Fig. 4 indicates suppression of production of viral antigen in HIV-1 infected cells.
Fig. 5 indicates inhibition of viral RNA synthesis in HIV-1 infected cells.
Fig. 6 indicates stabilization of oligonucleotides by modification of the 5' terminal.

[Sequence listing]

INFORMATION FOR SEQ ID NO.: 1

Sequence length: 15

Sequence type: Nucleic acid

Strandness: single

Topology: Linear

Hypothetical: No

Anti-sense: Yes

Sequence:

AGGTGGGTCT GAAAC

INFORMATION FOR SEQ ID NO.: 2

Sequence length: 15

Sequence type: Nucleic acid

Strandness: single

Topology: Linear

Hypothetical: No

Anti-sense: Yes

Sequence:

TCGGGGTTGG GAGGT

INFORMATION FOR SEQ ID NO.: 3

Sequence length: 15

Sequence type: Nucleic acid

Strandness: single

Topology: Linear

Hypothetical: No

Anti-sense: Yes

Sequence:

TTGGGAGGTG GGTCT

INFORMATION FOR SEQ ID NO.: 4

Sequence length: 21

Sequence type: Nucleic acid

Strandness: single

Topology: Linear

Sequence:

ATACTCAGTC ATTTTTAGCA G

INFORMATION FOR SEQ ID NO.: 5

Sequence length: 18

Sequence type: Nucleic acid

Strandness: single

Topology: Linear

Sequence:

GTGCCGGGGT CTTCGGGC

INFORMATION FOR SEQ ID NO.: 6

Sequence length: 15

Sequence type: Nucleic acid

Strandness: single

Topology: Linear

Sequence:

TGGGTCTGAA ACGAT

INFORMATION FOR SEQ ID NO.: 7

Sequence length: 16

Sequence type: Nucleic acid

Strandness: single

Topology: Linear

Sequence:

TAGGTGGGTC TGAAAC

INFORMATION FOR SEQ ID NO.: 8

Sequence length: 15

Sequence type: Nucleic acid

Strandness: single

Topology: Linear

Sequence:

GGTGGGTCTG AAACG

INFORMATION FOR SEQ ID NO.: 9

Sequence length: 15

Sequence type: Nucleic acid

Strandness: single

Topology: Linear

Sequence:

GGTGGGTTGC TTTGA

INFORMATION FOR SEQ ID NO.: 10

Sequence length: 15

Sequence type: Nucleic acid

Strandness: single

Topology: Linear

Sequence:

GGAGGTGGGT CTGAA

INFORMATION FOR SEQ ID NO.: 11

Sequence length: 15

Sequence type: Nucleic acid

Strandness: single

Topology: Linear

Sequence:

GGGAGGTGGG TCTGA

INFORMATION FOR SEQ ID NO.: 12

Sequence length: 15

Sequence type: Nucleic acid

Strandness: single

Topology: Linear

Sequence:

GTTGGGAGGT GGGTC

INFORMATION FOR SEQ ID NO.: 13

Sequence length: 15

Sequence type: Nucleic acid

Strandness: single

Topology: Linear

Sequence:

GGGTTGGGAG GTGGG

INFORMATION FOR SEQ ID NO.: 14

Sequence length: 15

Sequence type: Nucleic acid

Strandness: single

Topology: Linear

Sequence:

TGGGAGGTGG GTCTG

INFORMATION FOR SEQ ID NO.: 15

Sequence length: 14

Sequence type: Nucleic acid

Strandness: single

Topology: Linear

Sequence:

TGGGAGGTGG GTCT

INFORMATION FOR SEQ ID NO.: 16

Sequence length: 13

Sequence type: Nucleic acid

Strandness: single

Topology: Linear

Sequence:

TGGGAGGTGG GTC

INFORMATION FOR SEQ ID NO.: 17

Sequence length: 12

Sequence type: Nucleic acid

Strandness: single

Topology: Linear

Sequence:

TGGGAGGTGG GT

INFORMATION FOR SEQ ID NO.: 18

Sequence length: 15

Sequence type: Nucleic acid

Strandness: single

Topology: Linear

Sequence:

TGGGAGGTGG G

INFORMATION FOR SEQ ID NO.: 19

Sequence length: 10

Sequence type: Nucleic acid

Strandness: single

Topology: Linear

Sequence:

TGGGAGGTGG

INFORMATION FOR SEQ ID NO.: 20

Sequence length: 9

Sequence type: Nucleic acid

Strandness: single

Topology: Linear

Sequence:

TGGGAGGTG

## Claims

1. A compound represented by the general formula (1):

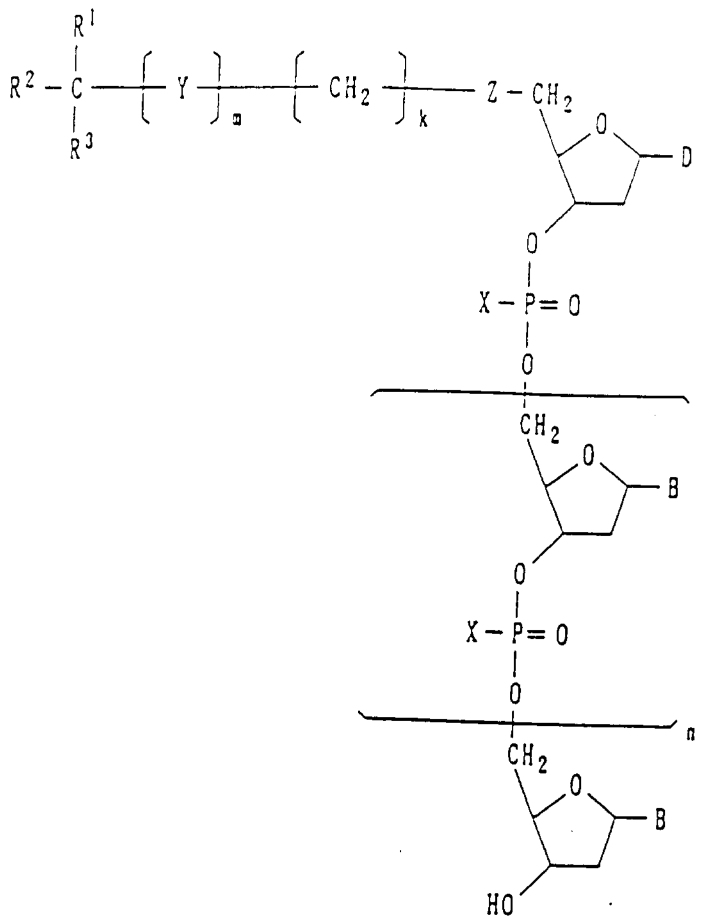

(1)

(wherein, k represents 0 or an integer of 1 to 20; m represents 0 or 1; n represents an integer of 4 to 29; X represents a hydroxyl group, a methyl group, a mercapto group, alkoxy group having 1 to 4 carbon atoms or a monoalkylamino group having 1 to 6 carbon atoms; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; and D and B each represent a residue independently selected from the following group $\beta$ in respective nucleotide units, provided that m = 0 when k = 0, and the base sequence containing B in the formula is the base sequence complementary to a tumor gene or a virus gene), or its salt.

[Group α]

Hydroxy group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group β]

Adenine (A), guanine (G), cytosine (C) and thymine (T).

2. A compound represented by the general formula (1):

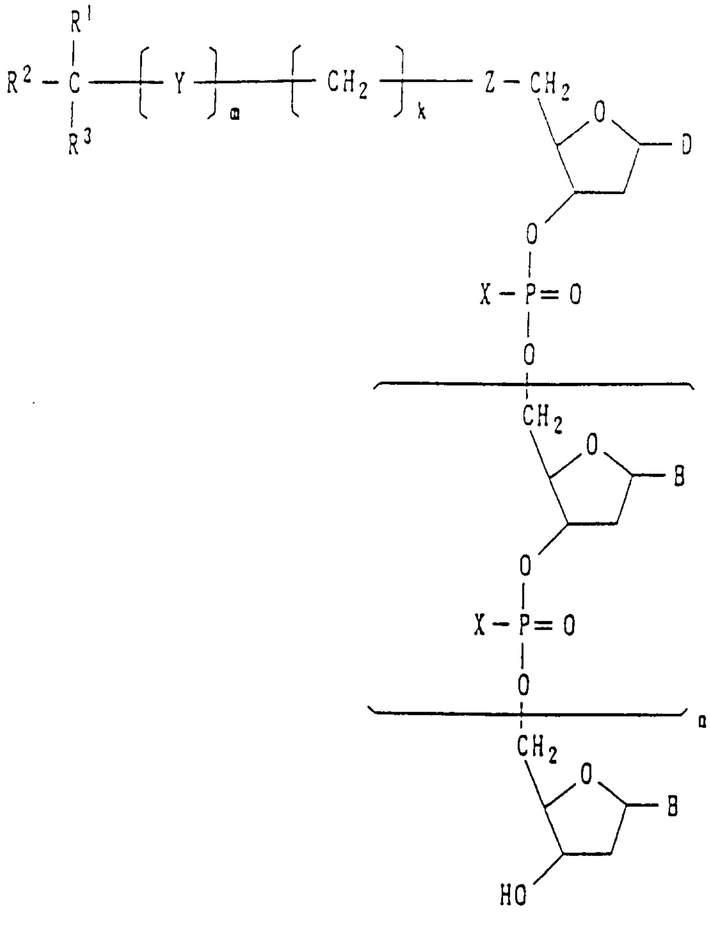

(1)

(wherein, k represents 0 or an integer of 1 to 20; m represents 0 or 1; n represents an integer of 4 to 29; X represents a hydroxyl group; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α; and D and B each represent a residue independently selected from the following group β in respective nucleotide units, provided that m = 0 when k = 0, and the base sequence containing B in the formula is the base sequence complementary to a tumor gene or a virus gene), or its salt.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group β]

Adenine (A), guanine (G), cytosine (C) and thymine (T).

3. A compound represented by the general formula (1):

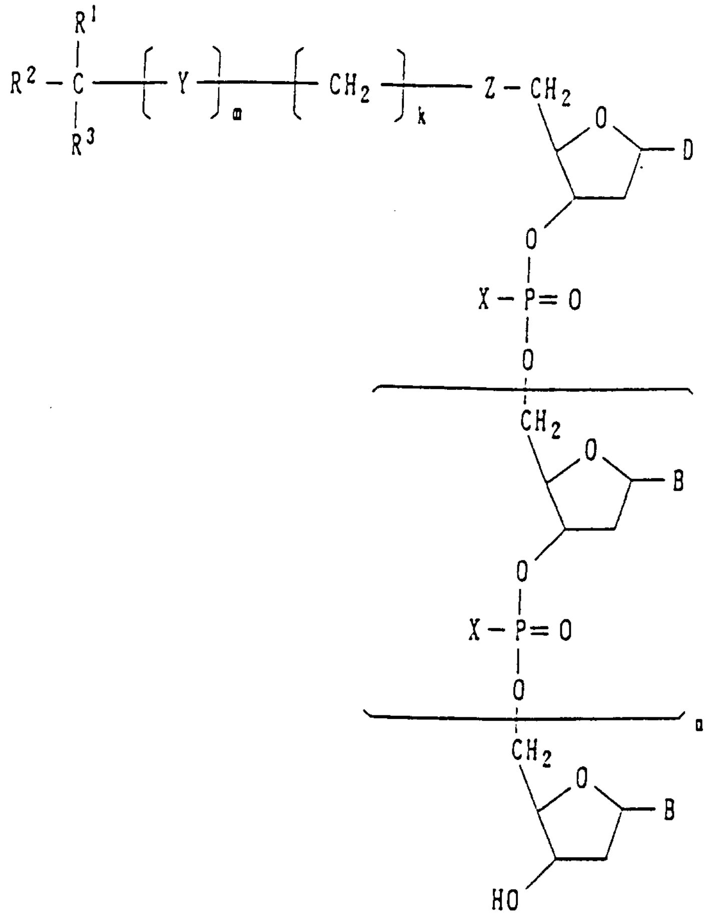

(1)

(wherein, k represents 0 or an integer of 1 to 20; m represents 0 or 1; n represents an integer of 4 to 29; X represents a methyl group; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; and D and B each represent a residue independently selected from the following group $\beta$ in respective nucleotide units, provided that m = 0 when k = 0, and the base sequence containing B in the formula is the base sequence complementary to a tumor gene or a virus gene), or its salt.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group $\beta$]

Adenine (A), guanine (G), cytosine (C) and thymine (T).

**4.** A compound represented by the general formula (1):

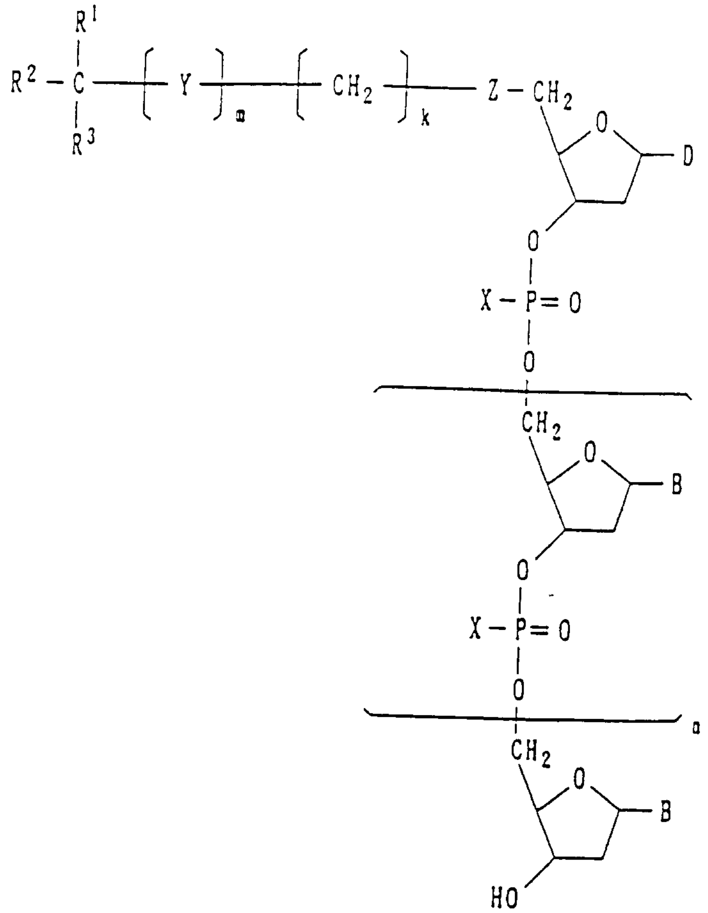

(1)

(wherein, k represents 0 or an integer of 1 to 20; m represents 0 or 1; n represents an integer of 4 to 29; X represents a mercapto group; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; and D and B each represent a residue independently selected from the following group $\beta$ in respective nucleotide units, provided that m = 0 when k = 0, and the base sequence containing B in the formula is the base sequence complementary to a tumor gene or a virus gene), or its salt.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group $\beta$]

Adenine (A), guanine (G), cytosine (C) and thymine (T).

**5.** A compound represented by the general formula (1):

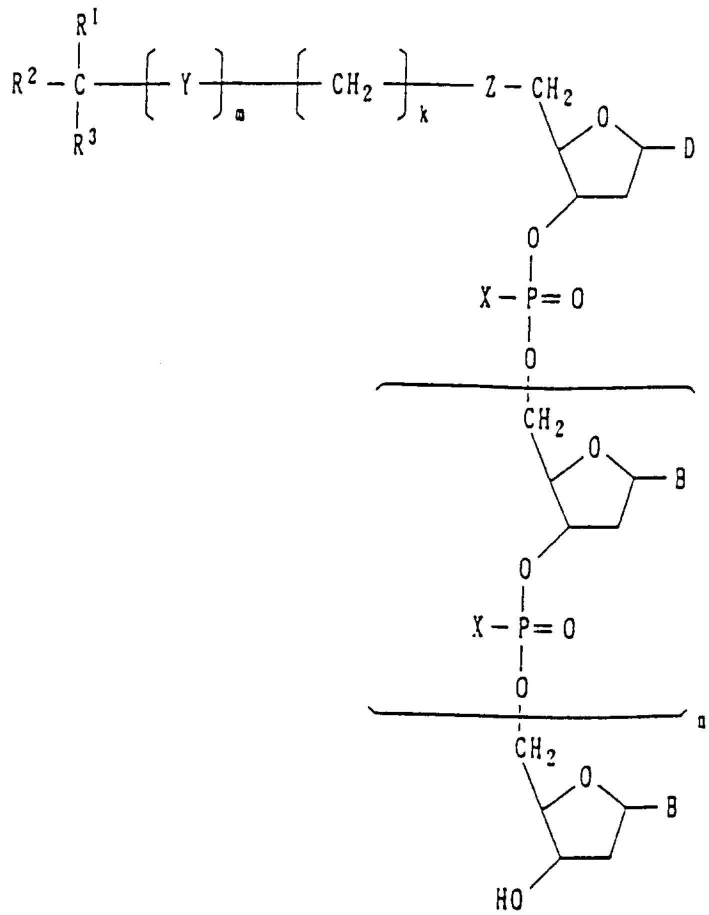

(1)

(wherein, k represents 0 or an integer of 1 to 20; m represents 0 or 1; n represents an integer of 4 to 29; X represents an alkoxy group having 1 to 4 carbon atoms; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; and D and B each represent a residue independently selected from the following group $\alpha$ in respective nucleotide units, provided that m = 0 when k = 0, and the base sequence containing B in the formula is the base sequence complementary to a tumor gene or a virus gene), or its salt.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group $\beta$]

Adenine (A), guanine (G), cytosine (C) and thymine (T).

**6.** A compound represented by the general formula (1):

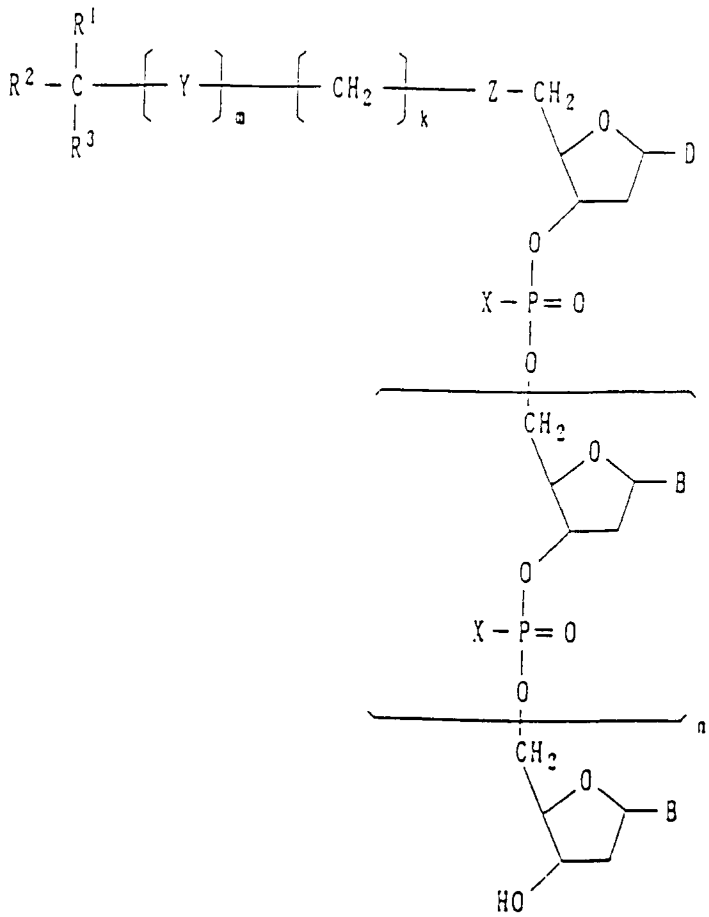

(1)

(wherein, k represents 0 or an integer of 1 to 20; m represents 0 or 1; n represents an integer of 4 to 29; x represents a monoalkylamino group having 1 to 6 carbon atoms; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; and D and B each represent a residue independently selected from the following group $\beta$in respective nucleotide units, provided that m = 0 when k = 0, and the base sequence containing B in the formula is the base sequence complementary to a tumor gene or a virus gene), or its salt.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group $\beta$]

Adenine (A), guanine (G), cytosine (C) and thymine (T).

**7.** The compound according to Claim 1 wherein:

k is 0 to 20,

m is 0 or 1,

n is 4 to 29,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence complementary to a tumor gene or virus gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy groups with an aryl moiety having 6 to 10 carbon atoms and an alkyl moiety having 1 or 2 carbon atoms.

**8.** The compound according to Claim 1 wherein:
k is 0 to 15,
m is 0 or 1,
n is 8 to 29,
X is a hydroxyl group, a methyl group, a mercapto group, an alkoxy group having 1 to 4 carbon atoms or a monoalkyl group having 1 to 6 carbon atoms,
Y is an oxygen or sulfur atom,
Z is an oxygen or sulfur atom,
$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following, group α and,
the base sequence containing B is a base sequence complementary to a tumor gene or virus gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**9.** The compound according to Claim 1 wherein:
k is 0 to 15,
m is 0 or 1,
n is 8 to 29,
X is a hydroxyl group,
Y is an oxygen or sulfur atom,
Z is an oxygen or sulfur atom,
$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence complementary to a tumor gene or virus gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**10.** The compound according to Claim 1 wherein:
k is 0 to 12,
m is 0,
n is 13 to 18,
x is a hydroxyl group, a methyl group, a mercapto group, an alkoxy group having 1 to 4 carbon atoms or a monoalkylamino group having 1 to 6 carbon atoms,
Y is an oxygen or sulfur atom,
Z is an oxygen or sulfur atom,
$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence complementary to a tumor gene or virus gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10

carbon atoms and an alkyl moiety having 1 or 2 carbon atoms.

**11.** The compound according to Claim 1 wherein:
k is 0 to 12,
m is 0 or 1,
n is 13 to 18,
X is a hydroxyl group,
Y is an oxygen or sulfur atom,
Z is an oxygen or sulfur atom,
$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence complementary to a tumor gene or virus gene.
[Group $\alpha$]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**12.** The compound according to Claim 1 wherein:
k is 0 to 12,
m is 0 or 1,
n is 13 to 18,
X is a hydroxyl group,
Y is an oxygen atom,
Z is an oxygen atom,
$R^1$, $R^2$ and $R^3$ may be the same or different from one another and each represent a hydrogen atom, an alkyl group having 1 to 4 carbon atoms, or an aryl group having 6 to 10 carbon atoms that may have a substituent selected from the following group $\alpha'$ and,
the base sequence containing B is a base sequence complementary to a tumor gene or virus gene.
[Group $\alpha'$]
Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.

**13.** The compound according to Claim 1 wherein:
k is 0 to 12,
m is 0 or 1,
n is 13 to 18,
X is a hydroxyl group,
Y is an oxygen atom,
Z is an oxygen atom,
$R^1$, $R^2$ and $R^3$ may be the same or different from one another and each represent a hydrogen atom, an alkyl group having 1 to 4 carbon atoms, or a phenyl group that may have a substituent selected from the following group $\alpha''$ and,
the base sequence containing B is a base sequence complementary to a tumor gene or virus gene.
[Group $\alpha''$]
Methyl group, ethyl group, propyl group, t-butyl group, methoxy group, ethoxy group, propoxy group, t-butoxy group and phenylmethyloxy group.

**14.** The compound according to Claim 1 wherein:
k is 0 to 20,
m is 0 or 1,
n is 4 to 29,
X is a hydroxyl group, a methyl group, a mercapto group, an alkoxy group having 1 to 4 carbon, atoms, or a monoalkylamino group having 1 to 6 carbon atoms,
Y is an oxygen or sulfur atom,
Z is an oxygen or sulfur atom,
$R^1$, $R^2$ and $R^3$ may be the same or different from one another and each represent a hydrogen

atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a nucleotide of base number 7947 to Base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy groups with an aryl moiety having 6 to 10 carbon atoms and an alkyl moiety having 1 or 2 carbon atoms.

**15.** The compound according to Claim 1 wherein:

k is 0 to 20,

m is 0 or 1,

n is 4 to 29,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl groups having 6 to 10 carbon atoms that may have a substituent selected from the following group α and,

the base sequence containing B is a base sequence of 9 to 30 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**16.** The compound according to Claim 1 wherein:

k is 0 to 15,

m is 0 or 1,

n is 8 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**17.** The compound according to Claim 1 wherein:

k is 0 to 15,

m is 0 or 1,

n is 8 to 29,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a

part of a nucleotide of base number 7947 to base number 7975 in HIV gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**18.** The compound according to Claim 1 wherein:
k is 0 to 12,
m is 0,
n is 13 to 18,
X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 9 to 30 bases complementary to all or a part of a nucleotide of base number 7947 to base number 7975 in HIV gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**19.** The compound according to Claim 1 wherein:
k is 0 to 12,
m is 0 or 1,
n is 13 to 18,
X is a hydroxyl group,
Y is an oxygen or sulfur atom,
Z is an oxygen or sulfur atom,
$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 9 to 30 bases complementary to all or a part of a nucleotide of base number 7947 to base number 7975 in HIV gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy groups with an aryl moiety having 6 to 10 carbon atoms and an alkyl moiety having 1 or 2 carbon atoms.

**20.** The compound according to Claim 1 wherein:
k is 0 to 12,
m is 0 or 1,
n is 13 to 18,
X is a hydroxyl group,
Y is an oxygen atom,
Z is an oxygen atom,
$R^1$, $R^2$ and $R^3$ may be the same or different from one another and each represent a hydrogen atom, an alkyl group having 1 to 4 carbon atoms, or an aryl group having 6 to 10 carbon atoms that may have a substituent selected from the following group α' and,
the base sequence containing B is a base sequence of 9 to 30 bases complementary to all or a part of a nucleotide of base number 7947 to base number 7975 in HIV gene.
[Group α']
Hydroxyl group, alkyl group having 1 to 4 carbon atoms, alkoxy group having 1 to 4 carbon atoms,

phenyl, naphthyl, phenyloxy, phenylmethyloxy and naphthylmethyloxy groups.

16) A compound wherein:

k is 0 to 12,

m is 0 or 1,

n is 13 to 18,

X is a hydroxyl group,

Y is an oxygen atom,

Z is an oxygen atom,

$R^1$, $R^2$ and $R^3$ may be the same or different from one another and each represent a hydrogen atom, an alkyl group having 1 to 4 carbon atoms, or a phenyl group that may have a substituent selected from the following group α" and,

the base sequence containing B is a base sequence of 9 to 30 bases complementary to all or a part of a nucleotide of base number 7947 to base number 7975 in HIV gene.

[Group α"]

Methyl group, ethyl group, propyl group, t-butyl group, methoxy group, ethoxy group, propoxy group, t-butoxy group and phenylmethyloxy group.

**21.** The compound according to Claim 1 wherein:

k is 0 to 20,

m is 0 or 1,

n is 4 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**22.** The compound according to Claim 1 wherein:

k is 0 to 20,

m is 0 or 1,

n is 4 to 29,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**23.** The compound according to Claim 1 wherein:

k is 0 to 15,

m is 0 or 1,

n is 8 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**24.** The compound according to Claim 1 wherein:

k is 0 to 15,

m is 0 or 1,

n is 8 to 29,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**25.** The compound according to Claim 1 wherein:

k is 0 to 12,

m is 0,

n is 13 to 18,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**26.** The compound according to Claim 1 wherein:

k is 0 to 12,

m is 0 or 1,

n is 13 to 18,

X is a hydroxyl group,

Y is an oxygen or sulfur atom,

Z is an oxygen or sulfur atom,

$R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 14 to 19 bases complementary to all or a part of a nucleotide of base number 7947 to base number 7975 in HIV gene.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**27.** The compound according to Claim 1 wherein:

k is 0 to 12,

m is 0 or 1,

n is 13 to 18,

X is a hydroxyl group,

Y is an oxygen atom,

Z is an oxygen atom,

$R^1$, $R^2$ and $R^3$ may be the same or different from one another and each represent a hydrogen atom, an alkyl group having 1 to 4 carbon atoms, or aryl group having 6 to 10 carbon atoms that may have a substituent selected from the following group α' and,

the base sequence containing B is a base sequence of 14 to 19 bases complementary to all or a part of a nucleotide of base number 7947 to base number 7975 in HIV gene.

[Group α']

Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.

**28.** The compound according to Claim 1 wherein:

k is 0 to 12,

m is 0 or 1,

n is 13 to 18,

X is a hydroxyl group,

Y is an oxygen atom,

Z is an oxygen atom,

$R^1$, $R^2$ and $R^3$ may be the same or different from one another and each represent a hydrogen atom, an alkyl group having 1 to 4 carbon atoms, or a phenyl group that may have a substituent selected from the following group α'' and,

the base sequence containing B is a base sequence of 14 to 19 bases complementary to all or a part of a nucleotide of base number 7947 to base number 7975 in HIV gene.

[Group α'']

Methyl group, ethyl group, propyl group, t-butyl group, methoxy group, ethoxy group, propoxy group, t-butoxy group and phenylmethyloxy group.

**29.** The compound according to Claim 1 wherein:

k is 0 to 20,

m is 0 or 1,

n is 4 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfuyr atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,

the base sequence containing B is a base sequence of 9 to 30 bases complementary to all or a part of a region of 30 nucleotides which is around a criterional nucleotide positioning less than 5

nucleotides from the translation initiation site in a tumor gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**30.** The compound according to Claim 1 wherein:
k is 0 to 20,
m is 0 or 1,
n is 4 to 29,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 9 to 30 bases complementary to all or a part of a region of 30 nucleotides which is around a criterional nucleotide positioning less than 5 nucleotides from the translation initiation site in a tumor gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**31.** The compound according to Claim 1 wherein:
k is 0 to 15,
m is 0 or 1,
n is 8 to 29,
X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 9 to 30 bases complementary to all or a part of a region of 30 nucleotides which is around a criterional nucleotide positioning less than 5 nucleotides from the translation initiation site in a tumor gene.
[Group α]
Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**32.** The compound according to Claim 1 wherein:
k is 0 to 15,
m is 0 or 1,
n is 8 to 29,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 9 to 30 bases complementary to all or a part of a region of 30 nucleotides which is around a criterional nucleotide positioning less than 5

nucleotides from the translation initiation site in a tumor gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**33.** The compound according to Claim 1 wherein:
k is 0 to 12,
m is 0,
n is 13 to 18,
X is a hydroxyl group, methyl group, mercapto group, alkoxy group having 1 to 4 carbon atoms, or monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 9 to 30 bases complementary to all or a part of a region of 30 nucleotides which is around a criterional nucleotide positioning less than 5 nucleotides from the translation initiation site in a tumor gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**34.** The compound according to Claim 1 wherein:
k is 0 to 12,
m is 0 or 1,
n is 13 to 18,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 9 to 30 bases complementary to all or a part of a region of 30 nucleotides which is a criterional nucleotide positioning less than 5 nucleotides from the translation initiation site in a tumor gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**35.** The compound according to Claim 1 wherein:
k is 0 to 12,
m is 0 or 1,
n is 13 to 18,
X is a hydroxyl group,
Y is an oxygen atom,
Z is an oxygen atom,
$R^1$, $R^2$, and $R^3$ may be the same or different from one another and each represent a hydrogen atom, an alkyl group having 1 to 4 carbon atoms, or an aryl group having 6 to 10 carbon atoms that may have a substituent selected from the following group α' and,
the base sequence containing B is a base sequence of 9 to 30 bases complementary to all or a part of a region of 5 nucleotides which is a criterional nucleotide positioning less than 30 nucleotides

from the translation initiation site in a tumor gene.
[Group α']
Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.

**36.** The compound according to Claim 1 wherein:
k is 0 to 12,
m is 0 or 1,
n is 13 to 18,
X is a hydroxyl group,
Y is an oxygen atom,
Z is an oxygen atom,
$R^1$, $R^2$, and $R^3$ may be the same or different from one another and each represent a hydrogen atom, an alkyl group having 1 to 4 carbon atoms, or a phenyl group that may have a substituent selected from the following group α'' and,
the base sequence containing B is a base sequence of 9 to 30 bases complementary to all or a part of a region of 30 nucleotides which is a criterional nucleotide positioning less than 5 nucleotides from the translation initiation site in a tumor gene.
[Group α'']
Methyl group, ethyl group, propyl group, t-butyl group, methoxy group, ethoxy group, propoxy group, t-butoxy group, and phenylmethyloxy group.

**37.** The compound according to Claim 1 wherein:
k is 0 to 20,
m is 0 or 1,
n is 4 to 29,
X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 14 to 19 bases complementary to a part of a region of 30 nucleotides which is a criterional nucleotide positioning less than 5 nucleotides from the translation initiation site in a tumor gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**38.** The compound according to Claim 1 wherein:
k is 0 to 20,
m is 0 or 1,
n is 4 to 29,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 14 to 19 bases complementary to a part of a region of 30 nucleotides which is a criterional nucleotide positioning less than 5 nucleotides from the translation initiation site in a tumor gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms,

aryloxy group having 6 to 10 carbon atoms and aralkyloxy group with an aryl moiety having 6 to 10 carbon atoms and an alkyl moiety having 1 or 2 carbon atoms.

**39.** The compound according to Claim 1 wherein:
k is 0 to 15,
m is 0 or 1,
n is 8 to 29,
X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 14 to 19 bases complementary to a part of a region of 30 nucleotides which is a criterional nucleotide positioning less than 5 nucleotides from the translation initiation site in a tumor gene.
[Group $\alpha$]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**40.** The compound according to Claim 1 wherein:
k is 0 to 15,
m is 0 or 1,
n is 8 to 29,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 14 to 19 bases complementary to a part of a region of 30 nucleotides which is a criterional nucleotide positioning less than 5 nucleotides from the translation initiation site in a tumor gene.
[Group $\alpha$]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**41.** The compound according to Claim 1 wherein:
k is 0 to 12,
m is 0,
n is 13 to 18,
X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 14 to 19 bases complementary to a part of a region of 30 nucleotides which is a criterional nucleotide positioning less than 5 nucleotides from the translation initiation site in a tumor gene.
[Group $\alpha$]
Hydroxyl group, alkyl group having 1 to 6 carbon atoms, alkoxy group having 1 to 6 carbon atoms,

methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**42.** The compound according to Claim 1 wherein:
k is 0 to 12,
m is 0 or 1,
n is 13 to 18,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 14 to 19 bases complementary to a part of a region of 30 nucleotides which is a criterional nucleotide positioning less than 5 nucleotides from the translation initiation site in a tumor gene.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**43.** The compound according to Claim 1 wherein:
k is 0 to 12,
m is 0 or 1,
n is 13 to 18,
X is a hydroxyl group,
Y is an oxygen atom,
Z is an oxygen atom,
$R^1$, $R^2$, and $R^3$ may be the same or different from one another and each represent a hydrogen atom, an alkyl group having 1 to 4 carbon atoms, or an aryl group having 6 to 10 carbon atoms that may have a substituent selected from the following group α' and,
the base sequence containing B is a base sequence of 14 to 19 bases complementary to a part of a region of 30 nucleotides which is a criterional nucleotide positioning less than 5 nucleotides from the translation initiation site in a tumor gene.
[Group α']
Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.
40) A compound wherein:
k is 0 to 12,
m is 0 or 1,
n is 13 to 18,
X is a hydroxyl group,
Y is an oxygen atom,
Z is an oxygen atom,
$R^1$, $R^2$, and $R^3$ may be the same or different from one another and each represent a hydrogen atom, an alkyl group having 1 to 4 carbon atoms, or a phenyl group that may have a substituent selected from the following group α'' and,
the base sequence containing B is a base sequence of 14 to 19 bases complementary to a part of a region of 30 nucleotides which is a criterional nucleotide positioning less than 5 nucleotides from the translation initiation site in a tumor gene.
[Group α'']
Methyl group, ethyl group, propyl group, t-butyl group, methoxy group, ethoxy group, propoxy group, t-butoxy group and phenylmethyloxy group.

**44.** The compound according to Claim 1 wherein:
k is 0 to 20,

m is 0 or 1,

n is 4 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence of 9 to 30 bases complementary to all or a part of a region of 30 nucleotides which is a criterional nucleotide Positioning less than 5 nucleotides from the single base site in a tumor gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**45.** The compound according to Claim 1 wherein:

k is 0 to 20,

m is 0 or 1,

n is 4 to 29,

X is a hydroxyl group,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence of 9 to 30 bases complementary to all or a part of a region of 30 nucleotides which is a criterional nucleotide positioning less than 5 nucleotides from the single base site in a tumor gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**46.** The compound according to Claim 1 wherein:

k is 0 to 15,

m is 0 or 1,

n is 8 to 29,

X is a hydroxyl group, a methyl group, a mercapto group, an alkoxy group having 1 to 4 carbon atoms, or a monoalkylamino group having 1 to 6 carbon atoms,

Y is an oxygen or sulfur atom,

Z is an oxygen or sulfur atom,

$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,

the base sequence containing B is a base sequence of 9 to 30 bases complementary to all or a part of a region of 30 nucleotides which is a criterional nucleotide positioning less than 5 nucleotides from the single base site in a tumor gene.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**47.** The compound according to Claim 1 wherein:
k is 0 to 15,
m is 0 or 1,
n is 8 to 29,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 9 to 30 bases complementary to all or a part of a region of 30 nucleotides which is a criterional nucleotide positioning less than 5 nucleotides from the single base site in a tumor gene.
[Group $\alpha$]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**48.** The compound according to Claim 1 wherein:
k is 0 to 12,
m is 0,
n is 13 to 18,
X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 9 to 30 bases complementary to all or a part of a region of 30 nucleotides which is a criterional nucleotide positioning less than 5 nucleotides from the single base site in a tumor gene.
[Group $\alpha$]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**49.** The compound according to Claim 1 wherein:
k is 0 to 12,
m is 0 or 1,
n is 13 to 18,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 9 to 30 bases complementary to all or a part of a region of 30 nucleotides which is a criterional nucleotide positioning less than 5 nucleotides from the single base site in a tumor gene.
[Group $\alpha$]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**50.** The compound according to Claim 1 wherein:
k is 0 to 12,
m is 0 or 1,
n is 13 to 18,
X is a hydroxyl group,
Y is an oxygen atom,
Z is an oxygen atom,
$R^1$, $R^2$, and $R^3$ may be the same or different from one another and each represent a hydrogen atom, an alkyl group having 1 to 4 carbon atoms, or an aryl group having 6 to 10 carbon atoms that may have a substituent selected from the following group $\alpha'$ and,
the base sequence containing B is a base sequence of 9 to 30 bases complementary to all or a part of a region of 30 nucleotides which is a criterional nucleotide positioning less than 5 nucleotides from the single base site in a tumor gene.
[Group $\alpha'$]
Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.

**51.** The compound according to Claim 1 wherein:
k is 0 to 12,
m is 0 or 1,
n is 13 to 18,
X is a hydroxyl group,
Y is an oxygen atom,
Z is an oxygen atom,
$R^1$, $R^2$, and $R^3$ may be the same or different from one another and each represent a hydrogen atom, an alkyl group having 1 to 4 carbon atoms, or a phenyl group that may have a substituent selected from the following group $\alpha''$ and,
the base sequence containing B is a base sequence of 9 to 30 bases complementary to all or a part of a region of 30 nucleotides which is a criterional nucleotide positioning less than 5 nucleotides from the single base site in a tumor gene.
[Group $\alpha''$]
Methyl group, ethyl group, propyl group, t-butyl group, methoxy group, ethoxy group, propoxy group, t-butoxy group and phenylmethyloxy group.

**52.** The compound according to Claim 1 wherein:
k is 0 to 20,
m is 0 or 1,
n is 4 to 29,
X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 14 to 19 bases complementary to a part of a region of 30 nucleotides which is a criterional nucleotide positioning less than 5 nucleotides from the single base site in ras and c-myb tumor genes.
[Group $\alpha$]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**53.** The compound according to Claim 1 wherein:
k is 0 to 20,
m is 0 or 1,
n is 4 to 29,

X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 14 to 19 bases complementary to a part of a region of 30 nucleotides which is a criterional nucleotide positioning less than 5 nucleotides from the single base site in ras and c-myb tumor genes,
[Group $\alpha$]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**54.** The compound according to Claim 1 wherein:
k is 0 to 15,
m is 0 or 1,
n is 8 to 29,
X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 14 to 19 bases complementary to a part of, a region of 30 nucleotides which is a criterional nucleotide positioning less than 5 nucleotides from the single base site in ras and c-myb tumor genes.
[Group $\alpha$]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**55.** The compound according to Claim 1 wherein:
k is 0 to 15,
m is 0 or 1,
n is 8 to 29,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group $\alpha$ and,
the base sequence containing B is a base sequence of 14 to 19 bases complementary to a part of a region of 30 nucleotides which is a criterional nucleotide positioning less than 5 nucleotides from the single base site in ras and c-myb tumor genes.
[Group $\alpha$]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**56.** The compound according to Claim 1 wherein:
k is 0 to 12,
m is 0,
n is 13 to 18,

X is a hydroxyl group, a methyl group, a mercapto group, an alkoxy group having 1 to 4 carbon atoms, or a monoalkylamino group having 1 to 6 carbon atoms,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 14 to 19 bases complementary to a part of a region of 30 nucleotides which is a criterional nucleotide positioning less than 5 nucleotides from the single base site in ras and c-myb tumor genes.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**57.** The compound according to Claim 1 wherein:
k is 0 to 12,
m is 0 or 1,
n is 13 to 18,
X is a hydroxyl group,
Y is an oxygen or sulfur atom,
Z is an oxygen or sulfur atom,
$R^1$, $R^2$, and $R^3$ may be the same or different from one another and each represent a hydrogen atom, an alkyl group having 1 to 6 carbon atoms, or an aryl group having 6 to 10 carbon atoms that may have a substituent selected from the following group α and,
the base sequence containing B is a base sequence of 14 to 19 bases complementary to a part of a region of 30 nucleotides which is a criterional nucleotide positioning less than 5 nucleotides from the single base site in ras and c-myb tumor genes.
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

**58.** The compound according to Claim 1 wherein:
k is 0 to 12,
m is 0 or 1,
n is 13 to 18,
X is a hydroxyl group,
Y is an oxygen atom,
Z is an oxygen atom,
$R^1$, $R^2$, and $R^3$ may be the same or different from one another and each represent a hydrogen atom, an alkyl group having 1 to 4 carbon atoms, or an aryl group having 6 to 10 carbon atoms that may have a substituent selected from the following group α' and,
the base sequence containing B is a baste sequence of 14 to 19 bases complementary to a part of a region of 30 nucleotides which is a criterional nucleotide positioning less than 5 nucleotides from the single base site in ras and c-myb tumor genes.
[Group α']
Hydroxyl group, alkyl group having 1 to 4 carbon atoms, alkoxy group having 1 to 4 carbon atoms, phenyl, naphthyl, phenyloxy, phenylmethyloxy and naphthylmethyloxy groups.

**59.** The compound according to Claim 1 wherein:
k is 0 to 12,
m is 0 or 1,
n is 13 to 18,
X is a hydroxyl group,
Y is an oxygen atom,

Z is an oxygen atom,

$R^1$, $R^2$, and $R^3$ may be the same or different from one another and each represent a hydrogen atom, an alkyl group having 1 to 4 carbon atoms, or a phenyl group that may have a substituent selected from the following group α'' and,

the base sequence containing B is a base sequence of 14 to 19 bases complementary to a part of a region of 30 nucleotides which is a criterional nucleotide positioning less than 5 nucleotides from the single base site in ras and c-myb tumor genes.

[Group α'']

Methyl group, ethyl group, propyl group, t-butyl group, methoxy group, ethoxy group, propoxy group, t-butoxy group and phenylmethyloxy group.

**60.** The compound according to Claim wherein:

k is 0,

m is 0,

X is a hydroxyl group,

Z is an oxygen atom,

$R^1$, $R^2$ and $R^3$ may be the same or different from one another and each represent a phenyl group that may have a substituent selected from the following group α''' and,

the base sequence is AGGTGGGTCTGAAAC.

[Group α''']

Methoxy group, ethoxy group, propoxy group and t-butoxy group.

**61.** The compound according to Claim wherein:

k is 0,

m is 0,

X is a hydroxyl group,

Z is an oxygen atom,

$R^1$, $R^2$ and $R^3$ may be the same or different from one another and each represent a phenyl group that may have a substituent selected from the following group α''' and,

the base sequence is TCGGGGTTGGGAGGT.

[Group α''']

Methoxy group, ethoxy group, propoxy group and t-butoxy group.

**62.** The compound according to Claim wherein:

k is 0,

m is 0,

X is a hydroxyl group,

Z is an oxygen atom,

$R^1$, $R^2$ and $R^3$ may be the same or different from one another and each represent a phenyl group that may have a substituent selected from the following group α''' and,

the base sequence is TTGGGAGGTGGGTCT.

[Group α''']

Methoxy group, ethoxy group, propoxy group and t-butoxy group.

**63.** The compound according to Claim wherein:

k is 0,

m is 0,

X is a hydroxyl group,

Z is an oxygen atom,

$R^1$, $R^2$ and $R^3$ may be the same or different from one another and each represent a phenyl group that may have a substituent selected from the following group α''' and,

the base sequence is ATACTCAGTCATTTTTAGCAG.

[Group α''']

Methoxy group, ethoxy group, propoxy group and t-butoxy group.

**64.** The compound according to Claim wherein:

k is 0,

m is 0,

X is a hydroxyl group,
Z is an oxygen atom,
$R^1$, $R^2$ and $R^3$ may be the same or different from one another and each represent a phenyl group that may have a substituent selected from the following group α''' and,
the base sequence is GTGCCGGGGTCTTCGGGC.
[Group α''']
Methoxy group.

**65.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGTCTGAAACGAT.
[Group α''']
Methoxy group, ethoxy group, propoxy group and t-butoxy group.

**66.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TAGGTGGGTCTGAAAC.
[Group α''']
Methoxy group.

**67.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is GGTGGGTCTGAAACG.
[Group α''']
Methoxy group, ethoxy group, propoxy group and t-butoxy group.

**68.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is GGTGGGTTGCTTTGA.
[Group α''']
Methoxy group, ethoxy group, propoxy group and t-butoxy group.

**69.** The compound according to Claim wherein:
k is 0,
m is 0,
X is a hydroxyl group,
Z is an oxygen atom,
$R^1$, $R^2$ and $R^3$ may be the same or different from one another and each represent a phenyl group

that may have a substituent selected from the following group α''' and,
the base sequence is GGAGGTGGGTCTGAA.
[Group α''']
Methoxy group, ethoxy group, propoxy group and t-butoxy group.

**70.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is GGGAGGTGGGTCTGA.
[Group α''']
Methoxy group, ethoxy group, propoxy group and t-butoxy group.

**71.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent group selected from the following group α''' and,
the base sequence is GTTGGGAGGTGGGTC.
[Group α''']
Methoxy group.

**72.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is GGGTTGGGAGGTGGG.
[Group α''']
Methoxy group, ethoxy group, propoxy group and t-butoxy group.

**73.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGGGTCTG.
[Group α''']
Methoxy group, ethoxy group, propoxy group and t-butoxy group.

**74.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGGGTCT.
[Group α''']

Methoxy-group, ethoxy group, propoxy group and t-butoxy group.

**75.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGGGTC.
[Group α''']
Methoxy group, ethoxy group, propoxy group and t-butoxy group.

**76.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGGGT.
(Group α''']
Methoxy group, ethoxy group, propoxy group and t-butoxy group.

**77.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGGG.
[Group α''']
Methoxy group, ethoxy group, propoxy group and t-butoxy group.

**78.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTGG.
[Group α''']
Methoxy group, ethoxy group, propoxy group and t-butoxy group.

**79.** The compound according to Claim wherein:
k is 0,
m is 0,
X is a hydroxyl group,
Z is an oxygen atom,
$R^1$, $R^2$ and $R^3$ may be the same or different from one another and each represent a phenyl group that may have a substituent selected from the following group α''' and,
the base sequence is TGGGAGGTG.
[Group α''']
Methoxy group, ethoxy group, propoxy group and t-butoxy group.

**80.** An antiviral agent comprising a compound represented by the general formula (1):

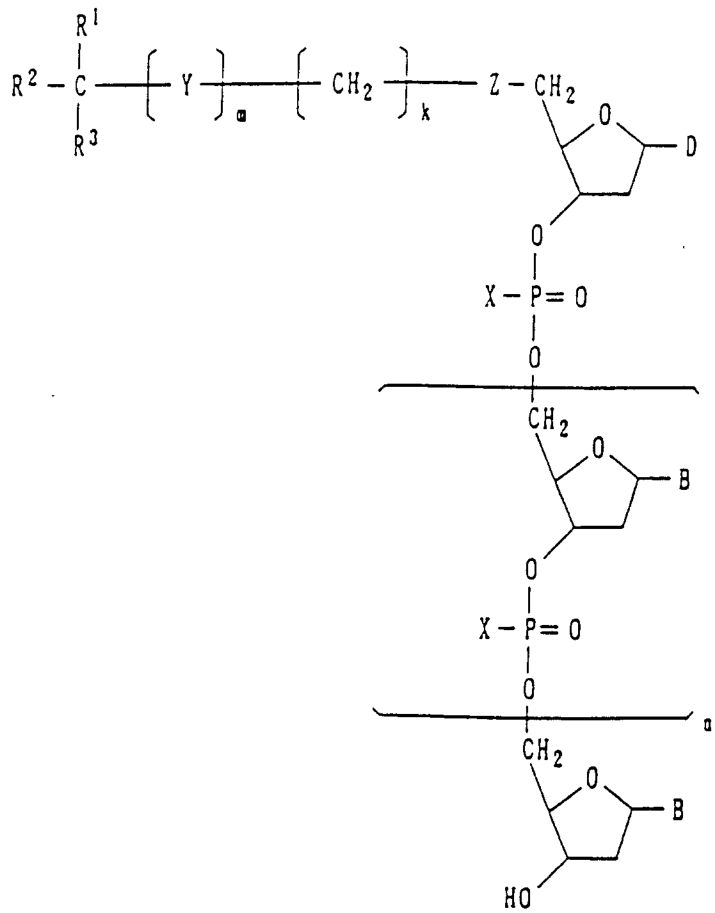

(1)

(wherein, k represents 0 or an integer of 1 to 20; m represents 0 or 1; n represents an integer of 4 to 29; X represents a hydroxyl group, a methyl group, a mercapto group, an alkoxy group having 1 to 4 carbon atoms, or a monoalkylamino group having 1 to 6 carbon atoms; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; and D and B each represent a residue independently selected from the following group $\alpha$ in respective nucleotide units, provided that m = 0 when k = 0, and the base sequence containing B in the formula is the base sequence complementary to a virus gene), or a compound selected from its salts with a pharmaceutically acceptable carrier or excipient.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group $\beta$]

Adenine (A), guanine (G), cytosine (C) and thymine (T).

**81.** An anti-AIDS agent comprising a compound represented by the general formula (1):

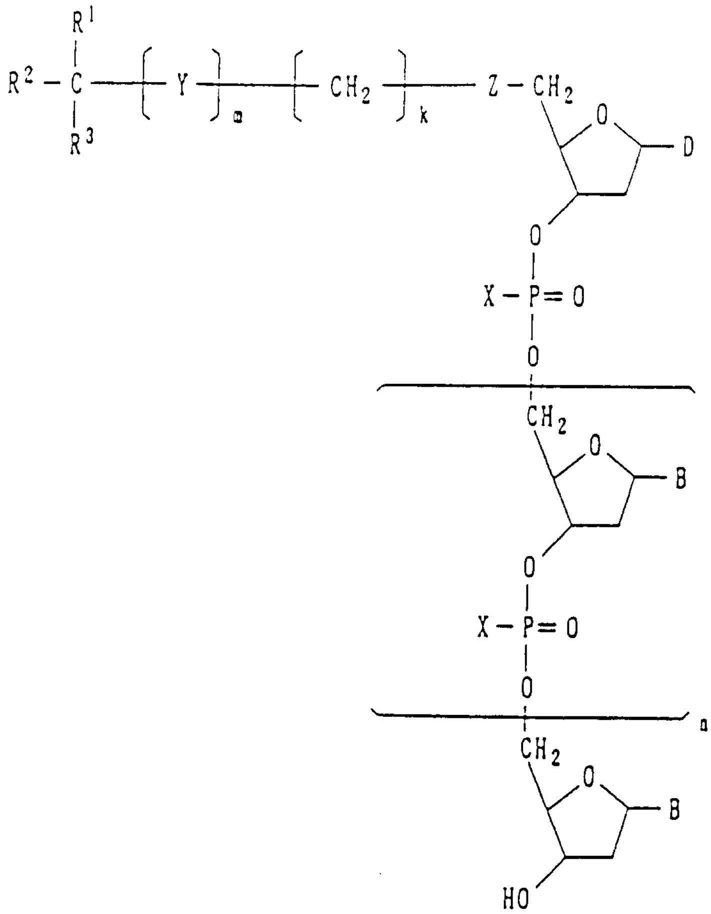

(1)

(wherein, k represents 0 or an integer of 1 to 20; m represents 0 or 1; n represents an integer of 4 to 29; X represents a hydroxyl group, a methyl group, a mercapto group, an alkoxy group having 1 to 4 carbon atoms, or a monoalkylamino group having 1 to 6 carbon atoms: Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; and D and B each represent a residue independently selected from the following group $\beta$ in respective nucleotide units, provided that m = 0 when k = 0, and the base sequence containing B in the formula is the base sequence complementary to a virus gene), or a compound selected from its salts with a pharmaceutically acceptable carrier or excipient.

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group $\beta$]

Adenine (A), guanine (G), cytosine (C) and thymine (T).

**82.** An antitumor agent comprising a compound represented by the general formula (1):

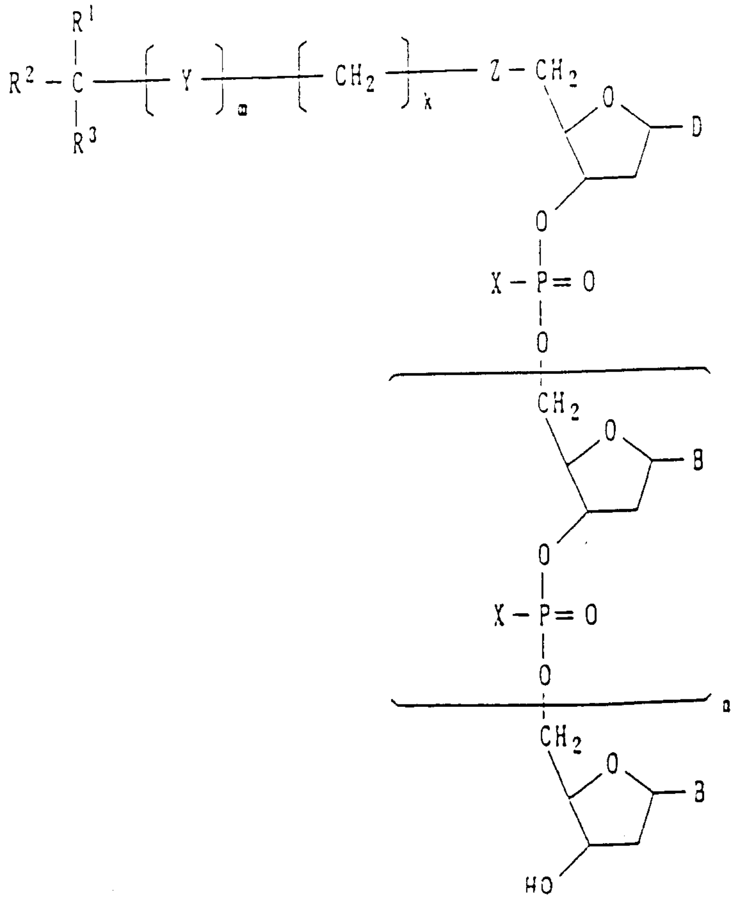

(1)

(wherein, k represents 0 or an integer of 1 to 20; m represents 0 or 1; n represents an integer of 4 to 29; X represents a hydroxyl group, a methyl group, a mercapto group, an alkoxy group having 1 to 4 carbon atoms, or a monoalkylamino group having 1 to 6 carbon atoms; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; and D and B each represent a residue independently selected from the following group $\beta$ in respective nucleotide units, provided that m = 0 when k = 0, and the base sequence containing B in the formula is the base sequence complementary to a tumor gene), or a compound selected from its salts with a pharmaceutically acceptable carrier or excipient.

[Group $\alpha$]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group $\beta$]

Adenine (A), guanine (G), cytosine (C) and thymine (T).

**83.** An anti-AIDS agent comprising an effective dose of a compound selected from the following compounds or a pharmacologically acceptable salt thereof with a pharmaceutically acceptalbe carrier or excipient.

5'-O-(4,4'-dimethoxytrityl)-TGGGAGGTGGGTCTG,
5'-O-(4-methoxytrityl)-TGGGAGGTGGGTCTG,
5'-O-trityl-TGGGAGGTGGGTCTG,
5'-O-diphenylmethyl-TGGGAGGTGGGTCTG,
5'-O-phenylmethyl-TGGGAGGTGGGTCTG,

5'-O-(2-naphthylmethyl)-TGGGAGGTGGGTCTG,
5'-O-[(3,5-dibenzyloxy)benzyl]-TGGGAGGTGGGTCTG,
5'-S-diphenylmethyl-TGGGAGGTGGGTCTG,
5'-O-(4,4'-dimethoxytrityl)-AGGTGGGTCTGAAAC,
5'-O-(4,4'-dimethoxytrityl)-TCGGGGTTGGGAGGT,
5'-O-(4,4'-dimethoxytrityl)-TTGGGAGGTGGGTCT,
5'-O-(4,4'-dimethoxytrityl)-TGGGTCTGAAACGAT,
5'-O-(4,4'-dimethoxytrityl)-GGGAGGTGGGTCTGA,
5'-O-(4,4'-dimethoxytrityl)-GTTGGGAGGTGGGTC,
5'-O-(4,4'-dimethoxytrityl)-GGGTTGGGAGTGGGG,
5'-O-trityl-TGGGAGGTGGGTCTG,
5'-O-(4,4'-dimethoxytrityl)-TGGGAGGTGGGTCT,
5'-O-(4,4'-dimethoxytrityl)-TGGGAGGTGGGTC,
5'-O-(4,4'-dimethoxytrityl)-TGGGAGGTGGGT,
5'-O-(4,4'-dimethoxytrityl)-TGGGAGGTGGG,
5'-O-(4,4'-dimethoxytrityl)-TGGGAGGTGG, and
5'-O-(4,4'-dimethoxytrityl)-TGGGAGGTG.

**84.** An antitumor agent comprising an effective dose of a compound selected from the following compounds or a pharmacologically acceptable salt thereof with a pharmaceutically acceptalbe carrier or excipient.
5'-O-(4,4'-dimethoxytrityl)-ATACTCAGTCATTTTTAGCAG,

**85.** A treatment or preventive method wherein a compound represented by the general formula (1):

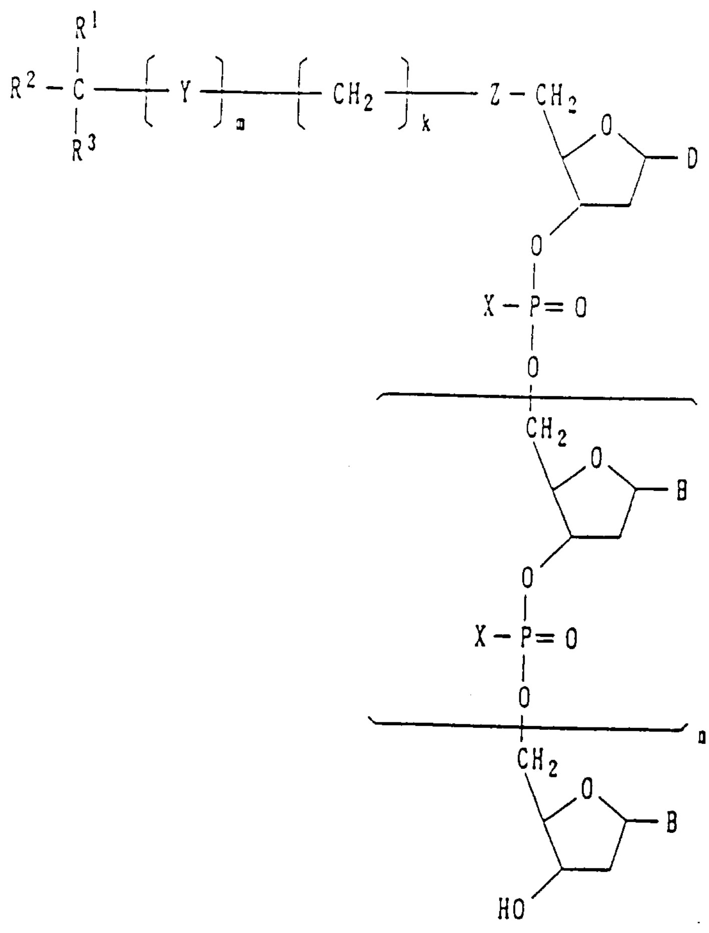

(1)

(wherein, k represents 0 or an integer of 1 to 20; m represents 0 or 1; n represents an integer of 4

to 29; X represents a hydroxyl group, a methyl group, a mercapto group, an alkoxy group having 1 to 4 carbon atoms, or a monoalkylamino group having 1 to 6 carbon atoms; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α; and D and B each represent a residue independently selected from the following group β in respective nucleotide units, provided that m = 0 when k = 0, and the base sequence containing B in the formula is the base sequence complementary to a virus gene), or its salt is administered to mammals suffering from a viral disease.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group β]

Adenine (A), guanine (G), cytosine (C) and thymine (T).

**86.** A treatment or preventive method wherein a compound represented by the general formula (1):

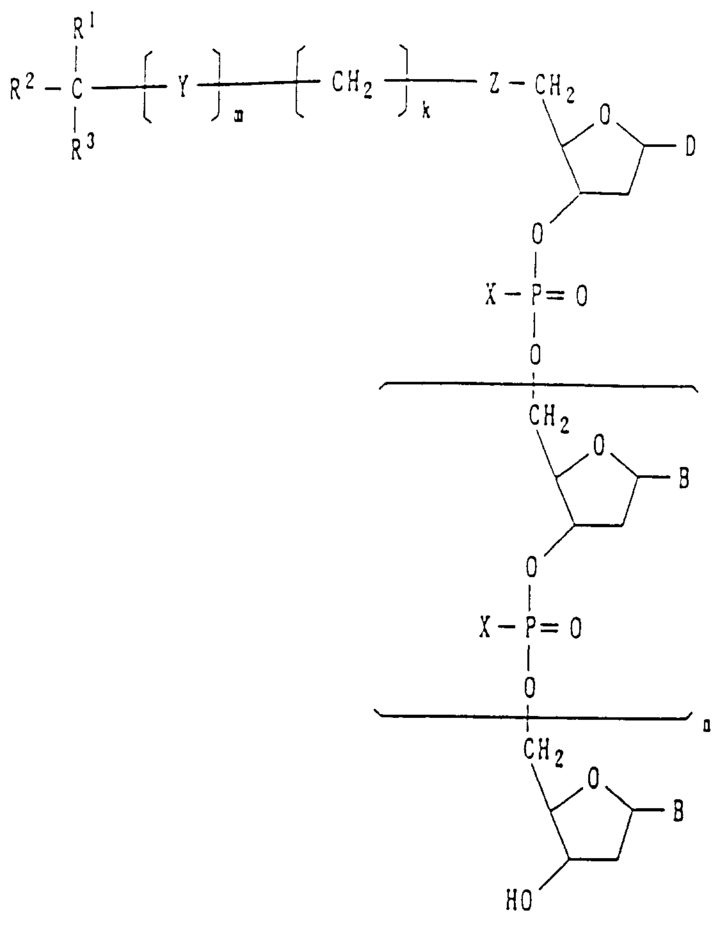

(1)

(wherein, k represents 0 or an integer of 1 to 20; m represents 0 or 1; n represents an integer of 4 to 29; X represents a hydroxyl group, a methyl group, a mercapto group, an alkoxy group having 1 to 4 carbon atoms, or a monoalkylamino group having 1 to 6 carbon atoms; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α; and D and B each represent a residue independently selected from the following group β in respective nucleotide units,

provided that m = 0 when k = 0, and the base sequence containing B in the formula is the base sequence complementary to a virus gene), or its salt is administered to mammals suffering from an anti-AIDS viral disease.

[Group α]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group β]

Adenine (A), guanine (G), cytosine (C) and thymine (T).

**87.** A treatment or preventive method wherein a compound represented by the general formula (1):

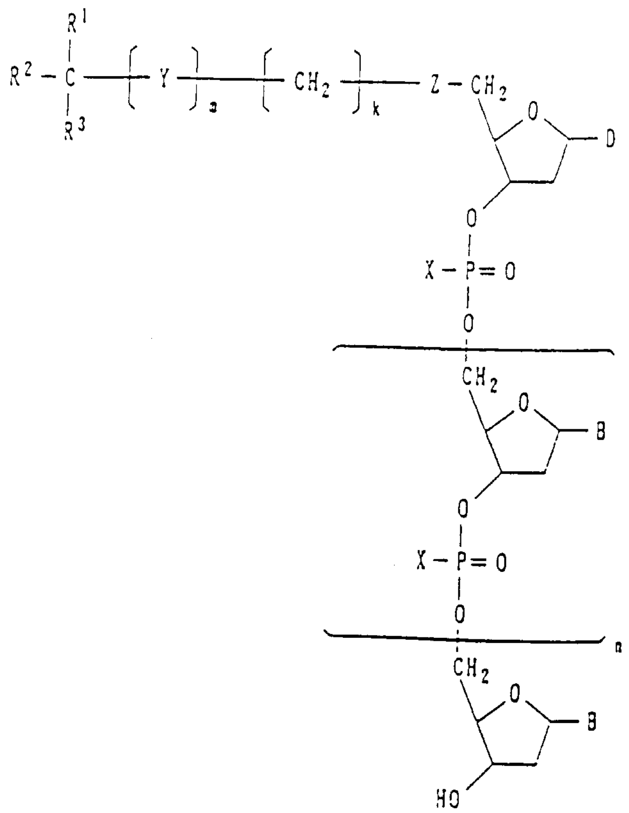

(1)

(wherein, k represents 0 or an integer of 1 to 20; m represents 0 or 1; n represents an integer of 4 to 29; X represents a hydroxyl group, a methyl group, a mercapto group, an alkoxy group having 1 to 4 carbon atoms, or a monoalkylamino group having 1 to 6 carbon atoms; Y and Z each represent an oxygen or sulfur atom; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α; and D and B each represent a residue independently selected from the following group β in respective nucleotide units, provided that m = 0 when k = 0, and the base sequence containing B in the formula is the base sequence complementary to a tumor gene), or its salt is administered to mammals suffering from a cancer disease.

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group β]

Adenine (A), guanine (G), cytosine (C) and thymine (T).

**88.** A treatment or preventive method wherein an effective dose of a compound selected from the compounds indicated below, or its pharmacologically acceptable salt, is administered to mammals suffering from an AIDS viral disease.

5'-O-(4,4'-dimethoxytrityl)-TGGGAGGTGGGTCTG,
5'-O-(4-methoxytrityl)-TGGGAGGTGGGTCTG,
5'-O-trityl-TGGGAGGTGGGTCTG,
5'-O-diphenylmethyl-TGGGAGGTGGGTCTG,
5'-O-phenylmethyl-TGGGAGGTGGGTCTG,
5'-O-(2-naphthylmethyl)-TGGGAGGTGGGTCTG,
5'-O-[(3,5-dibenzyloxy)benzyl]-TGGGAGGTGGGTCTG,
5'-S-diphenylmethyl-TGGGAGGTGGGTCTG,
5'-O-(4,4'-dimethoxytrityl)-AGGTGGGTCTGAAAC,
5'-O-(4,4'-dimethoxytrityl)-TCGGGGTTGGGAGGT,
5'-O-(4,4'-dimethoxytrityl)-TTGGGAGGTGGGTCT,
5'-O-(4,4'-dimethoxytrityl)-TGGGTCTGAAACGAT,
5'-O-(4,4'-dimethoxytrityl)-GGGAGGTGGGTCTGA,
5'-O-(4,4'-dimethoxytrityl)-GTTGGGAGGTGGGTC,
5'-O-(4,4'-dimethoxytrityl)-GGGTTGGGAGTGGGG,
5'-O-trityl-TGGGAGGTGGGTCTG,
5'-O-(4,4'-dimethoxytrityl)-TGGGAGGTGGGTCT,
5'-O-(4,4'-dimethoxytrityl)-TGGGAGGTGGGTC,
5'-O-(4,4'-dimethoxytrityl)-TGGGAGGTGGGT,
5'-O-(4,4'-dimethoxytrityl)-TGGGAGGTGGG,
5'-O-(4,4'-dimethoxytrityl)-TGGGAGGTGG, and
5'-O-(4,4'-dimethoxytrityl)-TGGGAGGTG.

**89.** A treatment or preventive method wherein an effective dose of a compound selected from the compounds indicated below, or its pharmacologically acceptable salt, is administered to mammals suffering from a cancer disease.

5'-O-(4,4'-dimethoxytrityl)-ATACTCAGTCATTTTTAGCAG,

**90.** A process for preparing the compound described in Claim 2, wherein said compound is obtained by condensing using (iii) (1) a condensing agent, a compound represented by the general formula:

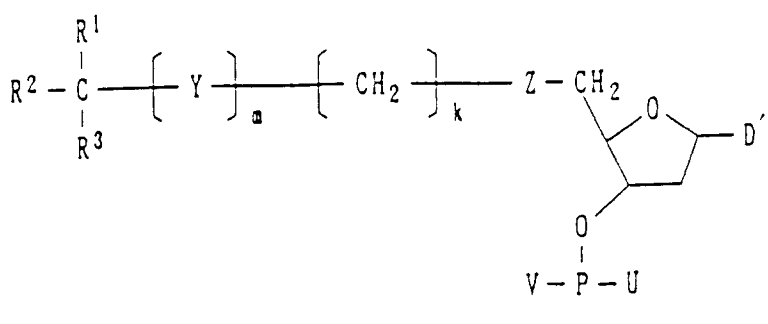

(wherein, k represents 0 or an integer of 1 to 20; m represents 0 or 1, $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; Y and Z each represent an oxygen atom or sulfur atom; V represents a dialkylamino group; U represents a heterocyclic group having a dialkylamino group or 1 or 2 oxygen atoms and/or nitrogen atoms within its ring; and D' represents a residue selected from the following group $\beta$ or homologous protected residue; provided that m = 0 when k = 0,

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group β]

Adenine (A), guanine (G), cytosine (C) and thymine (T)); with,
an oligonucleotide represented by the general formula:

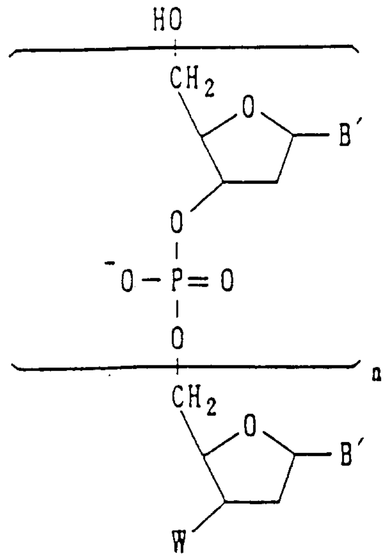

(wherein B's each represent a residue independently selected from the following group β in the respective nucleotide units or protected homologous residue; W represents a lower acyloxy group, an arylacyloxy group or a controlled pore glass, and n is an integer of 4 to 29, provided that the base sequence containing B' is a base sequence complementary to a tumor gene or virus gene,

[Group β]

Adenine (A), guanine (G), cytosine (C) and thymine (T)),

(ii) [1] which oligonucleotide is synthesized with a DNA synthesizer and is bonded to said controlled pore glass whose dimethoxytrityl group at the 5' terminal only is eliminated, with the base portion thereof being protected with a protecting group such as an acyl group, or [2] which is synthesized by a liquid phase method, and has a free hydroxyl group at the 5' terminal, with the base portion thereof being protected with a protecting group such as an acyl group,

to form triphosphite bonds, (2) oxidizing the resulting product to triphosphate using an oxidizing agent, and when said oligonucleotide is bonded to the controlled pore glass, removing the protecting group after severing from the controlled pore glass.

**91.** A process for preparing the compound described in Claim 2 wherein said compound is obtained by condensing using a condensing agent, a compound represented by the general formula:

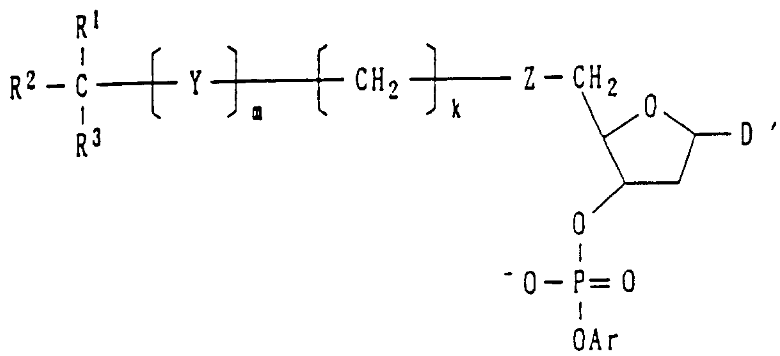

(wherein, k represents 0 or an integer of 1 to 20; m represents 0 or 1; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the

following group α; Ar represents an aryl group; and D' represents a residue selected from the following group β or homologous protected residue; provided that m = 0 when k = 0,
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.
[Group β]
Adenine (A), guanine (g), cytosine (C) and thymine (T)); with an oligonucleotide represented by the general formula:

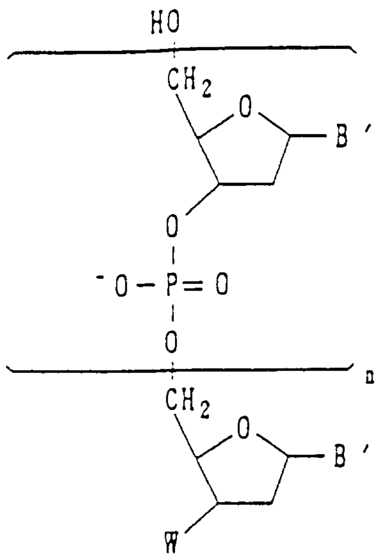

(wherein, B's each represents a residue independently selected from the following group β in respective nucleotide units or protected homologous residue; W represents a lower acyloxy group, an arylacyloxy group or a controlled pore glass; and n is an integer of 4 to 29, provided that the base sequence containing B' is a base sequence complementary to a tumor gene or virus gene,
[Group β]
Adenine (A), guanine (G), cytosine (C) and thymine (T)),
[1] which oligonucleotide is Synthesized with a DNA synthesized and is bonded to said controlled pore glass whose dimethoxytrityl group at the 5' terminal only is eliminated with the base portion, with the phosphate portion thereof being protected with a protecting group,
or [2] which is synthesized by a liquid phase method, and has a free hydroxyl group at the 5' terminal, with the base portion and phosphate portion thereof being protected with a protecting group,
to form triphosphate bonds, and eliminating the protecting group after severing from the controlled pore glass when the oligonucleotide is bonded to the controlled pore glass.

**92.** A process for preparing the compound described in Claim 2 wherein said compound is obtained by condensing a compound represented by the general formula:

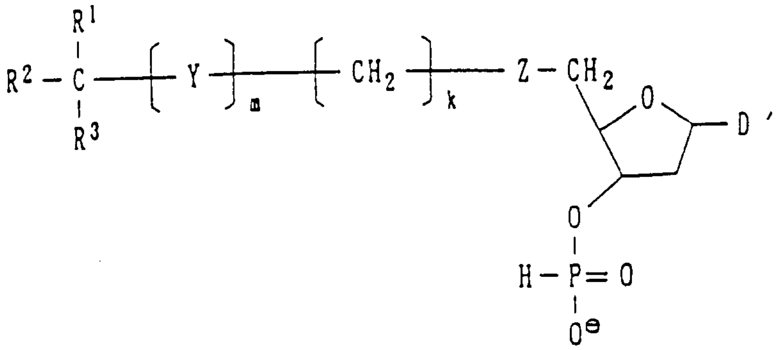

(wherein, k represents 0 or an integer of 1 to 20; m represents 0 or 1; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; and D' represents a residue selected from the following group $\beta$, provided that m = 0 when k = 0,

[Group $\alpha$]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group $\beta$]

Adenine (A), guanine (G), cytosine (C) and thymine (T)); with,

an oligonucleotide represented by the general formula:

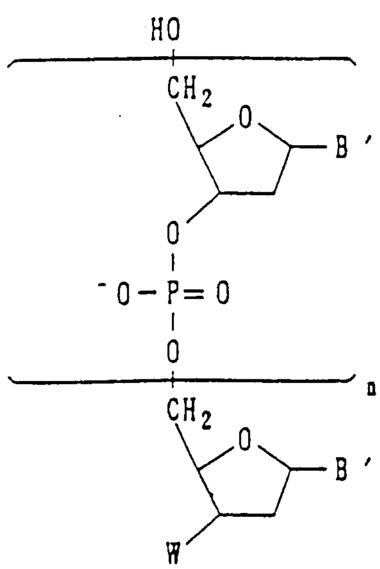

(wherein, B's each represent a residue independently selected from the following group $\beta$ in the respective nucleotide units or protected homologous residue; represents a lower acyloxy group, an arylacyloxy group or a controlled pore glass; and n is an integer of 4 to 29, provided that the base sequence containing B' is a base sequence complementary to a tumor gene or virus gene,

[Group $\beta$]

Adenine (A), guanine (G), cytosine (C) and thymine (T)),

which oligonucleotide is synthesized with a DNA synthesizer and is bonded to said controlled pore glass whose dimethoxytrityl group at the 5' terminal only is eliminated, with the base portion being protected with a protecting group;

in the presence of a condensing agent and a deoxidizer, to form H-phosphonic diester bonds, followed

by converting H-phosphonic acid bonds to diphosphate bonds using an oxidizing agent and removing the protecting group of the base portion simultaneous to severing the oligonucleotide from the controlled pore glass under basic conditions.

**93.** A process for preparing the compound described in Claim 3 wherein said compound is obtained by eliminating only the dimethoxytrityl group at the 5' terminal of a compound represented by the general formula:

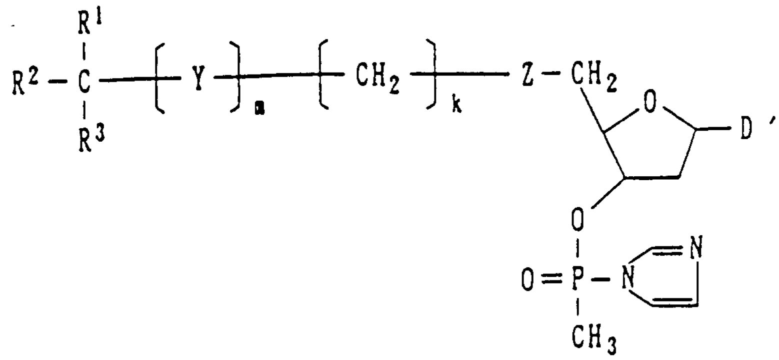

(wherein, k represents 0 or an integer of 1 to 20; m represents 0 or 1; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group $\alpha$; Y and Z each represent an oxygen or sulfur atom; and D' represents a residue selected from the following group $\beta$, provided that m 0 when k = 0,

[Group $\alpha$]

Hydroxyl group, alkyl group having 1 to 6 carbon atoms, alkoxy group having 1 to 6 carbon atoms, methylenedioxy group, nitro group, aside group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.

[Group $\beta$]

Adenine (A), guanine (G), cytosine (C) and thymine (T));

carrying out a condensation reaction in the presence of tetrazole on a methylphosphonate oligonucleotide represented by the general formula:

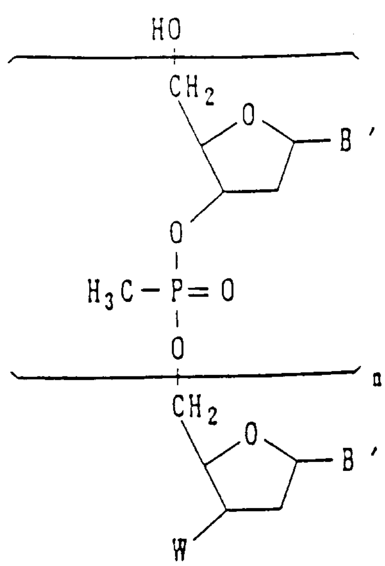

(wherein, B's each represent a residue independently selected from the following group $\beta$ in respective

nucleotide units or protected homologous residue; W represents a lower acyloxy group, an arylacyloxy group or a controlled pore glass; and n is an integer of 4 to 29, provided that the base sequence containing B' is a base sequence complementary to a tumor gene or virus gene,
[Group β]
Adenine (A), guanine (G), cytosine (C) and thymine (T)),
that is bonded to said controlled pore glass with the base portion thereof being protected with a protecting group, to form methylphosphonic diester bonds, followed by eliminating the protecting group of the base portion simultaneous to severing the oligonucleotide from the controlled glass beads under basic conditions.

**94.** A process for preparing the compound described in Claim 4 wherein said compound is obtained by forming phosphonic diester bonds in the presence of a condensing agent and oxidizing agent using a compound represented by the general formula:

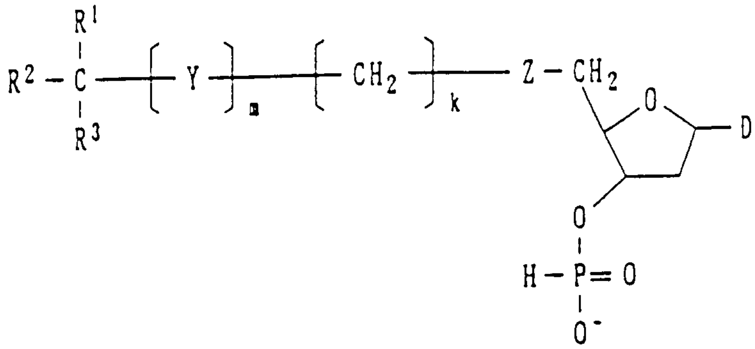

(wherein, k represents 0 or an integer of 1 to 20; m represents 0 or 1; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α; and D' represents a residue selected from the following group β or protected homologous residue, provided that m = 0 when k = 0,
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms,
[Group β]
Adenine (A), guanine (G), cytosine (C) and thymine (T)); and,
a thioate oligonucleotide represented by the general formula:

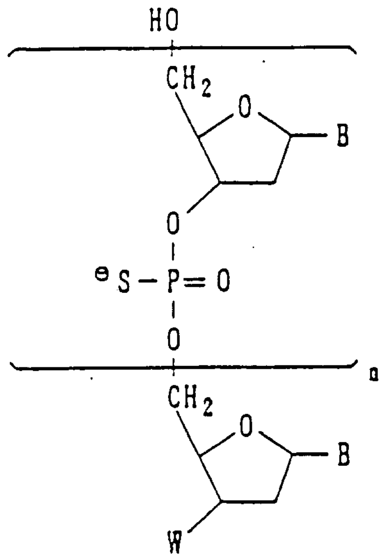

(wherein, B's each represent a residue independently selected from the following group β in respective nucleotide units or protected homogous residue; W represents a lower acyloxy group, an arylacyloxy group or a controlled pore glass: and n is an integer of 4 to 29, provided that the base sequence containing B' is a base sequence complementary to a tumor gene or virus gene,
[Group α]
Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms.
[Group β]
Adenine (A), guanine (G), cytosine (C) and thymine (T)),
which is synthesized with a DNA synthesizer and is bonded to said controlled pore glass whose dimethoxytrityl group at the 5' terminal only is eliminated;
followed by reacting sulfur dissolved in carbon disulfide in the presence of a base, and removing the protecting group of the base portion simultaneous to severing the oligonucleotide from the controlled pore glass under basic conditions.

**95.** A process for preparing the compound described in Claim 6 wherein said compound is obtained by eliminating the 5-position dimethoxytrityl group of a compound represented by the general formula:

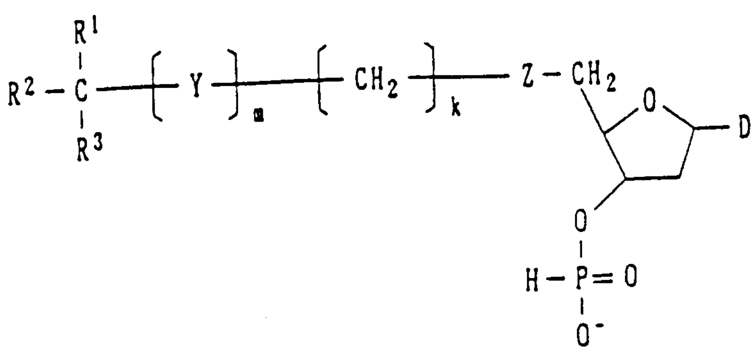

(wherein, k represents 0 or an integer of 1 to 20; m represents 0 or 1; $R^1$, $R^2$ and $R^3$ may be the same or different from one another and each represent a hydrogen atom, an alkyl group having 1 to 6 carbon atoms, or an aryl group having 6 to 10 carbon atoms that may have a substituent selected from the following group α; and D' represents a residue selected from the following group β; provided that m = 0 when k = 0,

[Group α]

Hydroxyl group, alkyl group having 1 to 6 carbon atoms, alkoxy group having 1 to 6 carbon atoms, methylenedioxy group, nitro group, azide group, halogen atom, aryl group having 6 to 10 carbon atoms, aryloxy group having 6 to 10 carbon atoms and aralkyloxy group with an aryl moiety having 6 to 10 carbon atoms and an alkyl moiety having 1 or 2 carbon atoms,

[Group β]

Adenine (A), guanine (G), cytosine (C) and thymine (T)); and

an alkylphosphoric amidate oligonucleotide represented by the general formula:

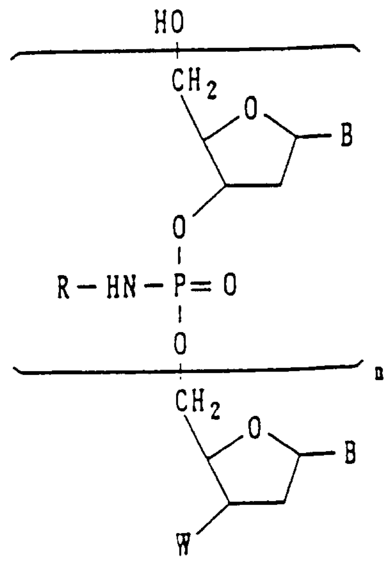

(wherein, B's each represent a residue independently selected from the following group β in respective nucleotide units; R represents a lower alkyl group; W represents a lower acyloxy group, an arylacyloxy group or a controlled pore glass; and n is an integer of 4 to 29, provided that the base sequence containing B' is a base sequence complementary to a tumor gene or virus gene,

[Group β]

Adenine (A), guanine (G), cytosine (C) and thymine (T)),

which is synthesized with a DNA synthesizer and is bonded to said controlled pore glass;

and reacting with a desired amine in the presence of a condensing agent followed by eliminating the oligonucleotide from the controlled pore glass and treating with a base.

FIG.1.

DmTr-ATACTCAGTCATTTTTAGCAG (Example 6)

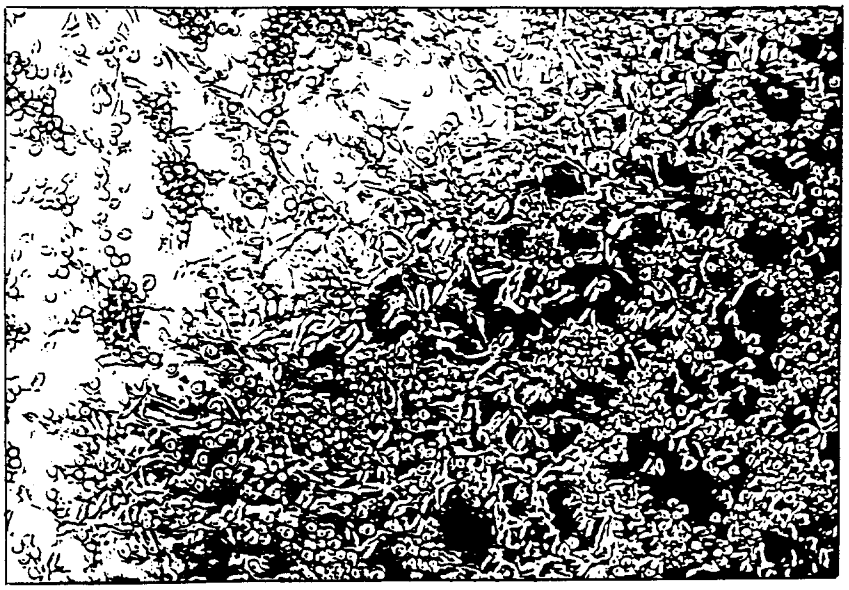

ATACTCAGTCATTTTTAGCAG

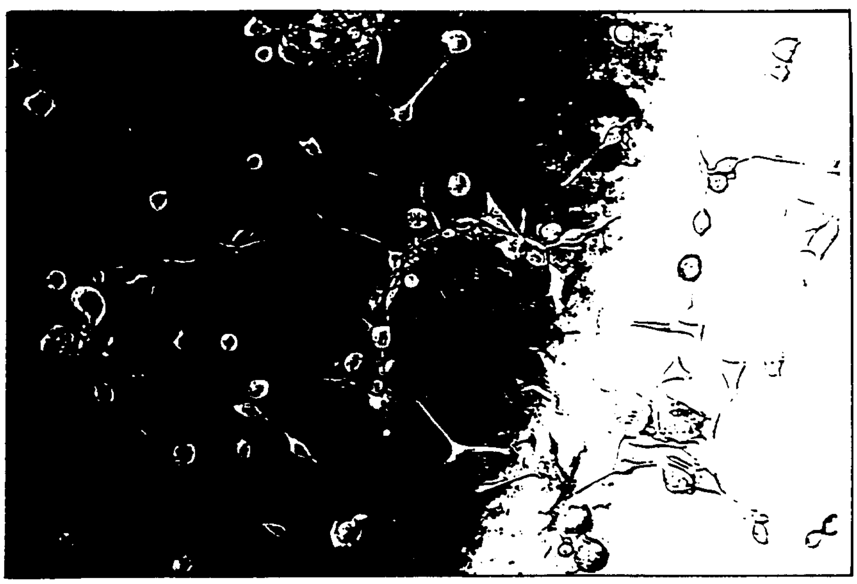

FIG.2.

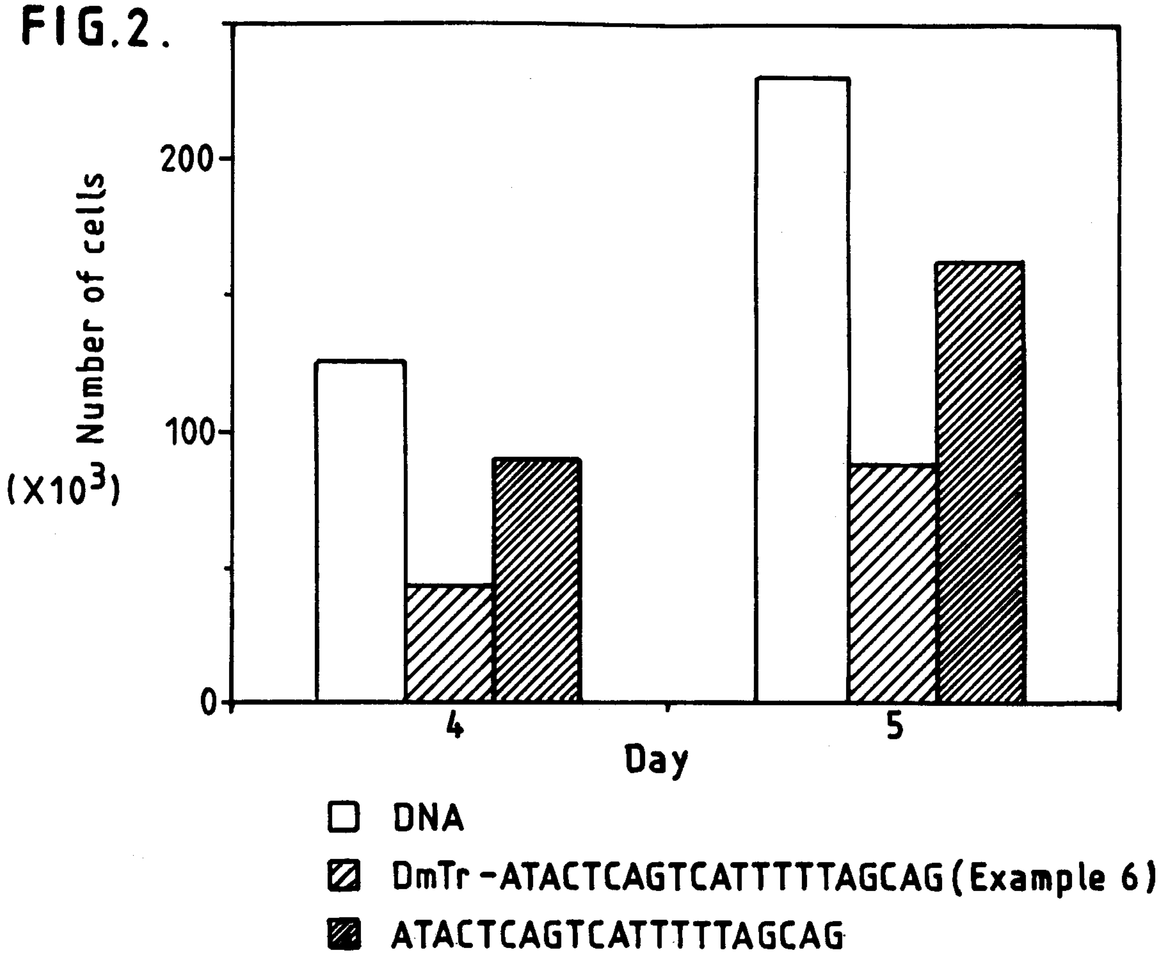
200
100
0
(X10³) Number of cells
4
5
Day
DNA
DmTr-ATACTCAGTCATTTTTAGCAG (Example 6)
ATACTCAGTCATTTTTAGCAG

FIG.3.

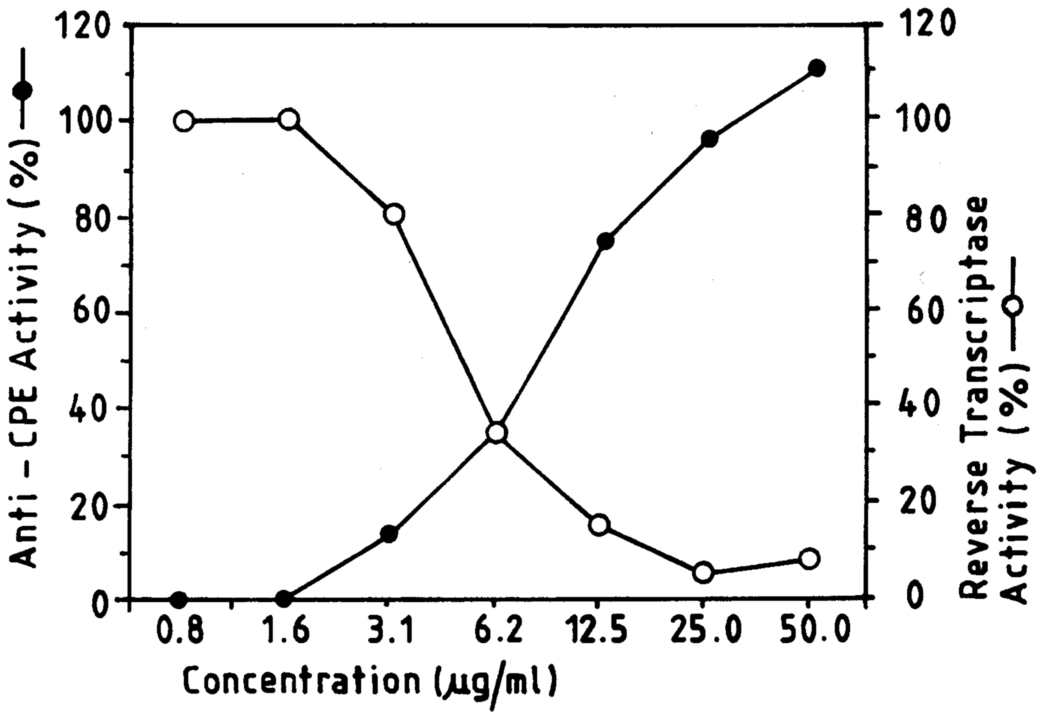
Anti-CPE Activity (%)
120
100
80
60
40
20
0
Reverse Transcriptase Activity (%)
0.8
1.6
3.1
6.2
12.5
25.0
50.0
Concentration (μg/ml)

FIG.4.

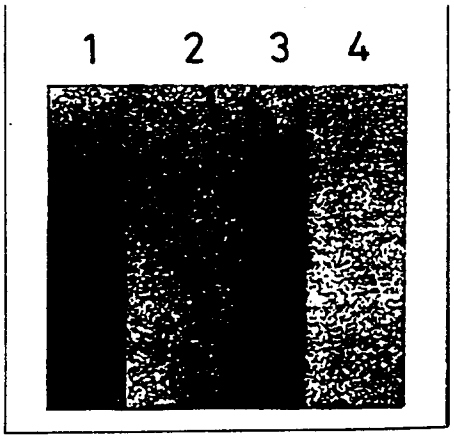


FIG.5.

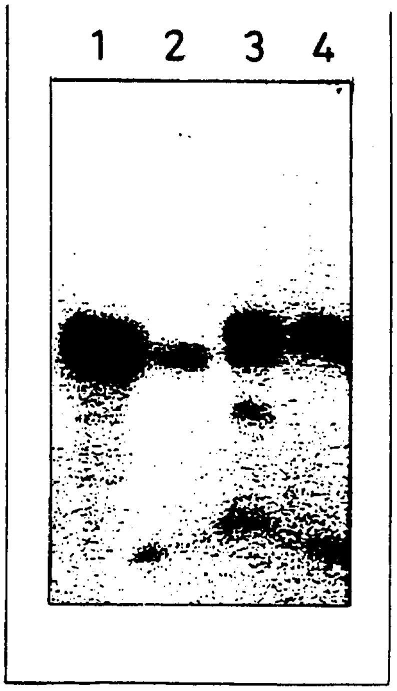

FIG.6.

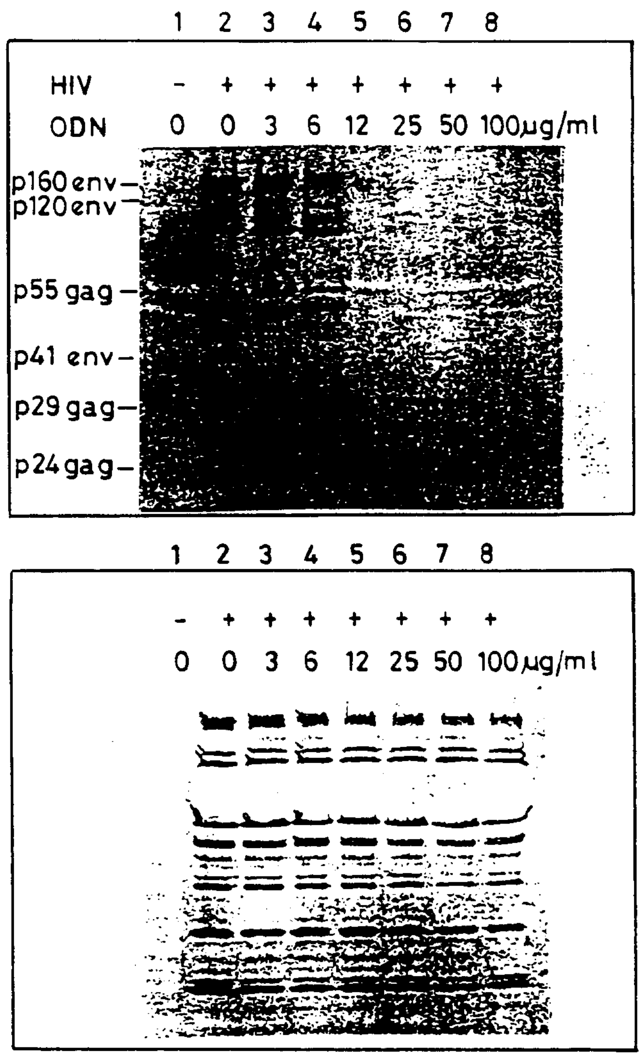
1 2 3 4 5 6 7 8
HIV - + + + + + + +
ODN 0 0 3 6 12 25 50 100μg/ml
p160 env
p120 env
p55 gag
p41 env
p29 gag
p24 gag
1 2 3 4 5 6 7 8
- + + + + + + +
0 0 3 6 12 25 50 100μg/ml

# INTERNATIONAL SEARCH REPORT

International Application No PCT/JP91/01572

**I. CLASSIFICATION OF SUBJECT MATTER** (if several classification symbols apply, indicate all) [6]

According to International Patent Classification (IPC) or to both National Classification and IPC

Int. Cl$^5$ C07H21/04, A61K31/70

**II. FIELDS SEARCHED**

Minimum Documentation Searched [7]

| Classification System | Classification Symbols |
|---|---|
| IPC | C07H21/00-21/04, A61K31/70 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | US, A, 4469863 (Paul O. P. Ts'o, et al.), September 4, 1984 (04. 09. 84) | 1-79, 90-95 |
| A | EP, A2, 288163 (US Dept Health & Human), October 26, 1988 (26. 10. 88) & WO, A, 88/7544 & JP, A, 1-503302 | 1-95 |
| A | Science, Vol. 248, P. 208-211 (13. 04. 1990), H. M. Buck et al. "Phosphate-Mothylated DNA Aimed at HIV-1 RNA Loops and Integratea DNA Inhibitors Viral In fectivity" | 80, 81 |
| A | EP, A2, 339842 (Ajinomoto Co., Inc.), April 13, 1989 (13. 04. 89), & JP, A, 02-16515 & JP, A, 03-128391 | 1-95 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 23, 1992 (23. 01. 92) | February 12, 1992 (12. 02. 92) |
| **International Searching Authority** | **Signature of Authorized Officer** |
| Japanese Patent Office | |